# FASCICULE DE BREVET EUROPEEN

(11) **EP 0 951 552 B1**
(45) Date de publication et mention de la délivrance du brevet: **26.01.2011**
(21) Numéro de dépôt: 97933727.6
(22) Date de dépôt: 11.07.1997
(51) Int. Cl.: C12N 15/31, C07K 14/22, C07K 16/12, A61K 39/095, C12Q 1/68, G01N 33/53

(54) **ADN ET PROTEINES OU PEPTIDES SPECIFIQUES DES BACTERIES DE L'ESPECE NEISSERIA MENINGITIDIS, LEURS PROCEDES D'OBTENTION ET LEURS APPLICATIONS BIOLOGIQUES**
DNA UND SPEZIFISCHE PROTEINE ODER PEPTIDE DER BAKTERIENSPEZIES NEISSERIA MENINGITIDIS, VERFAHREN ZU DEREN HERSTELLUNG UND BIOLOGISCHE ANWENDUNGEN
DNA AND SPECIFIC PROTEINS OR PEPTIDES OF THE NEISSERIA MENINGITIDIS SPECIES BACTERIA, METHOD FOR OBTAINING THEM AND THEIR BIOLOGICAL APPLICATIONS

(30) Priorité: 12.07.1996 FR 9608768
(43) Date de publication de la demande: 27.10.1999
(60) Demande divisionnaire: 09163902.1
(73) Titulaire: INSTITUT NATIONAL DE LA SANTE ET DE LA RECHERCHE MEDICALE (INSERM), 75654 Paris Cédex 13 (FR)
(72) Inventeur: NASSIF, Xavier, F-75015 Paris (FR); TINSLEY, Colin, F-75015 Paris (FR); ACHTMAN, Mark, D-10969 Berlin (DE); RUELLE, Jean-Louis, B-1300 Limal (BE); VINALS, Carla, B-4000 Liège (BE); MERKER, Petra, D-10997 Berlin (DE)
(74) Mandataire: Peaucelle, Chantal
(86) Numéro de dépôt international: PCT/FR1997/001295
(87) Numéro de publication internationale: WO 1998/002547

(56) Documents cités:
- EP-A- 0 337 896
- EP-A- 0 452 596
- WO-A-88/03957
- WO-A-90/15621
- WO-A-94/05703
- ZHOU J ET AL: "Sequence diversity within the argF, fbp and recA genes of natural isolates of Neisseria meningitidis: interspecies recombination within the argF gene." MOL MICROBIOL, AUG 1992, 6 (15) P2135-46, ENGLAND, XP000645119
- DEVI SJ ET AL: "Antibodies to poly[(2----8)-alpha-N-acetylneuraminic acid] and poly[(2----9)-alpha-N-acetylneuraminic acid] are elicited by immunization of mice with Escherichia coli K92 conjugates: potential vaccines for groups B and C meningococci and E. coli K1." PROC NATL ACAD SCI U S A, AUG 15 1991, 88 (16) P7175-9, UNITED STATES, XP002044636
- WOLFF K ET AL: "Identification and characterization of specific sequences encoding pathogenicity associated proteins in the genome of commensal Neisseria species." FEMS MICROBIOL LETT, JAN 15 1995, 125 (2-3) P255-63, NETHERLANDS, XP002044637
- PETERING H ET AL: "Genes associated with meningococcal capsule complex are also found in Neisseria gonorrhoeae." J BACTERIOL, JUN 1996, 178 (11) P3342-5, UNITED STATES, XP002044638
- FROSCH M ET AL: "Evidence for a common molecular origin of the capsule gene loci in gram-negative bacteria expressing group II capsular polysaccharides." MOL MICROBIOL, MAY 1991, 5 (5) P1251-63, ENGLAND, XP000647762
- FROSCH M ET AL: "Phospholipid substitution of capsular polysaccharides and mechanisms of capsule formation in Neisseria meningitidis." MOL MICROBIOL, MAY 1993, 8 (3) P483-93, ENGLAND, XP000647767
- FROSCH M ET AL: "CONSERVED OUTER-MEMBRANE PROTEIN OF NEISSERIA-MENINGITIDIS INVOLVED IN CAPSULE EXPRESSION" INFECTION AND IMMUNITY, 1992, 60, 798-803, XP002044642
- STRATHDEE CA ET AL: "IDENTIFICATION OF EPIDEMIOLOGIC MARKERS FOR NEISSERIA-MENINGITIDIS USING DIFFERENCE ANALYSIS" GENE, 1995, 166, 105-110, XP002044641
- SCHUTTE M ET AL: "Isolation of YAC insert sequences by representational difference analysis" NUCLEIC ACIDS RESEARCH, 23 (20). 1995. 4127-4133., XP002052562
- LAUERMAN LH ET AL: "Avian mycoplasma identification using polymerase chain reaction amplicon and restriction fragment length polymorphism analysis." AVIAN DIS, OCT-DEC 1995, 39 (4) P804-11, UNITED STATES, XP002052563
- LISITSYN N ET AL: "Cloning the differences between two complex genomes." SCIENCE, FEB 12 1993, 259 (5097) P946-51, UNITED STATES, XP002052564
- TINSLEY CR ET AL: "Analysis of the genetic differences between Neisseria meningitidis and Neisseria gonorrhoeae: two closely related bacteria expressing two different pathogenicities." PROC NATL ACAD SCI U S A, OCT 1 1996, 93 (20) P11109-14, UNITED STATES, XP002028346
- KLEE S.R. ET AL: 'Molecular and biological analysis of eight genetic islands taht distinguish Neisserai meningitidis from the closely related pathogen Neisseria gonorrheae' INFECTION ADN IMMUNITY vol. 68, no. 4, Avril 2000, pages 2082 - 2095, XP001113124
- FROSSCH M. ET AL: 'Phospholipid substitution of capsular mechanisms of capsule foramtion in Neisseria meningitidis' MOLECULAR MICROBIOLOGY vol. 8, no. 3, Mai 1993, pages 483 - 493, XP000646777
- SNYDER LORI A.S. ET AL: 'The majority of genes in the pathogenic Neisseria species are present in non-pathogenic Neisserai lactamica , including those designated as 'virulence genes'' BMC GENOMICS vol. 7, no. 128, 2006, pages 1 - 11
- TURNER D.P.J. ET AL: 'Autotransported serine protease A of Neisseria meningitidis : an immunogenic, surface-exposed outer membrane , and secreted protein' INFECTION AND IMMUNITY vol. 70, no. 8, Août 2002, pages 4447 - 4461
- DEMPSEY J.A.F. ET AL: 'The physical map of the chromosome of a serogroup A strain of neisseria meningitidis shows complex rearangements relative to the chromosomes of the two mapped strains of the closely realted species of n.gonorrhoeae' JOURNAL OF BACTERIOLOGY vol. 177, no. 22, 01 Novembre 1995, pages 6390 - 6400, XP001070651
- MOORE T.D.E. ET AL: 'Isolation and identification of a gluthatione peroxidase homolog gene , gpxA, present in Neisserai meningitid but absent in Neisserai gonorrhoeae' INFECTION AND IMMUNITY vol. 63, no. 4, Avril 1995, pages 1603 - 1607

### LISTE DE SEQUENCES

(1) INFORMATIONS GENERALES:
   (1) DEPOSANT.
      (A) NOM: I.N.S.E.R.M
      (B) RUE: 101, rue de Tolbiac
      (C) VILLE: PARIS CEDEX 13
      (E) PAYS: FRANCE
      (F) CODE POSTAL: 75654
   (ii) TITRE DE L' INVENTION: ADN, proteines et peptides spécifiques des bactéries de l'espèce Neisseria meningitidis, leurs procédés d'obtention et leurs applications biologiques.
   (iii) NOMBRE DE SEQUENCES: 99
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (CEB)
(2) INFORMATIONS POUR LA SEQ ID NO: 1:
   (i) CARACTERISTIQUES DE LA SEQUENCE:
      (A) LONGUEUR: 257 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOULTE : Z2491
   (xi) DESCRIPTION DE LA SEQUENCE: SEQ ID NO: 1:
   GATCCGCTGC CGGCAGACGA ATATCAAGAC ATCTTCGATT TTATGAAACA GTATGACTTG    60
   TCTTACCCGT ATGAATATCT GCAGGATTGG ATAGATTACT ATACGTTCAA AACCGATAAG   120
   CTGGTATTTG GTAACGCGAA GCGAGAGTGA GCCGTAAAAC TCTGAGCTCC TGTTTTATAG   180
   ATTACAACTT TAGGCCGTCT TAAAGCTGAA AGATTTTCGA AAGCTATAAA TTGAAGCCCT   240
   TCCACAGTAC ATAGATC                                                  257
(2) INFORMATIONS POUR LA SEQ ID NO: 2:
   (i) CARACTERISTIQUES DE LA SEQUENCE.
      (A) LONGUEUR: 276 paires de bases
      (B) TYPE: nucléotide
      (C) NOMBRE DE BRINS: simple
      (D) CONFIGURATICN: linéaire
   (ii) TYPE DE MOLECULE: ADN (génomique)
   (vi) ORIGINE.
      (A) ORGANISME: Neisseria meningitidis
      (B) SOUCHE: Z2491
   (xi) DESGRIPTICN DE LA SEQUENCE: SEQ ID NO: 2:
(2) INFORMATIONS POUR LA SEQ ID NO: 3:
   (i) CARACTERISTIQUES DE LA SEQUENCE:
      (A) LONGUEUR: 428 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) CRIGINE:
      (A) ORGANISME: Neisseria meningitidis
      (B) SOUCHE: Z2491
   (xi) DESCRIPTION DE LA SEQUENCE: SEQ ID NO: 3:
   GATCTGGTGG TGTTTGCACA GGTAGGCGCA TACTTGTTCG GGACTGAGTT TGCGGCGGAT 60
   AAGGGTGTCG ATGTGCTGAA TCAGCTGCGA ATCGAGCTTA TAGGGTTGTC GCTTACGCTG 120
   TTTGATAGTC CGGCTTTGCC GCTGGGCTTT TTCGGCGCTG TATTGCTGCC CTTGGGTGCG 180
   GTGCCGTCTG ATTTCGCGGC TGATGGTGCT TTTGTGGCGG TTAAGCTGTT TGGCGATTTC 240
   GGTGACGGTG CAGTGGCGGG ACAGGTATTG GATGTGGTAT CGTTCGCCTT GGGTCAGTTG 300
   CGTGTAGCTC ATGGCAATCT TTCTTGCAGG AAAGGCCGTA TGCTACCGCA TACTGGCCTT 360
   TTTCTGTTAG GGAAAGTTGC ACTTCAAATG CGAATCCGCC GACCTCTTTC AGTTACAGCA 420
   GCTTGATC 428
(2) INFORMATIONS POUR LA SEQ ID NO: 4:
   (i) CARACTERISTIQUES DE LA SEQUENCE:
      (A) LONGUEUR: 390 paires de bases
      (B) TYPE: nucléotide
      (C) NOMBRE DE BRINS: simple
      (D) CONFIGURATION: linéaire
   (il) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 4:
   GATCCTGCAT TGACATCGGC CTTGGCTGTC AGGGTATTGT GACCGGTAAA GTCGGCATTA    60
   CCGTTGGCCA ATAAGGATAC ATGACCGTCT GCAGAAACAG CATGAAGGCC GTCTGAAACG   120
   ATATTGCCCT GCAATGCGGT GGTTTCGAGA GCCTTGGCTG CGTTCAGCTT GGTATTGCGA   180
   AGCTGAATAT TGCCTTTGGC TGCCTGAATG TGCAGATTAC CCGAGTTGGT ACGCAGATTG   240
   GTATTGGTAA CATTCAGCAA GCCTGCCTCC ACACCCATGT CTTTTGAGGC AGTGAGGGTT   300
   TTACTGGTGC CGGTAATATG GGCAGCGTTA TCCGATTTCA AATGGATGCT GGCCGGCAGA   360
   CAAATCTTTA TCAACATTCA AATTCAGATC                                    390
(2) INFORMATIONS POUR LA SEQ ID NO: 5:
   (i) CARACTERISTIQUES DE LA SEQUENCE:
      (A) LONGUEUR: 177 paires de bases
      (B) TYPE: nucléotide
      (C) NOMBRE DE BRINS: simple
      (D) CCNFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 5:
   GATCAGATTG GTGAAGACGG TATTACCGTC AATGTTGCAG GCCGTTCGGG ATATACGGCG 60
   AAAATCGACG TGTCTCCGAG TACCGATTTG GCGGTTTATG GCCATATTGA AGTTGTACGG 120
   GGTGCAACGG GGTTGACCCA ATCCAATTCA GAGCCGGGTG GAACCGTCAA TTTGATC 177
(2) INFORMATIONS POUR LA SEQ ID NO: 6:
   (i) CARACTERISTIQUES DE LA SEQUENCE:
      (A) LONGUEUR: 341 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 6:
   GATCAATGAT GCTACTATTC AAGCGGGCAG TTCCGTGTAC AGCTCCACCA AAGGCGATAC 60
   TGAATTGGGT GAAAATACCC GTATTATTGC TGAAAACGTA ACCGTATTAT CTAACGGTAG 120
   TATTGGCAGT GCTGCTGTAA TTGAGGCTAA AGACACTGCA CACATTGAAT CGGGCAAACC 180
   GCTTTCTTTA GAAACCTCGA CCGTTGCCTC CAACATCCGT TTGAACAACG GTAACATTAA 240
   AGGCGGAAAG CAGCTTGCTT TACTGGCAGA CGATAACATT ACTGCCAAAA CTACCAATCT 300
   GAATACTCCC GGCAATCTGT ATGTTCATAC AGGTAAAGAT C 341
(2) INFORMATIONS POUR LA SEQ ID NO: 7:
   (i) CARACTERISTIQUES DE LA SEQUENCE:
      (A) LONGUEUR: 164 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 7:
(2) INFORMATIONS POUR LA SEQ ID NO: 8:
   (i) CARACTERISTIQUES DE LA SEQUENCE:
      (A) LONGUEUR: 219 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 8:
   GATCAATCAC ACATCTTGTC ATTTTTTCGA TTCCTTCATT TCGGTTTCTA ATGTTTCAAT 60
   TCTTGCGGCC ATTTCCTGAA TGGCTTTAGT CAAAACGGGG ATGAACGCTT CGTATTCGAC 120
   GGTGTAGGTA TCGTTTGTTT TATTTACCAT CGGCAATCGA CCATATTCAT CTTCCAGCGC 180
   AGCAATGTCC TGGGCAATAA ACCAATGCCG CAACCGATC 219
(2) INFORMATIONS POUR LA SEQ ID NO: 9:
   (i) CARACTERISTIQUES DE LA SEQUENCE:
      (A) LONGUEUR: 356 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 9:
   GATCTTGGGT AAGCCCCCAA CCTGCATAGA AAGGCAGGCC GTAGCAGCTG ACTTTTTTGC 60
   CGCGCAACAA GGCTTCAAAA CCGGTCAGCG AAGTCATGGT ATGTATTTCG TCTGCGTATT 120
   GGAGACAGGT CAGGATGTCG GCTTGTTCGG CGGTTTGGTC GGCATATCGT GCAGCATCAT 180
   CAGGGGAAAT ATGGCCGATG CGGTTACCGC TGACTACATC GGGATGCGGT TTGTAGATGA 240
   TATAGGCATT GGGGTTTCGT TCGCGTACGG TACGGAGCAA ATCCAGATTG CGGTAGATTT 300
   GGGGCGAACC GTAGCGGATA GACGCATCAT CTTCAACCTG GCCGGGAACG AGGATC 356
(2) INFORMATIONS POUR LA SEQ ID NO: 10:
   (i) CARACTERISTIQUES DE LA SEQUENCE:
      (A) LONGUEUR: 210 paires de bases
      (B) TYPE: nucléotide
      (C) NOMBRE DE BRINS: simple
      (D) CCNFIGURATION: lineaire
   (ii) TYPE DE MOLECULE: ADN (génomique)
   (vi) ORIGINE:
      (A) ORGANISME: Neisseria meningitidis
      (B) SOUCHE. Z2491
   (xi) DESCRIPTION DE LA SEQUENCE: SEQ ID NO: 10:
   GATCCGCTTT CAGTTTCCGT ACCGGTGGCA TCAGTCAAGT CCGTTTTGTG CACCAAACCG 60
   CGTCCATATG AAACATAAAA CAAATCGCTT AAGCCCAAAG GGTTATCGAA CGATAAAGCG 120
   ACATTTCCTT GATATTTGCC GGTCGTTTTG CCGCCCGCAT CATCTATACC GATACTGAAC 180
   CGTATGGGTT TATTCTGCTG CCATTTGATC 210
(2) INFORMATIONS POUR LA SEQ ID NO: 11:
   (i) CARACTERISTIQUES DE LA SEQUENCE:
      (A) LONGUEUR: 259 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 11:
   GATCCCGAAA CGCAATTGGT CGAAAGCTAT ATGCTGAACG ATGTGTTGCG GTTTTGGGAC 60
   AGCGCAGGTT TGGGCGATGG GAAAGAAGCC GACCGCGCCC ATCGGCAAAA ACTGATTGAT 120
   GTCCTGTCTA AAACCTATAC TCATTCGGAT GGGCAGTGGG GCTGGATAGA TTTGGTGTTC 180
   GTTATCCTTG ACGGCAGCTC CCGCGATTTG GGTACGGCCT ATGATTTGTT GAGGGATGTT 240
   ATCCTTAAAA TGATTGATC 259
(2) INFORMATIONS POUR LA SEQ ID NO: 12:
   (i) CARACTERISTIQUES DE LA SEQUENCE:
      (A) LONGUEUR: 436 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 12:
   GATCAAATGG ATGATTTATA TAGAATTTTC TTTTACGACT GCGTGCCGTT TGAAAAGAAA 60
   ATGCACAATC CCGTATCTCA TCGTGCCATA GATTTTTCAA AGACTCCGGA AGCCATATTT 120
   CGTTGCAATC TGCATACCGA ATTGAAGAAG AAGCGTAAAT TAGCGTTACG TTTAGGCAAG 180
   CTGTCGGACA ATACAGCATG GATATTAAAA CCCCAAGTCA TGAAAAATCT TCTGAAAAAC 240
   CCGTCAACTC AAATTACGGA AAACGATGTC GTGCTCGATG TTAAACAAAA AGGTGTAGAT 300
   ATGCGTATAG GCTTGGATAT TTCATCTATT ACCTTAAAAA AACAAGCCGA TAAAATCATC 360
   TTGTTTTCTG GTGATTCCGA TTTTGTCCCA GCAGCCAAAT TAGCCAGACG GGAAGGTATC 420
   GATTTTATTC TTGATC 436
(2) INFORMATIONS POUR LA SEQ ID NO: 13:
   (i) CARACTERISTIQUES DE LA SEQUENCE:
      (A) LONGUEUR: 363 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 13;
(2) INFORMATIONS POUR LA SEQ ID NO: 14:
   (i) CARACTERISTIQUES DE LA SEQUENCE:
      (A) LONGUEUR: 314 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 14:
   GATCTTGCGT CATTTATATC TTCACCGATA TTGCAATTAC CGCCGTTCCA GTTGAAATAA 60
   CAACGACTAA AATTGTAGTT CCTAAAAGAA TCATTCCTAT TCTTGCGTAC CATTTCCCAA 120
   TAATTGCGCC CGACAATTTC CATTTAATGC TCCATCAGTT CTTTTACTTC CGGAAATCTG 180
   CTGTAATCTG ACATAAGACG CATAATTGAA CTATCAACGC CGTAACAGCC ATAGGTTTTA 240
   ATACCGTTTT CGGCGTGTTC CCAAATGCAA TTACTGTATT CGTAGCCTTT TACAAATTTA 300
   TCGGTTTCGG GATC 314
(2) INFORMATIONS POUR LA SEQ ID NO: 15:
   (i) CARACTERISTIQUES DE LA SEQUENCE
      (A) LONGUEUR: 256 paires de bases
      (B) TYPE: nucleotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 15:
   GATCATACGA ATCTACCCTA AAATACCCCG TCGCCGATTT AGGATTGGCT ACATAAAGCT 60
   CATTATAAGG GTATTTTGAT GACATGATAC GGTTAAATTC ATTGCCGTTG TTTATCCTGA 120
   TTCTATAAAT TGGTTCAACA GCAAAGCCTC TGGATTCCCT TAATTGATTA TAATATTGCC 180
   TGTATGTTTG TACATCATGT CTTGTCCACG GCTCTCCAGG AGTCCTCAGA ATAGCAATCC 240
   CGTTAAATTT CGGATC 256
(2) INFORMATIONS POUR LA SEQ ID NO: 16:
   (i) CARACTERISTIQUES DE LA SEQUENCE:
      (A) LONGUEUR: 235 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 16:
   GATCCACGCC TGTGCCTACC TTGGCTTTTT GTTCGCCAAA CAAGGCATTT AAGGTTGAGG 60
   ACTTGCCGAC ACCTGTCGCA CCGACAAGCA AGACATCCAA ATGACGGAAA CCGGCTGCTG 120
   TGACTTTTTG CCCGATTTCA GAAATACGGT AACGATGCAT ATGCGCTCCT ACCAGCCAAA 180
   AAAAGAAGCA ACCGTGCTAA TCGCCCCTCC AATCGCTTTT GCAGCACCGC CGATC 235
(2) INFORMATIONS POUR LA SEQ ID NO: 17:
   (i) CARACTERISTIQUES DE LA SEQUENCE:
      (A) LONGUEUR: 259 paires de bases
      (B) TYPE: nucléotide
      (C) NOMBRE DE BRINS: simple
      (D) CCNFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 17:
   GATCCAACGG GCATCGCTGT CCTTACTCGG TGTGGTTTGA CCGCTGATTT GTCCTTCTTC 60
   GTCAACTTCT ATGGCCTGAC GCTGTTTGCT GCCGGCGGTC TGGATAATGG TGGCATCAAC 120
   GACGGCGGCG GATGCTTTCT CTATTTTTAG GCCTTTTTCG GTCAGTTGGC AGTTAATCAG 180
   TTTGAGTAAT TCGGACAGGG TGTCGTCTTG CGCCAGCCAG TTGCGGTAGC GGCATAAGGT 240
   ACTGTAATCG GGGATGATC 259
(2) INFORMATIONS POUR LA SEQ ID NO: 18:
   (i) CARACTERISTIQUES DE LA SEQUENCE:
      (A) LONGUEUR: 201 paires de bases
      (B) TYPE: nucléotide
      (C) NOMBRE DE BRINS: simple
      (D) CONFIGURATION: linéaire
   (ii) TYPE DE MOLECULE: ADN (génémique)
   (vi) ORIGINE:
      (A) ORGANISME: Neisseria meningitidis
      (B) SOUCHE: Z2491
   (xi) DESCRIPTION DE LA SEQUENCE: SEQ ID NO: 18:
   GATCTGTGCC GTTGATTTTA TCTTTCAGAT GCAGCATCGA ATATCGGAAA GCCAAATCAG 60
   CAATTCTTTT TGCATCGTGT GGATTTTGAG ACGGGCCTAA TGACCGTACC CGCTTAATAA 120
   AAAATGCACC GTCAATCAAA ATGGCGGTTT TCATATTGCT TCCCCTATAT TTGTCAAAGA 180
   TATAAAAAAG CCCTTGGGAT C 201
(2) INFORMATIONS POUR LA SEQ ID NO: 19:
   (i) CARACTERISTIQUES DE LA SEQUENCE:
      (A) LONGUEUR: 334 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 19:
   AATTCAAAGG AGGCATTTGT TGCAAGAAAA GTACAAAGTG ATTTGCAAAA AGCATTGAAT 60
   GCTAGCAACT ATAACAAGCA GCAATATGCA AGACGTGCGG CAACAGCGTT AGAGAATGCT 120
   TCAAAATCAA AAGTTATGGC AGCGAATTCT TTTTGATCTA TCTTGTGCGA ACGGGTCAAA 180
   TATTCTTCGT ACATTGAGTT AATCGTACCA ATCGCCCTAA CCACATTTTC ATCAGAAAAT 240
   ATGGAAATAA TAGCATCCCT ATACGCACCT AGTGTAATAT TGTTTCTATT ATTAGTTATA 300
   GCATTATTCG AATACATAAT AGCACCTCCA AATT 334
(2) INFORMATIONS POUR LA SEQ ID NO: 20:
   (i) CARACTERISTIQUES DE LA SEQUENCE:
      (A) LONGUEUR: 238 paires de bases
      (B) TYPE: nucléotide
      (C) NOMBRE DE BRINS: simple
      (D) CONFIGURATION: linéaire
   (ii) TYPE DE MOLECULE: ADN (genomique)
   (vi) ORIGINE:
      (A) ORGANISME: Neisseria meningitidis
      (B) SOUCHE: Z2491
   (xi) DESCRIPTION DE LA SEQUENCE SEQ ID NO: 20:
   AATTCCTGCG CACCTTTGCC GATGGGGAGA TAATCGCCTT TTTGCAGCAT TCTGCCCTGA 60
   TGGCCGCCGA AACCGGCTTT CAGGTCGGTA CTTCTCGAAC CCATCACTTC CGGCACATCA 120
   AATCCGCCCG CCACGCACAC ATAGCCGTAC ATGCCCTGCA CGGCACGCAC CAGTTTCAAG 180
   GTCTGCCCTT TGCGGGCGGT ATAACGCCAA TACGAATAGA CCGGTTCGCC GTCCAATT 238
(2) INFORMATIONS POUR LA SEQ ID NO: 21:
   (i) CARACTERISTIQUES DE LA SEQUENCE:
      (A) LONGUEUR: 249 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 21:
   AATTGGGCGA GATGCTGCCG GAAACGGATT TAAAACAGAT TGCGGCGGCA GTGTTGAAGA 60
   CGAACGATGA GGCGGCATTG CAGAAGGTGG TGAAAACGGC CAAAGGCAAT GCGCGGAAAC 120
   TGTCGAAGCT GCTGCTGATT GTGGACTATT TGTTGCAGGT TAACCCTGAT GTTGATTTGG 180
   ATGATGATGT AATCGAACAC GCGGAAACCT ATTTAATCCA CTAAACCTTT GACAGATAAG 240
   GCAATAATT 249
(2) INFORMATIONS POUR LA SEQ ID NO: 22:
   (i) CARACTERISTIQUES DE LA SEQUENCE
      (A) LONGUEUR: 212 paires de bases
      (B) TYPE: nucléotide
      (C) NOMBRE DE BRINS: simple
      (D) CONFIGURATION: lineaire
   (ii) TYPE DE MOLECULE: ADN (genomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 22:
   AATTTATGTA CGGTTTTGCC GTTTGCAGTC AGCCAGTCGG CAAGGCGCAG AAAAAAATCG 60
   CCGACAGGGC CTTGAAGCAG CAGGATATTT TCTGCGCTTT CAAGCAGGTT TTGCAGGTTA 120
   TTTTTGAGGA CGGTCTGTTT CATGTTGCAA TGTGGTTTTG TTTTTTATGT AATAGTTTTA 180
   GGTTGAACTT TCAAGCATAC GCCAAGAGAA TT 212
(2) INFORMATIONS POUR LA SEQ ID NO: 23:
   (i) CARACTERISTIQUES DE LA SEQUENCE:
      (A) LONGUEUR: 227 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 23:
   AATTCAGTGC CTGCGTCATA TCACGGCTAC CTTGTGGTTC AGGGTTACTG TATCGCCCGC 60
   GGCATCGACG GCTTCAATAT GCAGCTTCAG CCAGCCGTGC TGCGGGGCGG ATGCGGTTAC 120
   TTGGATGGAT TGGGCGCGTT TGGACTGAAT CACGGGCTGC AAGGCTTGCT CGGCGTACTG 180
   TTTGGCCAGT ACTTCGATGC GCTTTAAATG CTTTTGGCGG CGCAATT 227
(2) INFORMATIONS POUR LA SEQ ID NO: 24:
   (i) CARACTERISTIQUES DE LA SEQUENCE:
      (A) LONGUEUR: 167 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE.
      (A) ORGANISME: Neisseria meningitidis
      (B) SOUCHE: Z2491
   (xi) DESCRIPTION DE LA SEQUENCE: SEQ ID NO: 24:
   GATCCAGGAC TCAAAAACCG ATTTCCTAAT AGAGTGTCTA ATATCCCAAT CTTTTTTACC 60
   CCCTCTGCTG TAGAATTGAT AGAGAAAGTT TGTCTATCTT TTTCATATAC CCATGCCTTC 120
   TTTTTATCAT TGTAGCTAAC ATAACCGCCA AACAATGCTT CTAGATC 167
(2) INFORMATIONS POUR LA SEQ ID NO: 25:
   (i) CARACTERISTIQUES DE LA SEQUENCE:
      (A) LONGUEUR: 251 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 25:
   AATTCTTGCG GCCATTTCCT GAATGGCTTT AGTCAAAACG GGGATGAACG TTTCGTATTC 60
   GACGGTGTAG GTATCGTTTG TTTTATTTAC CATCGGCAAT CGACCATATT CATCTTCCAG 120
   CGCAGCAATG TCCTGGGCAA TAAACCAATG CCGCAACCGA TCTTCTTTAT GACTGCCGTC 180
   CTTGATTGGA TTCGCCCACC ATTCGCGGAC TTTGTCCGCT CGTTCATCTG CCGGCAAGTC 240
   TTTGAATAAT T 251
(2) INFORMATIONS POUR LA SEQ ID NO: 26:
   (i) CARACTERISTIQUES DE LA SEQUENCE:
      (A) LONGUEUR: 207 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 26:
   AATTCCCGAC TATCGCGGAT GCGTAGTTTT TGCCGGTGGG CAAGAGCAGG TGTGGGATAA     60
   GTTAGGTGAT TTGCCCGATG GCGTCAGCCT GACCCCGCCT GAATCGGTAA ATATTGACGG   120
   CTTAAAATCC GTAAAACTCG TCGCATTAAA TGCTGCCGCT CAGGCTTTTA TTAACAAGCA   180
   CGCCGGTATC GACAGCGTAC CTGAATT                                      207
(2) INFORMATIONS POUR LA SEQ ID NO: 27:
   (i) CARACTERISTIQUES DE LA SEQUENCE:
      (A) LONGUEUR: 379 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 27:
   AATTGTTTGG GAATAATCCA AACAAACAGC ATCAGGATAG CGGCGGCGGT CAGGCTGCCT    60
   GAAAGGATTT TGCCGGGGTT TTTTGTAGGC AAAGCGGACG AGAAACCAAA GCAACAGCAG 120
   CATGGTGTCC CAATAGCCGA TTGAGAATAG GATGGCCAAA CCTTCTAGGA AATGGCGTAA 180
   ATCGTTTGTG GTAACCATGG GTAGTTCCTG TGGTTAAATG TGCAGGCTGC TTTTTGCCGA 240
   ACCTTGCCGC ATCTCAAAAG CAGCCTGCGC TTCAGCGTTG CGTTACGCAG TAAAATAATG 300
   AATATTTGTA ACGGCTTGGG TATTTTTTGT CAATATTCCC GCCCTTCCCT TAACAGCTGC 360
   CGCGCTTTCC GTTAAAATT 379
(2) INFORMATIONS POUR LA SEQ ID NO: 28:
   (i) CARACTERISTIQUES DE LA SEQUENCE:
      (A) LONGUEUR: 274 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 28:
   AATTCGCCGA AATCAGGCTG CTGCTCGATA ATCGGCGCGG CCGATTGGCG TTGTGCCTCG 60
   ATTAAATCCA TCTTGTCTTG CAGACGTTTG GCCTGGCCTT TGCGGCGGCG TTCGGCCAGT 120
   TGTTCCATCC GCGTTTCCGC AAATGCCGCC CGTTTGTTGC CGTTGAATAC CGCTTTGCAA 180
   ATCACCTTGC CCTGCATATC CTTCACAATC ACATGGTCGG CATCGTGGAT GTCGTAAGCC 240
   ACCCGTACCT TCTGACCGCT GTAATCCAGC AATT 274
(2) INFORMATIONS POUR LA SEQ ID NO: 29:
   (i) CARACTERISTIQUES DE LA SEQUENCE:
      (A) LONGUEUR: 263 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 29:
   AATTCCGTTC TTATTGGGCT TTTTCCATCC ATCGGGTATG CCTGAAGGGA ACGCAAACCC     60
   TGCCACTTGC CCATCGCTCC ATTCCCGCAT TAGCGCGTCT GACGGCAAGT GTTCTCGCGC    120
   CCAATCAAGC CACGCCTGCC GCATTGCGGC CTTGTCCTGC TGAAAACTTC GCAGTGCTTT    180
   TGCAACCGGC CCATCATTAA CTTCAATCAA ATAAATCATT ATATTTGCGT TCATTTTTCC    240
   TACACCTTCG CCACATCCAA ATT                                            263
(2) INFORMATIONS POUR LA SEQ ID NO: 30:
   (i) CARACTERISTIQUES DE LA SEQUENCE:
      (A) LONGUEUR: 316 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 30:
   AATTGTTCAA GAAAAAAGTC GGCACGGCGC GGCAACGGGG AAAATGCGTT GACGCCGTCT     60
   TTTTCTAAGG TGATGTAGTA GGGGCGGAAA TAGCCTTCTT CAAACGCCCA GAAACTGGCT    120
   TGGTTTTCGT TTGCAATGCG TTTTGCAATG ACGTGATAAG GGCGTGTGTC GCCAAAGCAG    180
   ACAACGGCCT GGATGTGATG TTGAGTGATG TATTCTTGCA AAAACTCAGG AAAGGCGTCG    240
   TAGTTGTCGT TAAAAACAAC GGTATGCGCT TGAGTGGGCG GATAAAAATA GTCGTCGCCT    300
   GCATTAAAGT TGAATT                                                    316
(2) INFORMATIONS POUR LA SEQ ID NO: 31:
   (i) CARACTERISTIQUES DE LA SEQUENCE:
      (A) LONGUEUR: 324 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 31:
   AATTCAATCA ACGGAAAACA CATCAGCATC AAAAACAACG GTGGTAATGC CGACTTAAAA    60
   AACCTTAACG TCCATGCCAA AAGCGGGGCA TTGAACATTC ATTCCGACCG GGCATTGAGC   120
   ATAGAAAATA CCAAGCTGGA GTCTACCCAT AATACGCATC TTAATGCACA ACACGAGCGG   180
   GTAACGCTCA ACCAAGTAGA TGCCTACGCA CACCGTCATC TAAGCATTAC CGGCAGCCAG   240
   ATTTGGCAAA ACGACAAACT GCCTTCTGCC AACAAGCTGG TGGCTAACGG TGTATTGGCA   300
   CTCAATGCGC GCTATTCCCA AATT                                          324
(2) INFORMATIONS POUR LA SEQ ID NO: 32:
   (i) CARACTERISTIQUES DE LA SEQUENCE:
      (A) LONGUEUR: 230 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO. 32:
   AATTATGCAA AAAAACGCAA CGCCGAAAAA CTGGCACCGC GCGGATATTG TTGCTGCTTT 60
   GAAAAAGAAA GGCTGGTCAC TTCGAGCACT TTCAATAGAA GCGGGGTTGT CGCCGAATAC 120
   GCTTAGAAGC GCACTGGCCG CCCCTTATCT TAAGGGAGAA AGGATTATTG CCGCTGCAAT 180
   CGGAGTGGAA CCGGAAGAGA TTTGGTCCGA ACGGTATGCA GATCGGAATT 230
(2) INFORMATIONS POUR LA SEQ ID NO: 33:
   (i) CARACTERISTIQUES DE LA SEQUENCE:
      (A) LCNGUEUR: 249 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: Z2491
   (xi) DESCRIPTION DE LA SEQUENCE: SEQ ID NO: 33:
(2) INFORMATIONS POUR LA SEQ ID NO: 34:
   (i) CARACTERISTIQUES DE LA SEQUENCE:
      (A) LCNGUEUR: 343 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE. Z2491
   (xi) RESCRIPTION DE LA SEQUENCE: SEQ ID NO: 34:
(2) INFORMATIONS POUR LA SEQ ID NO: 35:
   (i) CARACTERISTIQUES DE LA SEQUENCE:
      (A) LONGUEUR: 184 paires de bases
      (B) TYPE: nucléotide
      (C) NOMBRE DE BRINS: simple
      (D) CONFIGURATION: linéaire
   (ii) TYPE DE MOLECULE: ADN (génomique)
   (vi) ORIGINE:
      (A) ORGANISME: Neisseria meningitidis
      (B) SOUCHE: 22491
   (xi) DESCRIPTION DE LA SEQUENCE: SEQ ID NO: 35:
   AATTCTTCAA ACATCGTTTC GATAATCGGG TCGGTGTACA CACTGATGCG GTCGCCCGCA 60
   CGGCTTTGAC CGGCTCGGAA AATATAGGCG GTGGCTTTGC CGTCGGCGAT GTCGACGCAC 120
   CAACGCCAGA TGGCGTCTTC GGTATTCAAA CAATCACCCG CACAGCTTTC ACCTGCGCGG 180
   AATT 184
   (xi) DESCRIPTION DE LA SEQUENCE: SEQ ID NO: 36:
(2) INFORMATIONS POUR LA SEQ ID NO: 37:
   (i) CARACTERISTIQUES DE LA SEQUENCE:
      (A) LONGUEUR: 580 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Protein
      (B) EMPLACEMENT:1..580
   (xi) DESCRIPTION DE LA SEQUENCE: SEQ ID NO: 37:
   Met Lys Phe Phe Pro Ala Pro Cys Leu Leu Val Ile Leu Ala Val Ile
   1 5 10 15
   Pro Leu Lys Thr Leu Ala Ala Asp Glu Asn Asp Ala Glu Leu Ile Arg
   20 25 30
   Ser Met Gln Arg Gln Gln His Ile Asp Ala Glu Leu Leu Thr Asp Ala
   35 40 45
   Asn Val Arg Phe Glu Gln Pro Leu Glu Lys Asn Asn Tyr Val Leu Ser
   50 55 60
   Glu Asp Glu Thr Pro Cys Thr Arg Val Asn Tyr Ile Ser Leu Asp Asp
   65 70 75 80
   Lys Thr Ala Arg Lys Phe Ser Phe Leu Pro Ser Val Leu Met Lys Glu
   85 90 95
   Thr Ala Phe Lys Thr Gly Met Cys Leu Gly Ser Asn Asn Leu Ser Arg
   100 105 110
   Leu Gln Lys Ala Ala Gln Gln Ile Leu Ile Val Arg Gly Tyr Leu Thr
   115 120 125
   Ser Gln Ala Ile Ile Gln Pro Gln Asn Met Asp Ser Gly Ile Leu Lys
   130 135 140
   Leu Arg Val Ser Ala Gly Glu Ile Gly Asp Ile Arg Tyr Glu Glu Lys
   145 150 155 160
   Arg Asp Gly Lys Ser Ala Glu Gly Ser Ile Ser Ala Phe Asn Asn Lys
   165 170 175
   Phe Pro Leu Tyr Arg Asn Lys Ile Leu Asn Leu Arg Asp Val Glu Gln
   180 185 190
   Gly Leu Glu Asn Leu Arg Arg Leu Pro Ser Val Lys Thr Asp Ile Gln
   195 200 205
   Ile Ile Pro Ser Glu Glu Glu Gly Lys Ser Asp Leu Gln Ile Lys Trp
   210 215 220
   Gln Gln Asn Lys Pro Ile Arg Phe Ser Ile Gly Ile Asp Asp Ala Gly
   225 230 235 240
   Gly Lys Thr Thr Gly Lys Tyr Gln Gly Asn Val Ala Leu Ser Phe Asp
   245 250 255
   Asn Pro Leu Gly Leu Ser Asp Leu Phe Tyr Val Ser Tyr Gly Arg Gly
               260                     265                     270
   Leu Val His Lys Thr Asp Leu Thr Asp Ala Thr Gly Thr Glu Thr Glu
           275                     280                     285
   Ser Gly Ser Arg Ser Tyr Ser Val His Tyr Ser Val Pro Val Lys Lys
       290                     295                     300
   Trp Leu Phe Ser Phe Asn His Asn Gly His Arg Tyr His Glu Ala Thr
   305                     310                     315                     320
   Glu Gly Tyr Ser Val Asn Tyr Asp Tyr Asn Gly Lys Gln Tyr Gln Ser
                   325                     330                     335
   Ser Leu Ala Ala Glu Arg Met Leu Trp Arg Asn Arg Phe His Lys Thr
               340                     345                     350
   Ser Val Gly Met Lys Leu Trp Thr Arg Gln Thr Tyr Lys Tyr Ile Asp
           355                     360                     365
   Asp Ala Glu Ile Glu Val Gln Arg Arg Arg Ser Ala Gly Trp Glu Ala
       370                     375                     380
   Glu Leu Arg His Arg Ala Tyr Leu Asn Arg Trp Gln Leu Asp Gly Lys
   385                     390                     395                     400
   Leu Ser Tyr Lys Arg Gly Thr Gly Met Arg Gln Ser Met Pro Ala Pro
                   405                     410                     415
   Glu Glu Asn Gly Gly Gly Thr Ile Pro Gly Thr Ser Arg Met Lys Ile
               420                     425                     430
   Ile Thr Ala Gly Leu Asp Ala Ala Ala Pro Phe Met Leu Gly Lys Gln
           435                     440                     445
   Gln Phe Phe Tyr Ala Thr Ala Ile Gln Ala Gln Trp Asn Lys Thr Pro
       450                     455                     460
   Leu Val Ala Gln Asp Lys Leu Ser Ile Gly Ser Arg Tyr Thr Val Arg
   465                     470                     475                     480
   Gly Phe Asp Gly Glu Gln Ser Leu Phe Gly Glu Arg Gly Phe Tyr Trp
                   485                     490                     495
   Gln Asn Thr Leu Thr Trp Tyr Phe His Pro Asn His Gln Phe Tyr Leu
   500 505 510
   Gly Ala Asp Tyr Gly Arg Val Ser Gly Glu Ser Ala Gln Tyr Val Ser
   515 520 525
   Gly Lys Gln Leu Met Gly Ala Val Val Gly Phe Arg Gly Gly His Lys
   530 535 540
   Val Gly Gly Met Phe Ala Tyr Asp Leu Phe Ala Gly Lys Pro Leu His
   545 550 555 560
   Lys Pro Lys Gly Phe Gln Thr Thr Asn Thr Val Tyr Gly Phe Asn Leu
   565 570 575
   Asn Tyr Ser Phe
   580
(2) INFORMATIONS POUR LA SEQ ID NO. 38:
   (i) CARACTERISTIQUES DE LA SEQUENCE:
      (A) LONGUEUR: 1981 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Peptide
      (B) EMPLACEMENT:1..1981
   (xi) DESCRIPTION DE LA SEQUENCE: SEQ ID NO: 38:
   Met Asn Lys Gly Leu His Arg Ile Ile Phe Ser Lys Lys His Ser Thr
   1 5 10 15
   Met Val Ala Val Ala Glu Thr Ala Asn Ser Gln Gly Lys Gly Lys Gln
   20 25 30
   Ala Gly Ser Ser Val Ser Val Ser Leu Lys Thr Ser Gly Asp Leu Cys
   35 40 45
   Gly Lys Leu Lys Thr Thr Leu Lys Thr Leu Val Cys Ser Leu Val Ser
   50 55 60
   Leu Ser Met Val Leu Pro Ala His Ala Gln Ile Thr Thr Asp Lys Ser
   65 70 75 80
   Ala Pro Lys Asn Gln Gln Val Val Ile Leu Lys Thr Asn Thr Gly Ala
   85 90 95
   Pro Leu Val Asn Ile Gln Thr Pro Asn Gly Arg Gly Leu Ser His Asn
   100 105 110
   Arg Tyr Thr Gln Phe Asp Val Asp Asn Lys Gly Ala Val Leu Asn Asn
   115 120 125
   Asp Arg Asn Asn Asn Pro Phe Leu Val Lys Gly Ser Ala Gln Leu Ile
   130 135 140
   Leu Asn Glu Val Arg Gly Thr Ala Ser Lys Leu Asn Gly Ile Val Thr
   145 150 155 160
   Val Gly Gly Gln Lys Ala Asp Val Ile Ile Ala Asn Pro Asn Gly Ile
   165 170 175
   Thr Val Asn Gly Gly Gly Phe Lys Asn Val Gly Arg Gly Ile Leu Thr
   180 185 190
   Ile Gly Ala Pro Gln Ile Gly Lys Asp Gly Ala Leu Thr Gly Phe Asp
   195 200 205
   Val Arg Gln Gly Thr Leu Thr Val Gly Ala Ala Gly Trp Asn Asp Lys
   210 215 220
   Gly Gly Ala Asp Tyr Thr Gly Val Leu Ala Arg Ala Val Ala Leu Gln
   225 230 235 240
   Gly Lys Leu Gln Gly Lys Asn Leu Ala Val Ser Thr Gly Pro Gln Lys
   245 250 255
   Val Asp Tyr Ala Ser Gly Glu Ile Ser Ala Gly Thr Ala Ala Gly Thr
   260 265 270
   Lys Pro Thr Ile Ala Leu Asp Thr Ala Ala Leu Gly Gly Met Tyr Ala
   275 280 285
   Asp Ser Ile Thr Leu Ile Ala Asn Glu Lys Gly Val Gly Val Lys Asn
   290 295 300
   Ala Gly Thr Leu Glu Ala Ala Lys Gln Leu Ile Val Thr Ser Ser Gly
   305 310 315 320
   Arg Ile Glu Asn Ser Gly Arg Ile Ala Thr Thr Ala Asp Gly Thr Glu
   325 330 335
   Ala Ser Pro Thr Tyr Leu Ser Ile Glu Thr Thr Glu Lys Gly Ala Ala
   340 345 350
   Gly Thr Phe Ile Ser Asn Gly Gly Arg Ile Glu Ser Lys Gly Leu Leu
   355 360 365
   Val Ile Glu Thr Gly Glu Asp Ile Ser Leu Arg Asn Gly Ala Val Val
   370 375 380
   Gln Asn Asn Gly Ser Arg Pro Ala Thr Thr Val Leu Asn Ala Gly His
   385 390 395 400
   Asn Leu Val Ile Glu Ser Lys Thr Asn Val Asn Asn Ala Lys Gly Ser
   405 410 415
   Ala Asn Leu Ser Ala Gly Gly Arg Thr Thr Ile Asn Asp Ala Thr Ile
   420 425 430
   Gln Ala Gly Ser Ser Val Tyr Ser Ser Thr Lys Gly Asp Thr Glu Leu
   435 440 445
   Gly Glu Asn Thr Arg Ile Ile Ala Glu Asn Val Thr Val Leu Ser Asn
   450 455 460
   Gly Ser Ile Gly Ser Ala Ala Val Ile Glu Ala Lys Asp Thr Ala His
   465 470 475 480
   Ile Glu Ser Gly Lys Pro Leu Ser Leu Glu Thr Ser Thr Val Ala Ser
   485 490 495
   Asn Ile Arg Leu Asn Asn Gly Asn Ile Lys Gly Gly Lys Gln Leu Ala
   500 505 510
   Leu Leu Ala Asp Asp Asn Ile Thr Ala Lys Thr Thr Asn Leu Asn Thr
   515 520 525
   Pro Gly Asn Leu Tyr Val His Thr Gly Lys Asp Leu Asn Leu Asn Val
   530 535 540
   Asp Lys Asp Leu Ser Ala Ala Ser Ile His Leu Lys Ser Asp Asn Ala
   545 550 555 560
   Ala His Ile Thr Gly Thr Ser Lys Thr Leu Thr Ala Ser Lys Asp Met
   565 570 575
   Gly Val Glu Ala Gly Leu Leu Asn Val Thr Asn Thr Asn Leu Arg Thr
   580 585 590
   Asn Ser Gly Asn Leu His Ile Gln Ala Ala Lys Gly Asn Ile Gln Leu
   595 600 605
   Arg Asn Thr Lys Leu Asn Ala Ala Lys Ala Leu Glu Thr Thr Ala Leu
   610 615 620
   Gln Gly Asn Ile Val Ser Asp Gly Leu His Ala Val Ser Ala Asp Gly
   625 630 635 640
   His Val Ser Leu Leu Ala Asn Gly Asn Ala Asp Phe Thr Gly His Asn
   645 650 655
   Thr Leu Thr Ala Lys Ala Asp Val Asn Ala Gly Ser Val Gly Lys Gly
   660 665 670
   Arg Leu Lys Ala Asp Asn Thr Asn Ile Thr Ser Ser Ser Gly Asp Ile
   675 680 685
   Thr Leu Val Ala Gly Asn Gly Ile Gln Leu Gly Asp Gly Lys Gln Arg
   690 695 700
   Asn Ser Ile Asn Gly Lys His Ile Ser Ile Lys Asn Asn Gly Gly Asn
   705 710 715 720
   Ala Asp Leu Lys Asn Leu Asn Val His Ala Lys Ser Gly Ala Leu Asn
   725 730 735
   Ile His Ser Asp Arg Ala Leu Ser Ile Glu Asn Thr Lys Leu Glu Ser
   740 745 750
   Thr His Asn Thr His Leu Asn Ala Gln His Glu Arg Val Thr Leu Asn
   755 760 765
   Gln Val Asp Ala Tyr Ala His Arg His Leu Ser Ile Thr Gly Ser Gln
   770 775 780
   Ile Trp Gln Asn Asp Lys Leu Pro Ser Ala Asn Lys Leu Val Ala Asn
   785 790 795 800
   Gly Val Leu Ala Leu Asn Ala Arg Tyr Ser Gln Ile Ala Asp Asn Thr
   805 810 815
   Thr Leu Arg Ala Gly Ala Ile Asn Leu Thr Ala Gly Thr Ala Leu Val
   820 825 830
   Lys Arg Gly Asn Ile Asn Trp Ser Thr Val Ser Thr Lys Thr Leu Glu
   835 840 845
   Asp Asn Ala Glu Leu Lys Pro Leu Ala Gly Arg Leu Asn Ile Glu Ala
   850 855 860
   Gly Ser Gly Thr Leu Thr Ile Glu Pro Ala Asn Arg Ile Ser Ala His
   865 870 875 880
   Thr Asp Leu Ser Ile Lys Thr Gly Gly Lys Leu Leu Leu Ser Ala Lys
   885 890 895
   Gly Gly Asn Ala Gly Ala Pro Ser Ala Gln Val Ser Ser Leu Glu Ala
   900 905 910
   Lys Gly Asn Ile Arg Leu Val Thr Gly Glu Thr Asp Leu Arg Gly Ser
   915 920 925
   Lys Ile Thr Ala Gly Lys Asn Leu Val Val Ala Thr Thr Lys Gly Lys
   930 935 940
   Leu Asn Ile Glu Ala Val Asn Asn Ser Phe Ser Asn Tyr Phe Pro Thr
   945 950 955 960
   Gln Lys Ala Ala Glu Leu Asn Gln Lys Ser Lys Glu Leu Glu Gln Gln
   965 970 975
   Ile Ala Gln Leu Lys Lys Ser Ser Pro Lys Ser Lys Leu Ile Pro Thr
   980 985 990
   Leu Gln Glu Glu Arg Asp Arg Leu Ala Phe Tyr Ile Gln Ala Ile Asn
   995 1000 1005
   Lys Glu Val Lys Gly Lys Lys Pro Lys Gly Lys Glu Tyr Leu Gln Ala
   1010 1015 1020
   Lys Leu Ser Ala Gln Asn Ile Asp Leu Ile Ser Ala Gln Gly Ile Glu
   1025 1030 1035 1040
   Ile Ser Gly Ser Asp Ile Thr Ala Ser Lys Lys Leu Asn Leu His Ala
   1045 1050 1055
   Ala Gly Val Leu Pro Lys Ala Ala Asp Ser Glu Ala Ala Ala Ile Leu
   1060 1065 1070
   Ile Asp Gly Ile Thr Asp Gln Tyr Glu Ile Gly Lys Pro Thr Tyr Lys
   1075 1080 1085
   Ser His Tyr Asp Lys Ala Ala Leu Asn Lys Pro Ser Arg Leu Thr Gly
   1090 1095 1100
   Arg Thr Gly Val Ser Ile His Ala Ala Ala Ala Leu Asp Asp Ala Arg
   1105 1110 1115 1120
   Ile Ile Ile Gly Ala Ser Glu Ile Lys Ala Pro Ser Gly Ser Ile Asp
   1125 1130 1135
   Ile Lys Ala His Ser Asp Ile Val Leu Glu Ala Gly Gln Asn Asp Ala
   1140 1145 1150
   Tyr Thr Phe Leu Lys Thr Lys Gly Lys Ser Gly Lys Ile Ile Arg Lys
   1155 1160 1165
   Thr Lys Phe Thr Ser Thr Arg Asp His Leu Ile Met Pro Ala Pro Val
   1170 1175 1180
   Glu Leu Thr Ala Asn Gly Ile Thr Leu Gln Ala Gly Gly Asn Ile Glu
   1185 1190 1195 1200
   Ala Asn Thr Thr Arg Phe Asn Ala Pro Ala Gly Lys Val Thr Leu Val
   1205 1210 1215
   Ala Gly Glu Glu Leu Gln Leu Leu Ala Glu Glu Gly Ile His Lys His
   1220 1225 1230
   Glu Leu Asp Val Gln Lys Ser Arg Arg Phe Ile Gly Ile Lys Val Gly
   1235 1240 1245
   Lys Ser Asn Tyr Ser Lys Asn Glu Leu Asn Glu Thr Lys Leu Pro Val
   1250 1255 1260
   Arg Val Val Ala Gln Thr Ala Ala Thr Arg Ser Gly Trp Asp Thr Val
   1265 1270 1275 1280
   Leu Glu Gly Thr Glu Phe Lys Thr Thr Leu Ala Gly Ala Asp Ile Gln
   1285 1290 1295
   Ala Gly Val Gly Glu Lys Ala Arg Val Asp Ala Lys Ile Ile Leu Lys
   1300 1305 1310
   Gly Ile Val Asn Arg Ile Gln Ser Glu Glu Lys Leu Glu Thr Asn Ser
   1315 1320 1325
   Thr Val Trp Gln Lys Gln Ala Gly Arg Gly Ser Thr Ile Glu Thr Leu
   1330 1335 1340
   Lys Leu Pro Ser Phe Glu Ser Pro Thr Pro Pro Lys Leu Ser Ala Pro
   1345 1350 1355 1360
   Gly Gly Tyr Ile Val Asp Ile Pro Lys Gly Asn Leu Lys Thr Glu Ile
   1365 1370 1375
   Glu Lys Leu Ser Lys Gln Pro Glu Tyr Ala Tyr Leu Lys Gln Leu Gln
   1380 1385 1390
   Val Ala Lys Asn Ile Asn Trp Asn Gln Val Gln Leu Ala Tyr Asp Arg
   1395 1400 1405
   Trp Asp Tyr Lys Gln Glu Gly Leu Thr Glu Ala Gly Ala Ala Ile Ile
   1410 1415 1420
   Ala Leu Ala Val Thr Val Val Thr Ser Gly Ala Gly Thr Gly Ala Val
   1425 1430 1435 1440
   Leu Gly Leu Asn Gly Ala Ala Ala Ala Ala Thr Asp Ala Ala Phe Ala
   1445 1450 1455
   Ser Leu Ala Ser Gln Ala Ser Val Ser Phe Ile Asn Asn Lys Gly Asp
   1460 1465 1470
   Val Gly Lys Thr Leu Lys Glu Leu Gly Arg Ser Ser Thr Val Lys Asn
   1475 1480 1485
   Leu Val Val Ala Ala Ala Thr Ala Gly Val Ala Asp Lys Ile Gly Ala
   1490 1495 1500
   Ser Ala Leu Asn Asn Val Ser Asp Lys Gln Trp Ile Asn Asn Leu Thr
   1505 1510 1515 1520
   Val Asn Leu Ala Asn Ala Gly Ser Ala Ala Leu Ile Asn Thr Ala Val
   1525 1530 1535
   Asn Gly Gly Ser Leu Lys Asp Asn Leu Glu Ala Asn Ile Leu Ala Ala
   1540 1545 1550
   Leu Val Asn Thr Ala His Gly Glu Ala Ala Ser Lys Ile Lys Gln Leu
   1555 1560 1565
   Asp Gln His Tyr Ile Val His Lys Ile Ala His Ala Ile Ala Gly Cys
   1570 1575 1580
   Ala Ala Ala Ala Ala Asn Lys Gly Lys Cys Gln Asp Gly Ala Ile Gly
   1585 1590 1595 1600
   Ala Ala Val Gly Glu Ile Val Gly Glu Ala Leu Thr Asn Gly Lys Asn
   1605 1610 1615
   Pro Asp Thr Leu Thr Ala Lys Glu Arg Glu Gln Ile Leu Ala Tyr Ser
   1620 1625 1630
   Lys Leu Val Ala Gly Thr Val Ser Gly Val Val Gly Gly Asp Val Asn
   1635 1640 1645
   Ala Ala Ala Asn Ala Ala Glu Val Ala Val Lys Asn Asn Gln Leu Ser
   1650 1655 1660
   Asp Lys Glu Gly Arg Glu Phe Asp Asn Glu Met Thr Ala Cys Ala Lys
   1665 1670 1675 1680
   Gln Asn Asn Pro Gln Leu Cys Arg Lys Asn Thr Val Lys Lys Tyr Gln
   1685 1690 1695
   Asn Val Ala Asp Lys Arg Leu Ala Ala Ser Ile Ala Ile Cys Thr Asp
   1700 1705 1710
   Ile Ser Arg Ser Thr Glu Cys Arg Thr Ile Arg Lys Gln His Leu Ile
   1715 1720 1725
   Asp Ser Arg Ser Leu His Ser Ser Trp Glu Ala Gly Leu Ile Gly Lys
   1730 1735 1740
   Asp Asp Glu Trp Tyr Lys Leu Phe Ser Lys Ser Tyr Thr Gln Ala Asp
   1745 1750 1755 1760
   Leu Ala Leu Gln Ser Tyr His Leu Asn Thr Ala Ala Lys Ser Trp Leu
   1765 1770 1775
   Gln Ser Gly Asn Thr Lys Pro Leu Ser Glu Trp Met Ser Asp Gln Gly
   1780 1785 1790
   Tyr Thr Leu Ile Ser Gly Val Asn Pro Arg Phe Ile Pro Ile Pro Arg
   1795 1800 1805
   Gly Phe Val Lys Gln Asn Thr Pro Ile Thr Asn Val Lys Tyr Pro Glu
   1810 1815 1820
   Gly Ile Ser Phe Asp Thr Asn Leu Lys Arg His Leu Ala Asn Ala Asp
   1825 1830 1835 1840
   Gly Phe Ser Gln Glu Gln Gly Ile Lys Gly Ala His Asn Arg Thr Asn
   1845 1850 1855
   Phe Met Ala Glu Leu Asn Ser Arg Gly Gly Arg Val Lys Ser Glu Thr
   1860 1865 1870
   Gln Thr Asp Ile Glu Gly Ile Thr Arg Ile Lys Tyr Glu Ile Pro Thr
   1875 1880 1885
   Leu Asp Arg Thr Gly Lys Pro Asp Gly Gly Phe Lys Glu Ile Ser Ser
   1890 1895 1900
   Ile Lys Thr Val Tyr Asn Pro Lys Lys Phe Ser Asp Asp Lys Ile Leu
   1905 1910 1915 1920
   Gln Met Ala Gln Asn Ala Ala Ser Gln Gly Tyr Ser Lys Ala Ser Lys
   1925 1930 1935
   Ile Ala Gln Asn Glu Arg Thr Lys Ser Ile Ser Glu Arg Lys Asn Val
   1940 1945 1950
   Ile Gln Phe Ser Glu Thr Phe Asp Gly Ile Lys Phe Arg Ser Tyr Phe
   1955 1960 1965
   Asp Val Asn Thr Gly Arg Ile Thr Asn Ile His Pro Glu
   1970 1975 1980
(2) INFORMATIONS POUR LA SEQ ID NO: 39:
   (i) CARACTERISTIQUES DE LA SEQUENCE:
      (A) LONGUEUR: 143 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Peptide
      (B) EMPLACEMENT:1..143
   (xi) DESCRIPTION DE LA SEQUENCE: SEQ ID NO: 39:
   Met Lys Asn Asn Ile Phe Leu Asn Leu Asn Lys Lys Ser Ile Asn Asn
   1 5 10 15
   Asn His Phe Val Ile Ser Ile Phe Phe Glu Thr Ile Tyr Gln Phe Glu
   20 25 30
   Thr Lys Asp Thr Leu Leu Glu Cys Phe Lys Asn Ile Thr Thr Thr Gly
   35 40 45
   His Phe Gly Val Ile Gly Ala Gln Tyr Glu Lys Ile Asp Ala Thr Arg
   50 55 60
   Trp Ile Gly Asp Tyr Glu Glu Val Asn Gly Phe Glu Tyr Ile Asp Lys
   65 70 75 80
   Ala Pro Ser Ile Tyr Phe Ser Val Gly Asp Asp Phe Asn Pro Glu Glu
   85 90 95
   Leu Ile Ile Pro Ile Asn Leu Ala Tyr His Tyr Phe Asn Ile Ala Ile
   100 105 110
   Ser Asp Phe Leu Ile Ala His Pro Glu Tyr Gln Lys Lys Cys Lys Glu
   115 120 125
   Ile Gln Lys Thr Tyr Ser Gln Thr Asn Cys Ser Leu His Glu Thr
   130 135 140
(2) INFORMATIONS POUR LA SEQ ID NO: 40:
   (i) CARACTERISTIQUES DE LA SEQUENCE.
      (A) LCNGUEUR: 833 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Peptide
      (B) EMPLACEMENT:1..833
   (xi) DESCRIPTION DE LA SEQUENCE: SEQ ID NO: 40:
(2) INFORMATIONS POUR LA SEQ ID NO: 41.
   (i) CARACTERISTIQUES DE LA SEQUENCE:
      (A) LONGUEUR: 833 acides amines
      (B) TYPE: acide aminé
      (D) CONFIGURATION: lineaire
   (ii) TYPE DE MOLECULE: protéine
   (xi) DESCRIPTION DE LA SEQUENCE: SEQ ID NO: 41:
   Val Leu Lys Thr Pro Pro Thr Leu Ala Ala Glu Leu Ser Gly Lys Thr
    1               5                  10                  15
   Gly Val Ser Ile Ser Ala Pro Tyr Ala Asn Glu Asn Ser Arg Ile Leu
               20                  25                  30
   Leu Ser Thr Thr Asp Ile Ser Ser Glu Asn Gly Lys Ile Lys Ile Gln
           35                  40                  45
   Ser Tyr Gly Asp Gln Tyr Tyr Tyr Ala Arg Gln Ser Glu Leu Tyr Thr
       50                  55                  60
   Phe Glu Arg Arg Ser Tyr Lys Thr Gly Lys Trp Tyr Asn Arg Lys His
   65                  70                  75                  80
   Ile Thr Glu Val Lys Glu His Lys Asn Ala Lys Pro Asp Ala Val Asn
                   85                  90                  95
   Leu Ser Ala Ser Gln Gly Ile Asp Ile Lys Ser Gly Gly Ser Ile Asp
               100                 105                 110
   Ala Tyr Ala Thr Ala Phe Asp Ala Pro Lys Gly Ser Ile Asn Ile Glu
           115                 120                 125
   Ala Gly Arg Lys Leu Thr Leu Tyr Ala Val Glu Glu Leu Asn Tyr Asp
       130                 135                 140
   Lys Leu Asp Ser Gln Lys Arg Arg Arg Phe Leu Gly Ile Ser Tyr Ser
   145                 150                 155                 160
   Lys Ala His Asp Thr Thr Thr Gln Val Met Lys Thr Ala Leu Pro Ser
                   165                 170                 175
   Arg Val Val Ala Glu Ser Ala Asn Leu Gln Ser Gly Trp Asp Thr Lys
   180 185 190
   Leu Gln Gly Thr Gln Phe Glu Thr Thr Leu Gly Gly Ala Thr Ile Arg
   195 200 205
   Ala Gly Val Gly Glu Gln Ala Arg Ala Asp Ala Lys Ile Ile Leu Glu
   210 215 220
   Gly Ile Lys Ser Ser Ile His Thr Glu Thr Val Ser Ser Ser Lys Ser
   225 230 235 240
   Thr Leu Trp Gln Lys Gln Ala Gly Arg Gly Ser Asn Ile Glu Thr Leu
   245 250 255
   Gln Leu Pro Ser Phe Thr Gly Pro Val Ala Pro Val Leu Ser Ala Pro
   260 265 270
   Gly Gly Tyr Ile Val Asp Ile Pro Lys Gly Asn Leu Lys Thr Gln Ile
   275 280 285
   Glu Thr Leu Thr Lys Gln Pro Glu Tyr Ala Tyr Leu Lys Gln Leu Gln
   290 295 300
   Val Ala Lys Asn Ile Asn Trp Asn Gln Val Gln Leu Ala Tyr Asp Lys
   305 310 315 320
   Trp Asp Tyr Lys Gln Glu Gly Met Thr Pro Ala Ala Ala Ala Val Val
   325 330 335
   Val Ile Val Val Thr Val Leu Thr Tyr Gly Ala Leu Ser Ala Pro Ala
   340 345 350
   Ala Ala Gly Thr Ala Gly Ala Ala Gly Ala Gly Ala Gly Gly Ala Ala
   355 360 365
   Ala Gly Thr Ala Ala Gly Thr Gly Val Ala Ala Gly Thr Ala Ala Thr
   370 375 380
   Thr Gly Val Ala Ala Gly Thr Ser Ala Ala Ala Ile Thr Thr Ala Ala
   385 390 395 400
   Gly Lys Ala Ala Leu Ala Ser Leu Ala Ser Gln Ala Ala Val Ser Leu
   405 410 415
   Ile Asn Asn Lys Gly Asp Ile Asn His Thr Leu Lys Glu Leu Gly Lys
   420 425 430
   Ser Ser Thr Val Arg Gln Ala Ala Thr Ala Ala Val Thr Ala Gly Val
   435 440 445
   Leu Gln Gly Ile Ser Gly Leu Asn Thr Gln Ala Ala Glu Ala Val Ser
   450 455 460
   Lys His Phe His Ser Pro Ala Ala Gly Lys Leu Thr Ala Asn Leu Ile
   465 470 475 480
   Asn Ser Thr Ala Ala Ala Ser Val His Thr Ala Ile Asn Gly Gly Ser
   485 490 495
   Leu Lys Asp Asn Leu Gly Asp Ala Ala Leu Gly Ala Ile Val Ser Thr
   500 505 510
   Val His Gly Glu Val Ala Ser Lys Ile Lys Phe Asn Leu Ser Glu Asp
   515 520 525
   Tyr Ile Ala His Lys Ile Ala His Ala Val Ala Gly Cys Ala Ser Ala
   530 535 540
   Val Ala Asn Lys Gly Lys Cys Arg Asp Gly Ala Ile Gly Ala Ala Val
   545 550 555 560
   Gly Glu Met Val Gly Glu Thr Leu Leu Asp Gly Arg Asp Val Gly Lys
   565 570 575
   Leu Ser Pro Gln Glu Arg Gln Lys Val Ile Ala Tyr Ser Gln Ile Ile
   580 585 590
   Ala Gly Ser Ala Val Ala Leu Val Lys Gly Asp Val Asn Thr Ala Val
   595 600 605
   Asn Ala Ala Thr Val Ala Val Glu Asn Asn Ser Leu Leu Ala Arg Arg
   610 615 620
   Arg Val Asn Ile Arg Trp Thr Pro Arg Gln Glu Leu Glu His Glu Tyr
   625 630 635 640
   Ala Ile Leu Glu Ile Gln Ala Ile Thr Asn Gln Ile Arg Arg Leu Asp
   645 650 655
   Pro Lys Phe Asn Gly Ile Ala Ile Leu Arg Thr Pro Gly Glu Pro Trp
   660 665 670
   Thr Arg His Asp Val Gln Thr Tyr Arg Gln Tyr Tyr Asn Gln Leu Arg
   675 680 685
   Glu Ser Arg Gly Phe Ala Val Glu Pro Ile Tyr Arg Ile Arg Ile Asn
   690 695 700
   Asn Gly Asn Glu Phe Asn Arg Ile Met Ser Ser Lys Tyr Pro Tyr Asn
   705 710 715 720
   Glu Leu Tyr Val Ala Asn Pro Lys Ser Ala Thr Gly Tyr Phe Arg Val
   725 730 735
   Asp Ser Tyr Asp Pro Ala Thr Arg Glu Ile Ile Ser Arg Lys Phe Thr
   740 745 750
   Gln Phe Ser Gln Ile Gln Glu Ser Thr Gly Ile Gly Tyr Ile Lys Glu
   755 760 765
   Ala Val Arg Lys Tyr Ser Pro Gly Thr Val Ile Ser Asn Val Pro Ser
   770 775 780
   Thr Pro Thr Thr Ile Arg Gly Arg Lys Leu Glu Gly Lys Leu Ile Leu
   785 790 795 800
   Glu Val Pro Ala Gln Val Asn Pro Ile Pro Gln Ser Val Leu Arg Ala
   805 810 815
   Ala Gln Glu Glu Asn Val Ile Ile Arg Asp Thr Thr Gly Arg Ile Tyr
   820 825 830
   Lys
(2) INFORMATIONS POUR LA SEQ ID NO: 42:
   (i) CARACTERISTIQUES DE LA SEQUENCE:
      (A) LONGUEUR: 162 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Peptide
      (B) EMPLACEMENT:1..162
   (xi) DESCRIPTION DE LA SEQUENCE: SEQ ID NO: 42:
   Met Lys Lys Asp Ile Phe Tyr Cys Glu Gln Trp Ser Tyr Gly Tyr Lys
   1               5                   10                  15
   Arg Leu His Lys Pro Phe Ser Glu Lys Gln Ala Glu Glu Lys His Leu
               20                  25                  30
   Lys Gly Glu Leu Tyr Thr Ala Val Ile Gly Ser Ala Thr Gln Pro Glu
           35                  40                  45
   Tyr Val Ile Thr Leu Arg Glu Glu Val Gly Phe Phe Ser Val Asn Phe
       50                  55                  60
   Phe Asp Lys Phe Gly Arg Asp Tyr Leu Thr His Gln Phe Gln Lys Tyr
   65                  70                  75                  80
   Ser Asn Ser Asn Tyr Tyr Phe Leu Ser Met Ala Val Trp Arg Asp Tyr
                   85                  90                  95
   Ile Thr Leu Glu Ser His Asp Leu Ala Glu Gly Tyr Thr Tyr Phe Phe
               100                 105                 110
   Asn Glu Asn Thr Asp Asp Cys Tyr Val Leu Lys Gln Asp Phe Ile Asn
           115                 120                 125
   Asn Glu Arg Tyr Glu Lys Thr Glu Leu Tyr Ser Gln Lys Asp Lys Val
       130                 135                 140
   Ile Leu Phe Pro Lys Phe Gly Glu Tyr Asp Leu Val Leu Asn Pro Asp
   145                 150                 155                 160
   Ile Ile
(2) INFORMATIONS POUR LA SEQ ID NO: 43:
   (i) CARACTERISTIQUES DE LA SEQUENCE:
      (A) LONGUEUR: 90 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Peptide
      (B) EMPLACEMENT:1..90
   (xi) DESCRIPTION DE LA SEQUENCE: SEQ ID NO: 43:
   Met Asn Lys Arg Met Lys Met Cys Pro Ala Cys Gln Gln Gly Tyr Leu
   1               5                   10                  15
   Tyr His Ser Lys Pro Lys Tyr Leu His Asp Glu Ile Ile Leu Cys Asp
               20                  25                  30
   Glu Cys Asp Ala Val Trp Leu Lys Gly Met Asn Ile Phe Tyr Gly Glu
           35                  40                  45
   Tyr Glu Lys Asp Phe Tyr Ser Tyr Val Pro Phe Met Glu Ser Gln Gly
       50                  55                  60
   Ile Thr Ser Glu Cys Ile Trp Glu Gly Asp Leu Phe Asp His Pro Tyr
   65                  70                  75                  80
   Tyr Glu Asp Glu Asn Ser Asn Asp Met Asp
                   85                  90
(2) INFORMATIONS POUR LA SEQ ID NO: 44:
   (i) CARACTERISTIQUES DE LA SEQUENCE:
      (A) LONGUEUR: 313 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Peptide
      (B) EMPLACEMENT:1..313
   (xi) DESCRIPTION DE LA SEQUENCE: SEQ ID NO: 44:
   Met Ser Ala Thr Glu Ile Glu Lys Ala Lys Ala Lys Ile Thr Ala Tyr
   1 5 10 15
   Ser Lys Leu Val Ala Gly Thr Ala Ser Ala Val Val Gly Gly Asp Val
   20 25 30
   Asn Thr Ala Ala Asn Ala Ala Gln Ile Ala Val Glu Asn Asn Thr Leu
   35 40 45
   Tyr Pro Arg Cys Val Gly Ala Lys Cys Asp Glu Phe Gln Lys Glu Gln
   50 55 60
   Gln Lys Trp Ile Arg Glu Asn Pro Glu Glu Tyr Arg Glu Val Leu Leu
   65 70 75 80
   Phe Gln Thr Gly Phe Ile Pro Ile Ile Gly Asp Ile Gln Ser Phe Val
   85 90 95
   Gln Ala Gln Thr Ala Ala Asp His Leu Phe Ala Leu Leu Gly Val Val
   100 105 110
   Pro Gly Ile Gly Glu Ser Ile Gln Ala Tyr Lys Val Ala Lys Ala Ala
   115 120 125
   Lys Asn Leu Gln Gly Met Lys Lys Ala Leu Asp Lys Ala Ala Thr Val
   130 135 140
   Ala Thr Ala Gln Gly Tyr Val Ser Lys Thr Lys Ile Lys Ile Gly Gln
   145 150 155 160
   Thr Glu Leu Arg Val Thr Ala Ala Thr Asp Lys Gln Leu Leu Lys Ala
   165 170 175
   Ile Gly Glu Gly Arg Asp Thr Thr Gly Lys Met Thr Glu Gln Leu Phe
   180 185 190
   Asp Ser Leu Ala Lys Gln Asn Gly Phe Arg Val Leu Ser Gly Gly Lys
   195 200 205
   Tyr Gly Gly Asn Asn Gly Phe Asp His Val Trp Gln Ala Ala Asp Gly
   210 215 220
   Ser Val Val Leu Ile Val Glu Ser Lys Gln Ile Arg Asn Gly Thr Val
   225 230 235 240
   Gln Leu Asn Pro Asn Gly Ala Gly Gly Tyr Thr Gln Met Ser Glu Asp
   245 250 255
   Trp Ile Arg Gln Val Leu Asp Gln Leu Pro Asp Gly Ser Pro Ala Lys
   260 265 270
   Ala Ala Val Phe Lys Ala Asn Lys Asn Gly Thr Leu Lys Thr Ala Ile
   275 280 285
   Ala Gly Val Asp Arg Gln Thr Gly Lys Ala Val Ile Leu Pro Val Lys
   290 295 300
   Val Pro Ser Lys Thr Asn Ile Arg Arg
   305 310
(2) INFORMATIONS POUR LA SEQ ID NO: 45:
   (i) CARACTERISTIQUES DE LA SEQUENCE:
      (A) LONGUEUR: 311 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: Peptide
      (B) EMPLACEMENT:1..311
   (xi) DESCRIPTION DE LA SEQUENCE: SEQ ID NO: 45:
   Met Gly His Asn Met Met Thr Thr Gln Lys Trp Tyr Glu His Ile Thr
   1 5 10 15
   Asn Val Ile Ile Gly Asn Thr Ala Asn Phe Asn Ser Gly Cys Leu Asp
   20 25 30
   Ser Ile Asp Tyr Val Asp Glu Arg Lys Gly Val Pro Leu Ala Ala Met
   35 40 45
   Gln His Ile Phe Met Asp Val Arg Ala Ala Ala Ser His Ala Tyr Leu
   50 55 60
   Phe Glu His Asp Leu Lys Lys Phe Lys Gln Tyr Ala Tyr Val Ala Gly
   65 70 75 80
   Lys Leu Gly Val Leu Leu Ser Val Asn Ser Thr Asp Pro Glu Pro Phe
   85 90 95
   Phe Phe Pro Cys Asp Met Leu Asn Ile Gln Asn Pro Met Phe Leu Met
   100 105 110
   Leu Met Ser Asp Ser Pro Gln Leu Arg Glu Phe Leu Val Arg Asn Ile
   115 120 125
   Asp Asn Ile Ala Asn Asp Thr Glu Ala Phe Ile Asn Arg Tyr Asp Leu
   130 135 140
   Asn Arg His Met Ile Tyr Asn Thr Leu Leu Met Val Glu Gly Lys Gln
   145 150 155 160
   Leu Asp Arg Leu Lys Gln Arg Ser Glu Lys Val Leu Ala His Pro Thr
   165 170 175
   Pro Ser Lys Trp Leu Gln Lys Arg Leu Tyr Asp Tyr Arg Phe Phe Leu
   180 185 190
   Ala Phe Ala Glu Gln Asp Ala Glu Ala Met Lys Ala Ala Leu Glu Pro
   195 200 205
   Leu Phe Asp Lys Lys Thr Ala Arg Met Ala Ala Lys Glu Thr Leu Ser
   210 215 220
   Tyr Phe Asp Phe Tyr Leu Gln Pro Gln Ile Val Thr Tyr Ala Lys Ile
   225 230 235 240
   Ala Ser Met His Gly Phe Asp Leu Gly Ile Asp Gln Glu Ile Ser Pro
   245 250 255
   Arg Asp Leu Ile Val Tyr Asp Pro Leu Pro Ala Asp Glu Tyr Gln Asp
   260 265 270
   Ile Phe Asp Phe Met Lys Gln Tyr Asp Leu Ser Tyr Pro Tyr Glu Tyr
   275 280 285
   Leu Gln Asp Trp Ile Asp Tyr Tyr Thr Phe Lys Thr Asp Lys Leu Val
   290 295 300
   Phe Gly Asn Ala Lys Arg Glu
   305 310
(2) INFORMATIONS POUR LA SEQ ID NO: 46:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (il) TYPE DE MOLECULE: ADN (génomique)
   (xi) DESCRIPTION DE LA SEQUENCE: SEQ ID NO: 46:
      GCCACCGGTA CGGAAACTGA A 21
(2) INFORMATIONS POUR LA SEQ ID NO: 47:
   (i) CARACTERISTIQUES DE LA SEQUENCE:
      (A) LONGUEUR: 30 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 47:
      CCTGAATTCA TGTCTATTCC ATTTTGAAGA 30
(2) INFORMATIONS POUR LA SEQ ID NO: 48:
   (i) CARACTERISTIQUES DE LA SEQUENCE:
      (A) LONGUEUR: 31 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 48:
      CCGAGATCTT TAACCCTTTG GGCTTAAGCG A 31
(2) INFORMATIONS POUR LA SEQ ID NO: 49:
   (i) CARACTERISTIQUES DE LA SEQUENCE:
      (A) LONGUEUR: 29 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 49:
      GGGAGATCTC CCGCTCGTGT TGTGCATTA 29
(2) INFORMATIONS POUR LA SEQ ID NO: 50:
   (i) CARACTERISTIQUES DE LA SEQUENCE.
      (A) LONGUEUR: 28 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 50:
      AAGAGATCTG CAGCCAAGGC TCTCGAAA 28
(2) INFORMATIONS POUR LA SEQ ID NO: 51:
   (i) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 51:
      GGGAGATCTC AGGCTGCCGC CGTTGA 26
(2) INFORMATIONS POUR LA SEQ ID NO: 52:
   (i) CARACTERISTIQUES DE LA SEQUENCE:
      (A) LONGUEUR: 28 paires de bases
      (B) TYPE: nucléotide
      (C) NOMBRE DE BRINS: simple
      (D) CONFIGURATION: linéaire
   (ii) TYPE DE MOLECULE ADN (génomique)
   (iii) HYPOTHETIQUE: NON
   (iv) ANTI-SENS: NON
   (xi) DESCRIPTION DE LA SEQUENCE: SEQ ID NO: 52:
      GGGAGATCTC ACCCCAAGAA CGCCAAAA 28
(2) INFORMATIONS POUR LA SEQ ID NO: 53:
   (i) CARACTERISTIQUES DE LA SEQUENCE:
      (A) LONGUEUR: 31 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON (xi) DESCRIPTION DE LA SEQUENCE: SEQ ID NO: 53:
      GGGAGATCTG AACGTATAGT AATCTATCCA A 31
(2) INFORMATIONS POUR LA SEQ ID NO: 54:
   (i) CARACTERISTIQUES DE LA SEQUENCE:
      (A) LONGUEUR: 12 paires de bases
      (B) TYPE: nucléotide
      (C) NOMBRE DE BRINS: simple
      (D) CONFIGURATION: linéaire
   (il) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 54:
      AGTGGCTCCT AG 12
(2) INFORMATIONS POUR LA SEQ ID NO: 55:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 55:
      AGCACTCTCC AGCCTCTCAC CGAG 24
(2) INFORMATIONS POUR LA SEQ ID NO: 56:
   (i) CARACTERISTIQUES DE LA SEQUENCE:
      (A) LONGUEUR: 12 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 56:
      AGTGGCTCTT AA 12
(2) INFORMATIONS POUR LA SEQ ID NO: 57:
   (i) CARACTERISTIQUES DE LA SEQUENCE:
      (A) LONGUEUR: 10 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 57:
      AGTGGCTGGC 10
(2) INFORMATIONS POUR LA SEQ ID NO: 58:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO:58:
      AGCACTCTCC AGCCTCTCAC CGAC 24
(2) INFORMATIONS POUR LA SEQ ID NO: 59:
   (i) CARACTERISTIQUES DE LA SEQUENCE:
      (A) LONGUEUR: 12 paires de bases
      (B) TYPE: nucléotide
      (C) NOMBRE DE BRINS: simple
      (D) CONFIGURATION: linéaire
   (ii) TYPE DE MOLECULE: ADN (genomique)
   (iii) HYPOTHETIQUE: NON
   (iv) ANTI-SENS: NON
   (xi) DESCRIPTION DE LA SEQUENCE: SEQ ID NO: 59:
      GTACTTGCCT AG 12
(2) INFORMATIONS POUR LA SEQ ID NO: 60:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 60:
      ACCGACGTCG ACTATCCATG AACG 24
(2) INFORMATIONS POUR LA SEQ ID NO: 61:
   (i) CARACTERISTIQUES DE LA SEQUENCE.
      (A) LONGUEUR: 12 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE. SEQ ID NO: 61:
      GTACTTGCTT AA 12
(2) INFORMATIONS POUR LA SEQ ID NO: 62:
   (i) CARACTERISTIQUES DE LA SEQUENCE:
      (A) LONGUEUR: 10 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 62:
      GTACTIGGGC 10
(2) INFORMATIONS POUR LA SEQ ID NO: 63:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 63:
      ACCGACGTCG ACTATCCATG AACC 24
(2) INFORMATIONS POUR LA SEQ ID NO: 64
   (i) CARACTERISTIQUES DE LA SEQUENCE:
      (A) LONGUEUR: 12 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE NON
   (iv) ANTI-SENS: NON
   (xi) DESCRIPTION DE LA SEQUENCE: SEQ ID NO: 64:
      AATTCTCCCT CG
(2) INFORMATIONS POUR LA SEQ ID NO: 65
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 65:
      AGGCAACTGT GCTATCCGAG GGAG
(2) INFORMATIONS POUR LA SEQ ID NO: 66:
   (i) CARACTERISTIQUES DE LA SEQUENCE:
      (A) LONGUEUR: 140 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 66:
   GATCAACTTT TCCCTGTTTG TCCCATTACC GGTTTGAATG AACCGATTGC GCGCCGCGCG 60
   TGTTGTTGGA CATTACCTGC GATTCAGACG GTACGATTGA CCACTACATC GAGGAGAACG 120
   GCAATCAGGG TACAATGCTA 140
(2) INFORMATIONS POUR LA SEQ ID NO: 67:
   (i) CARACTERISTIQUES DE LA SEQUENCE.
      (A) LONGUEUR: 192 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 67:
   GATCCGCGTA CTTGGTTTTT CATATTTTGC ATAGTCTTGT CGGTCGGGCA TCTTCCCCGA 60
   CATCATCTAA ATTTGTCTTT ATTGGTTTTT ACGCCACTCA TTGCGGATAA ACAATATTCC 120
   GCCTTGCCGT CGCGAATGTT CAAGCTAGCC TGCATCACCG TAATCAGGTT GCCCGTTACC 180
   GAGCCTTCGA GA 192
(2) INFORMATIONS POUR LA SEQ ID NO: 68:
   (i) CARACTRISTIQUES DE LA SEQUENCE:
      (A) LONGUEUR: 188 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 68:
   GATCCGGCTG CCCGACGCGC GCAAAATTGC CGCCGAGGAA AGCGCGCACA ACCACGACGG 60
   CAAAACCAGC GTATGGCAAT ACAAACATCT CGTGTTCGGT ACGGCAGGCA TTTTCTGCTA 120
   TGTCGGCGCG GAGGTGTCTA TCGGTTCGTT GATGGTCAAC GTATTGGGTT ATCTGAAAGG 180
   GCTGGATC 188
(2) INFORMATIONS POUR LA SEQ ID NO 69:
   (i) CARACTERISTIQUES DE LA SEQUENCE:
      (A) LONGUEUR: 304 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 69:
   GATCCCCCAC TTTACCTCGG GCAGATTTTG CGCGTTCATT ACAATAGCGT ATTTATGCGT 60
   TTGCGTTTGC GCTTGCCGCT GCCCCCCCCC CGCCGGTATG GGAAAACATC AATATGGCGG 120
   TATAAAGCGC GGTATGGCGG AAAACCTGCC GTTTCCAAGT TTTATTCATC TTTTATTCCT 180
   TGAGTTTGCC TTCACGGGAC GGGGCGGCGC GCGGAACGCG GGGTTCGGTA AACCGCCCGA 240
   TTCCGCGCCC GCCGAATTGC TGATTGAAAA GCTTACTTCC CCATTTTAAC TTTGCACACT 300
   GATC 304
(2) INFORMATIONS POUR LA SEQ ID NO: 70:
   (i) CARACTERISTIQUES DE LA SEQUENCE.
      (A) LONGUEUR: 243 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS. NON
   (xi) DESCRIPTION DE LA SEQUENCE: SEQ ID NO: 70:
   GATCAGACCC ATTTTCAGCG CACCGTAAGC GCGGATTTTC TCGAATTTTT CCAAAGCTGC 60
   GGCATCGTTG TTGATGTCGT CTTGCAACTC TTTGCCCGTG TAGCCCAAGT CGGCGGCATT 120
   CAGGAAAACG GTCGGAATGC CCGCGTTGAT GAGCGTGGCT TTCAAACGGC CTATATTCGG 180
   CACATCAATT TCATCGACCA AATTGCCGGT TGGGAACATA CTGCCTTCGC CGTCGGCTGG 240
   ATC 243
(2) INFORMATIONS POUR LA SEQ ID NO: 71
   (i) CARACTERISTIQUES DE LA SEQUENCE.
      (A) LONGUEUR: 236 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 71:
(2) INFORMATIONS POUR LA SEQ ID NO: 72:
   (i) CARACTERISTIQUES DE LA SEQUENCE:
      (A) LONGUEUR: 280 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE NON
   (iv) ANTI-SENS: NON
   (xi) DESCRIPTION DE LA SEQUENCE SEQ ID NO: 72:
   CGGTCAATCA CAAGAAAGTC AGCCGTCTGA TGGCGAAGAC GGGGCTGAAG GCAGTGATAT 60
   GGCGGCGCAA ATACCGCTCG TTCAAAGGAG AAGTCGGCAA AATTGCGCCG AATATCCTGC 120
   GACGCTGTTT CCATGCAGAA AAGCCGAATG AGAAATGGGT AACGGACGTT GCCGAGTTCA 180
   ATGTAGGCGG AGAAAAGATA TACCTTTCTC CGATTATGGA TTTGTTTAAC GGGGAAATCG 240
   TCAGTTACCG TATTCAGACC CGCCCGACTT TCGATTTGGC 280
(2) INFORMATIONS POUR LA SEQ ID NO: 73:
   (i) CARACTERISTIQUES DE LA SEQUENCE:
      (A) LONGUEUR: 120 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 73:
   CGGTCAGAAA CAGGCAAGGT AATGAAAATG CCTGAGGCAC GGACTGTGCT GCGAACGAAA 60
   ACTCCTTACC GAAGTCTTCT ATACCCAGGC TCAATAGCCG CTCAAGGAGA GAGCTATCAT 120
(2) INFORMATIONS POUR LA SEQ ID NO: 74:
   (i) CARACTERISTIQUES DE LA SEQUENCE:
      (A) LONGUEUR: 120 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS NON
   (xi) DESCRIPTION DE LA SEQUENCE. SEQ ID NO: 74
   CGGTCAGAAA CAGGCAAGGT AATGAAAATG CCTGAGGCAC GGACTGTGCT GCGAACGAAA 60
   ACTCCTTACC GAAGTCTTCT ATACCCAGGC TCAATAGCCG CTCAAGGAGA GAGCTATCAT 120
(2) INFORMATIONS POUR LA SEQ ID NO: 75.
   (i) CARACTERISTIQUES DE LA SEQUENCE:
      (A) LONGUEUR: 152 paires de bases
      (B) TYPE: nucléotide
      (C) NOMBRE DE BRINS: simple
      (D) CONFIGURATION linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE. SEQ ID NO: 75:
   CGGTGTTTTT CTTAACAATT CGCCGACTTC ATGGCGATAT TTAAGTGACA GTTGCTCCGC 60
   CCACGCAGTT GCGCCGAACT CAGCACCACG ACATTATACT GATTATGCAC ATCGGCAAGA 120
   TCAAACTGAC CTATCGTAGT ATCGCAGACT GT 152
(2) INFORMATIONS POUR LA SEQ ID NO 76
   (i) CARACTERISTIQUES DE LA SEQUENCE:
      (A) LONGUEUR: 381 paires de bases
      (B) TYPE: nucléotide
      (C) NOMBRE DE BRINS: simple
      (D) CONFIGURATION: linéaire
   (ii) TYPE DE MOLECULE. ADN (génomique)
   (iii) HYPOTHETIQUE: NON
   (iv) ANTI-SENS: NON
   (xi) DESCRIPTION DE LA SEQUENCE: SEQ ID NO: 76
(2) INFORMATIONS POUR LA SEQ ID NO: 77
   (i) CARACTERISTIQUES DE LA SEQUENCE:
      (A) LONGUEUR. 269 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 77:
(2) INFORMATIONS POUR LA SEQ ID NO: 78
   (i) CARACTERISTIQUES DE LA SEQUENCE:
      (A) LONGUEUR: 203 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 78:
(2) INFORMATIONS POUR LA SEQ ID NO: 79:
   (i) CARACTERISTIQUES DE LA SEQUENCE:
      (A) LONGUEUR: 229 paires de bases
      (B) TYPE: nucléotide
      (C) NOMBRE DE BRINS: simple
      (D) CONFIGURATION: linéaire
   (il) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 79
   CGGTTTCAGG TTGTCGCGAA GGCTCGGTAA CGGGCAACCT GATTACGGGT GATGCAGGCA 60
   GCTTGAACAT TCGCGACGGC AAGGCGGAAT ATGTTTATCC GCAATGAGTG GCGTAAAAAC 120
   CAATAAAGAC AAATTTAGAT GATGTCGGGG AAGATGCCCG ACCGACAAGA CTATGCAAAA 180
   TATGAAAAAC CAAGTACGCG GATCAGGCAT GGATGCACGA TCCAATCCG 229
(2) INFORMATIONS POUR LA SEQ ID NO: 80:
   (i) CARACTERISTIQUES DE LA SEQUENCE:
      (A) LONGUEUR: 207 paires de bases
      (B) TYPE: nucléotide
      (C) NOMBRE DE BRINS. simple
      (D) CONFIGURATION: linéaire
   (ii) TYPE DE MOLECULE: ADN (genomique)
   (iii) HYPOTHETIQUE: NON
   (iv) ANTI-SENS: NON
   (xi) DESCRIPTION DE LA SEQUENCE: SEQ ID NO: 80:
   CGGGTCGCTT TATTTTGTGC AGGCATTATT TTTCATTTTT GGCTTGACAG TTTGGAAATA 60
   TTGTGTATCG GGGGGGGGTA TTTGCTGACG TAAAAAACTA TAAACGCCGC GCAAAATATG 120
   GCTGACTATA TTATTGACTT TGATTTTGTC CTGCGCGGTG ATGGATAAAA TCGCCAGCGA 180
   TAAAGAATTT GCGAGAACCT GATGCCG 207
(2) INFORMATIONS POUR LA SEQ ID NO: 81
   (i) CARACTERISTIQUES DE LA SEQUENCE:
      (A) LONGUEUR: 224 paires de bases
      (B) TYPE: nucléotide
      (C) NOMBRE DE BRINS: simple
      (D) CONFIGURATION: linéaire
   (ii) TYPE DE MOLECULE. ADN (génomique)
   (iii) HYPOTHETIQUE: NON
   (iv) ANTI-SENS: NON
   (xi) DESCRIPTION DE LA SEQUENCE: SEQ ID NO: 81:
   CGGCAACGAT TTGAGCTATC GCGGTTACGA CATTCTGGAT TTGGCACAAA AATGCGAGTT    60
   TGAAGAAGTC GCCCACCTGC TGATTCACGG CCATCTGCCC AACAAATTCG AGCTGGCCGC   120
   TTATAAAACC AAGCTCAAAT CCATGCGCGG CCTGCCTATC CGTGTGATTA AAGTTTTGGA   180
   AAGCCTGCCT GCACATACCC ATCCGATGGA CGTAATGCGT ACCG                    224
(2) INFORMATIONS POUR LA SEQ ID NO: 82:
   (i) CARACTERISTIQUES DE LA SEQUENCE
      (A) LONGUEUR: 212 paires de bases
      (B) TYPE: nucléotide
      (C) NOMBRE DE BRINS: simple
      (D) CONFIGURATION linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE. SEQ ID NO: 82:
   CGGGAACAGC CATTGCCCAC GCCCACGCCC CCCAAGAAAG ACGGAAACTA CTGCCTAAAT    60
   TTTCGGCAAT CAAGTTGACG ATTAAAGGGT TGGGGGCAGT TGCAGTAATA AACATAGCCG   120
   ACGAAATGGG ATTGGAATGA TAGTTGACCA AAGCCAAATA TTTACCCATC TTGCCTTCTG   180
   TGCCTTTTGC GGGATTGGAG CCGTAACTGC CG                                 212
(2) INFORMATIONS POUR LA SEQ ID NO: 83
   (i) CARACTERISTIQUES DE LA SEQUENCE.
      (A) LONGUEUR: 353 paires de bases
      (B) TYPE: nucléotide
      (C) NOMBRE DE BRINS: simple
      (D) CONFIGURATION: linéaire
   (ii) TYPE DE MOLECULE ADN (génomique)
   (iii) HYPOTHETIQUE: NON
   (iv) ANTI-SENS: NON
   (xi) DESCRIPTION DE LA SEQUENCE: SEQ ID NO: 83
   CGGGAATTCT GAGCAGAATG AAAGAAAGCA GGCTTGATAA TTTCATAAAG TTATTGGAAG 60
   AAAAAGGATT TACCGTCCAT TTCGGTATTC ACAATACGGC TGATTACGGA ATTCCCCAAA 120
   GCCGTAAAAG ATTTACGTTA ATTGCAAACA GAATAACCAA AGAAAAGCTG GAACCAGTCA 180
   AGTATTCGGG CAAACGGCTT ACGGTAGCCG ATGTTTTGGG AATGGAAATG GCTTTCCCAA 240
   CATTATTGCA GGACACCAAG ACGAAACGGA TTTTATGCAT AGCTGTGCGG GAATTATCTG 300
   ATATCACTTG AACGATTGGC TTGATACCTA AAAACGGAGG AACCGTTGGC TTT 353
(2) INFORMATIONS POUR LA SEQ ID NO: 84:
   (i) CARACTERISTIQUES DE LA SEQUENCE:
      (A) LONGUEUR: 308 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 84:
   AATTCCGTAT CCAAACTTTG CGGGTTAGAT AAAGGGGTGT AGTCTGTCCC GCTTATGGTA 60
   ACGCTGTCGC GGCGGACTAC GCCCGGAGCC TTTTTCCAGT AAGTTTTCGG AAATCAGGCT 120
   GTGGGTGGTT TTTAAGAAAT CCAACCAGTC AAACGGCTCG GGGCTGTCCA AACCGGACAC 180
   AGGTGCCGGT AACTTTCCCT CAGGTTGATT AACATTACGG CATCCGAATA TAACTTCCCG 240
   CCTGCGGTTT GCCCGAGTTT AAGCAATGCC TGCGTATCGT ATTGATTATA AAGTGTTTCC 300
   TTCCAATT 308
(2) INFORMATIONS POUR LA SEQ ID NO: 85:
   (i) CARACTERISTIQUES DE LA SEQUENCE:
      (A) LONGUEUR: 104 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS. NON
   (xi) DESCRIPTION DE LA SEQUENCE: SEO ID NO: 85:
   AATTCGTGTG CCGCGTCGAC AAACCGCTGA CGTAGCGGAT GTCTCATGCC ACGTTTCAAA 60
   GCAGGTTGAT GGCGGTTAGC AACCCTCTGA TTTCACTGGG ATAT 104
(2) INFORMATIONS POUR LA SEQ ID NO: 86:
   (i) CARACTERISTIQUES DE LA SEQUENCE:
      (A) LONGUEUR: 89 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS NON
   (xi) DESCRIPTION DE LA SEQUENCE. SEQ ID NO: 86:
   AATTGCGTAG AGTGGGCTTC AGCCACGTTT TTTCTTTTTC GGTCGTTGAT TGGTGGGCTG 60
   AACCACTTGT TTCGGAAATC CGTATCATG 89
(2) INFORMATIONS POUR LA SEQ ID NO: 87:
   (i) CARACTERISTIQUES DE LA SEQUENCE.
      (A) LONGUEUR: 273 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 87:
   AATTTCCACC TATGCCCTAC GCAGCGATTA TCCGTGGTTT ACCCAAAGGG TGATTATGGC 60
   AAAAGCGCGG GGTTGAGCGA CCGCCTTTTG TTGCCGGCGT TCAAACGGGT TTTGATAGGA 120
   AATGCAGGCA CGAAGCCTCG GCTGATTGTG ATGCACCTGA TGGGTTCGCA CAGTGATTTT 180
   TGCACACGTT TGGATAAGGA TGCGCGGCGG TTTCAGTATC AAACTGAAAA AATATCCTGC 240
   TATGTTTCCA TCAATCGCGC AAACCGATAA ATT 273
(2) INFORMATIONS POUR LA SEQ ID NO: 88:
   (i) CARACTERISTIQUES DE LA SEQUENCE:
      (A) LONGUEUR: 270 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 88:
   AATTCTTCCG CACGGGGAGG CTTGTTTTTC TTCCCTTCTG TTCCGACCGA TTCTCAAATA 60
   AAAATCATTG ATTTCATCGA AGTTCATTCC TATACCATTA TCTTTAATAA CGATTTTATG 120
   CTCCGGTTTA TCGAATAACC TAACTTCCAC TTCCGTAGCA CATGCATCGT AGGCATTCGC 180
   TATCAACTCG GCAATCGCAG GAACAGTGTG CGAATACAAT CTTTACACCC AAATGTTCGA 240
   TTACGGTTGG CTCGAAACTC AATTTCAATT 270
(2) INFORMATIONS POUR LA SEQ ID NO: 89:
   (i) CARACTERISTIQUES DE LA SEQUENCE:
      (A) LONGUEUR: 267 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 89:
   AATTATGAAC ACACGCATCA TCGTTTCGGC TGCGTTCGTT GCGTTGGCAT TAGCAGGTTG 60
   CGGCTCAATC AATAATGTAA CCGTTTCCGA CCAGAAACTT CAGGAACGTG CCGCGTTTGC 120
   CTTGGGCGTC ACCAATGCCG TAAAAATCAG CAACCGCAGC AATGAAGGCA TACGCATCAA 180
   CTTTACCGCA ACTGTGGGTA AGCGCGTGAC CAATGCTATG TTACCAGTGT AATCAGCACA 240
   ATCGGCGTTA CCACTTCCGA TGCAATT 267
(2) INFORMATIONS POUR LA SEQ ID NO: 90:
   (i) CARACTERISTIQUES DE LA SEQUENCE:
      (A) LONGUEUR: 234 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 90:
   AATTTTTATT TGGTTCGTAG TCATTTTGTG CAACTGAACG ATATTCGTTT TCATCATTGC 60
   TAACGTCTAG TGCCCATTGT GGCCCGTAAT AAGAGATTTC GTCTCCTTTT ACATGTTTGA 120
   CGCTGACGGC ATACTGGGGA TCGATGACGG ATAATGTACG TCTGTTGACA TCTGCAACGC 180
   TAAATCAATC ATCGGTATTG GATAATGCGT TGCCGATGTT TTGACTTGTA TGTT 234
(2) INFORMATIONS POUR LA SEQ ID NO: 91:
   (i) CARACTERISTIQUES DE LA SEQUENCE:
      (A) LONGUEUR: 295 paires de bases
      (B) TYPE: nucléotide
      (C) NOMBRE CE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 91:
   AATTCGGCCG GCTGTGTCAA ATAATGCGTT ACTTTGGCCG GGTCTTGTTC TTTGTAAGTG 60
   GTGGTCTTTT TTTGCGCGTT ATCCCCATCT GTTTGAGTGC ATAGCAAATG GTGGCTGCCG 120
   TACAATCAAA TGTTTGGCGT TCATGCAGAT AGGCATCATG GTGTTGCCCA ATATATTGAG 180
   CCGGTTTTTG CCTATCCGAT TTGACGGCAT TTAGACCGGT AACTTGATGT TTTAAGCTGC 240
   CTGTTTGTTT AAAGGCGAAT CCACAAGTAA AGCGTGTTTC TTGACAGGTT AAACG 295
(2) INFORMATIONS POUR LA SEQ ID NO: 92:
   (i) CARACTERISTIQUES DE LA SEQUENCE:
      (A) LONGUEUR: 259 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 92:
   AATTGTGTAT ATCAAGTAGG ATGGGCATTT ATGCCTGACC TACAAAACCA AAAACAACCT 60
   ACCACCCTTA ATCAACTCCA CAAACCCTCT TCAGACAACC TCGTTTTTTG AAAAACAATC 120
   TGTAAACAGA TAACTGCTGA AGAATACCGT TGCCGAGCCC CAAAACCCGT ACTGCAACTT 180
   TTATTGTGAA CTTCCCATTA TGAGAAAATC CCTTTTCGTC CTCTTTCTGT ATTCGTCCCT 240
   ACTTACTGCC AGCGAAATT 259
(2) INFORMATIONS POUR LA SEQ ID NO: 93:
   (i) CARACTERISTIQUES DE LA SEQUENCE:
      (A) LONGUEUR: 379 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 93:
   AATTGCACCA CGCGATGATG GGTACGCCTC TGTTGCCATT GCGACCGCCG CCGCCGTGCC 60
   CGGTACGCTG GTCAACCTTG CCGCGGCGGA ACGGGTAAAG AAGTGCGCTT CGGGCATCCT 120
   TCCGGTACAT TGCGCGTCGG TGCAGCGCCG AATGTCAGGA CGGACAATGG ACGGCCACCA 180
   AAGCGGTTAT GAGCCGCAGC GCACGCGTGA TGATGGAAGG TTGGGTCAGG GTGCCGGAAG 240
   ATTGTTTTTA AATTGGACGG CGAACCGGTC TATTCGTATT GGCGTTATAC CGCCGCAAAG 300
   GCAGACCTTG AAACTGGTGC GTGCCGTGCA GGGCATGTAC GGCTATGTGT GCGTGGCGGG 360
   CGGATTTGAT GTGCGGAAT 379
(2) INFORMATIONS POUR LA SEQ ID NO: 94:
   (i) CARACTERISTIQUES DE LA SEQUENCE:
      (A) LONGUEUR: 308 paires de bases
      (B) TYPE: nucléotide
      (C) NOMBRE DE BRINS: simple
      (D) CONFIGURATION: linéaire
   (il) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 94:
   AATTTGTTGG GCAGATGGCC GTGAATCAGC AGGTGGGCGA CTTCTTCAAA CTCGCATTTT 60
   TGTGCCAAAT CCAGAATGTC GTAACCGCGA TACGTCAAAT CGTTGCCGGT ACGCAACGGT 120
   ACACAAAGCG GTATTACCGG CCGCAACGCC AGAAAGCGCA ACGGATTTTT AGGTTTGAGG 180
   GTCGGGGTTT GAGTAGTTTC AGTCATGGTA TTTCTCCTTT GTGTTTTTAT GGGTTTCGGG 240
   TTTTCAGACG ACCGATGCGG ATTTGTTGAA AGGCAGTCTG AAAGCGGTAA ATCATTTTTG 300
   AAACAATT 308
(2) INFORMATIONS POUR LA SEQ ID NO: 95:
   (i) CARACTERISTIQUES DE LA SEQUENCE:
      (A) LONGUEUR: 286 paires de bases
      (B) TYPE. nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEO ID NO: 95:
   AATTCGGAGG AGCAGTACCG CCAAGCGTTG CTCGCCTATT CCGGCGGTGA TAAAACAGAC 60
   GAGGGTATCC GCCTGATGCA ACAGAGCGAT TACGGCAACT TGTCCTACCA CATCCGTAAT 120
   AAAAACATGC TTTTCATTTT TTCGGCAAGC AATGACGCAC AAGCTCAGCC CAACACAACT 180
   GACCCTATTG CCATTTTATG AAAAAGACGC TCAAAAAGGC ATTATCACAG TTGCAGGCGT 240
   AGACCGCAGT GGAGAAAAGT TCAATGGCTC CAACCATTGC GGAATT 286
(2) INFORMATIONS POUR LA SEQ ID NO: 96:
   (i) CARACTERISTIQUES DE LA SEQUENCE:
      (A) LONGUEUR: 238 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 96:
   AATTTGGATA CGTTGGAAAA GGGATATTTG ATTGGGAATG GGATGAAGAT AAGCGTAGAT 60
   GAGTTGGGGA AAAAAGTGTT AGAACATATC GGTAAGAATG AACCGTTATT GTTGAAAAAT 120
   CTACTGGTTA ACTTCAATCA GGGAAAACAT GAAGAAGTTA GGAAGTTGAT TTATCAGTTG 180
   ATAGAGTTAG ATTTTCTGGA ACTTTTGTGA GGGATTCTAT GAAAAACTGG AAGCAATT 238
(2) INFORMATIONS POUR LA SEQ ID NO: 97
   (i) CARACTERISTIQUES DE LA SEQUENCE:
      (A) LONGUEUR: 322 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 97:
   AATTCGGCAC GCAGGTTTTC TAAAAAAAGG CCGTTGATGA CTTTGTCGAT ATTGGCGGCT 60
   TCGGTGTAGT GCGCGCCCGC TTCGGCCGCT CTTGCGCGTC CATGACGGAT TGGAAGAGCG 120
   TGCCGAAGAT TTCTGGACTG ATGTTGCGCC AGTCGAAATT GCCGACACGG GAGGAATACC 180
   TGCCAACAAG AGTGCAGGCA GCGTAATCAA ACCACCCCCA CCCGCAATCG CATCGATAAA 240
   TCCGGCAATC ATCGCAACCA AACCCAAAGC GAGTATTATG TATAAATCTT CCATGTTTCT 300
   TAATCCTGTT AACTTGCACC AA 322
(2) INFORMATIONS POUR LA SEQ ID NO. 98.
   (i) CARACTERISTIQUES DE LA SEQUENCE:
      (A) LONGUEUR: 316 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 98
   AATTTGTCGG CAATCTTCCC GGGTCGCTTT ATTTTGTGCA GGCATTATTT TTCATTTTTG 60
   GCTTGACAGT TTGGAGATAT TGTGTATCGG GGGGGGGTAT TTGCTGACGT AAAAAACTAT 120
   AAACGCCGCA GCAAAATATG GCTGACTATA TTATTGACTT TGATTTTGTC CTGCGCGGTG 180
   ATGGATAAAA TCGCCAGCGA TAAAGATTTG CGAGAACCTG ATGCCGGCCT GTTGTTGAAT 240
   ATTTTCGACC TGTAATTACG ATTTGGCTTC CGCGCCGGCA CAATATGCCG CCAAGCGGCG 300
   CCCACATTTT GGAAGC 316
(2) INFORMATIONS POUR LA SEQ ID NO: 99:
   (i) CARACTERISTIQUES DE LA SEQUENCE:
      (A) LONGUEUR: 217 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE. SEQ ID NO: 99:
   AATTCGGACA GTATGAATAC AGCGGATTAA TACAAGGTAA GTTCATTACA ACGGAAAAAC      60
   CTTTAAAGAA TAATATGAAA GGTATTACCT TGTTTGCCAA CGGGAATGGT AAATATGCCC     120
   GAGTTTTTCA CTGAATAGCG AATCCAGCCA TTTCTATTCA TATTTGACTG GATGGCTGAA     180
   TGTGGACTTT ATAGATAATG ACGATGAAGA TTTAATT                             217

## Description

L'invention est relative aux ADN, et aux protéines et peptides, spécifiques des bactéries de l'espèce *Neisseria meningitidis* (ci-après en abrégé Nm), à leur procédé d'obtention et à leurs applications biologiques, en particulier pour la détection d'infections à méningocoques et de méningites.

On sait que Nm constitue l'un des principaux agents de la méningite cérébrospinale.

Des études menées aux Etats-Unis ont montré que de 5 à 10% de la population sont porteurs asymptomatiques de souche(s) de Nm. Les facteurs de transmission de Nm sont mal connus. Pour une proportion des personnes infectées, Nm pénètre le flux sanguin, où elle peut provoquer une méningococcémie et/ou progresse dans le flux cérébrospinal pour provoquer une méningite. Sans traitement antibiotique rapide, l'infection peut se développer de manière fulgurante et devenir mortelle.

Comparée aux autres pathogènes, Nm présente la caractéristique de pouvoir franchir la barrière hémato-encéphalique afin de coloniser les méninges. L'étude de la pathogénicité de Nm est donc non seulement importante dans le cadre de la méningite, mais aussi dans le cadre de toute maladie touchant le cerveau.

On conçoit alors l'intérêt de disposer d'outils spécifiques de cette espèce bactérienne pour les applications envisagées ci-dessus.

Nm est génétiquement très proche des bactéries de l'espèce *Neisseria gonorrhoeae* (ci-après en abrégé Ng) et de l'espèce *Neisseria lactamica* (ci-après en abrégé Nl). Leur pathogénicité est toutefois très différente.

Nm colonise le nasopharynx, puis traverse l'épithélium pharyngé pour envahir l'espace sous- muqueux, étant alors responsable de septicémie et de méningite.

Ng est surtout responsable d'infections localisées du tractus génito-urinaire. Elle colonise la muqueuse génitale, puis traverse l'épithélium, envahit ensuite le sous-épithélium où elle se multiplie et est responsable d'une forte réaction inflammatoire. Des infections gonococciques disséminées sont possibles, mais restent rares et sont le fait de seulement certaines souches. Quant à Nl, on considère qu'il s'agit d'une souche non pathogène, étant donné qu'elle n'est pas responsable d'invasion localisée ou générale.

Ainsi, une première considération amène à prendre en compte le fait que Nm et Ng , tout en étant des bactéries très proches, présentent des pouvoirs pathogènes différents.

Le génome de ces bactéries étant fortement homologue, seules des parties limitées du génome de Nm et de Ng doivent coder pour des facteurs de virulence spécifiques, responsables de leur pathogénèse.

Il est clair que Nm présente par rapport à Ng des séquences d'ADN qui lui sont spécifiques et qui doivent intervenir au niveau de l'expression de son pouvoir pathogène spécifique.

L'espèce Nm est subdivisée en sérogroupes basés sur la nature des polysaccharides capsulaires.

Au moins 13 sérogroupes ont été définis, parmi lesquels les sérogroupes A, B et C sont responsables d'environ 90% des cas de méningites. Les groupes A et C sont observés dans les formes épidémiques de la maladie. Le groupe B est le sérogroupe le plus couramment isolé en Europe et aux Etats-Unis.

La capsule, présente chez Nm et absente chez Ng, a servi de base pour l'élaboration de vaccins anti-méningite méningococcique.

Les polysaccharides de la capsule de Nm ont été utilisés pour l'élaboration d'un vaccin qui s'est montré efficace pour prévenir chez les adultes la méningite provoquée par les méningocoques de sérogroupes A, C, W135 et Y.

Cependant, le polysaccharide de Nm groupe C s'est révélé faiblement immunogène chez les enfants de moins de deux ans, alors que le polysaccharide de Nm groupe B est non immunogène chez l'homme et partage des épitopes avec des glycoprotéines d'adhésion présentes dans les cellules neuronales humaines.

Il n'existe donc pas de vaccin universel capable de prévenir les infections provoquées par l'ensemble des sérogroupes des méningocoques et capable de répondre à la variabilité antigénique propre aux pathogènes bactériens en général et à Nm en particulier.

En raison de la réactivité croisée du polysaccharide groupe B de Nm avec les antigènes humains, de la multiplicité des sérogroupes et de la variabilité antigénique de Nm, les stratégies proposées à ce jour ne peuvent conduire à un vaccin efficace dans toutes les situations.

Les recherches se sont alors concentrées sur l'étude d'éléments caractéristiques responsables de la spécificité de la pathogénèse méningococcique.

La plupart des gènes qui ont été étudiés dans l'une quelconque des deux bactéries Nm ou Ng possèdent leur homologue dans la deuxième bactérie.

De la même manière, la plupart des facteurs de virulence jusqu'ici identifiés dans Nm ont une contrepartie dans Ng, c'est-à-dire la piline, les protéines PilC, les protéines d'opacité et les récepteurs de la lactoferrine et de la transferrine.

Les attributs spécifiques des méningocoques caractérisés dans l'art antérieur sont la capsule, les protéines Frp analogues aux toxines RTX, les protéines de la membre externe Opc, la peroxydase glutathione, la porine PorA et le gène rotamase.

Parmi ceux-ci, seule la capsule est invariablement présente dans les souches virulentes de Nm. Cependant, de nombreux pathogènes extra-cellulaires possèdent une capsule sans pour autant traverser la barrière hémato-encéphalique.

Des attributs non encore identifiés doivent donc être responsables de la spécificité de la pathogénèse meningococcale. Ces attributs sont vraisemblablement codés par des séquences d'ADN présentes parmi les méningocoques mais absentes chez les gonocoques.

Les inventeurs ont développé une nouvelle voie d'approche basée sur l'isolement soustractif des gènes Nm-spécifiques, ces gènes devant être liés à la pathogénèse spécifique de Nm, et, plus particulièrement au franchissement de la barrière hémato-encéphalique.

La méthode soustractive développée dans l'art antérieur a abouti à la production de marqueurs épidémologiques pour certains isolats de Nm. Ces marqueurs sont d'une utilité limitée : ils ne couvrent pas l'ensemble des sérogroupes de l'espèce Nm.

Par contraste avec ces études, les travaux des inventeurs ont conduit, en confrontant Nm à l'ensemble du chromosome de Ng, cisaillé de manière aléatoire, à la mise au point de moyens pour cloner l'ensemble des ADN présents chez Nm et absents chez Ng, fournissant ainsi des outils de haute spécificité vis-à-vis de Nm et permettant ainsi de répondre pour la première fois à la variabilité génétique de l'espèce.

Les termes "présent" et "absent", tel qu'utilisés dans la description et les revendications en rapport avec les ADN d'une souche, ou leurs produits d'expression, sont appréciés par rapport à des conditions d'hybridation identiques (16h à 65°C, avec NaPO₄ 0,5M, pH 7,2; EDTA-Na 0,001M, 1% d'albumine de sérum bovin et 7% de dodécylsulfate de sodium, suivies de 2 lavages dans une solution comprenant NaPO₄ 40 mM pH 7,2, EDTA 1mM, et SDS 1%; le lavage final étant réalisé à 65°C pendant 5 min), en utilisant une même sonde et une même intensité de marquage de la sonde, une même quantité d'ADN chromosomique et un même élément de comparaison (ADN chromosomique de la souche homologue).
Ainsi, on considère que l'ADN est présent lorsque le signal obtenu avec la sonde est pratiquement le même que celui obtenu avec la souche de référence.

En revanche, on considère que l'ADN est absent lorsque ce signal apparaît très faible.

Une deuxième considération sur les pathogénicités de Nm et de Ng conduit à prendre en compte leur aptitude commune à coloniser et à pénétrer la muqueuse puis à envahir l'espace sous-épithélial de cette dernière. Il est fort vraissemblable que ce processus implique des facteurs de virulence communs aux deux pathogènes. A cet égard, on sait qu'un certain nombre de facteurs de virulence ont été déjà identifiés chez Nm et chez Ng, comme les protéines pili, PilC, les protéines d'opacité, les protéases d'IgA, les protéines de liaison à la transferrine et à la lactoferrine, et des lipooligosaccharides.

La démarche des inventeurs s'est donc étendue à la recherche de régions de Nm, spécifiques de Nm et de Ng, mais absentes chez l'espèce non pathogène Nl, et d'une manière générale à la recherche, par les moyens mis au point conformément à l'invention, de régions chromosomiques d'ADN et de leurs produits d'expression, spécifiques d'une espèce donnée.

L'invention a donc pour but de fournir un ADN isolé spécifique de Nm ou de Nm + Ng et des moyens pour l'obtenir, notamment en élaborant des banques formées exclusivement de ces ADN Nm-spécifiques.

Elle vise également les produits dérivés de ces séquences d'ADN.

L'invention vise également la mise à profit des caractères spécifique et exhaustif de ces banques pour élaborer des outils utilisables en diagnostic.

### (Rev.1)

L'ADN de l'invention est **caractérisé en ce qu'**il s'agit de
- l'ADN de séquence SEQ ID N°10 ou l'ADN de séquence SEQ ID N°36 ou d'un ADN spécifique de Nm souche Z2491 s'hybridant sur Southern blot avec l'ADN de séquence SEQ ID N°10, mais ne s'hybridant pas sur Southern blot à une séquence d'ADN d'une souche de *Neisseria gonorrhoeae* (ci-après Ng), dans les conditions d'hybridation suivantes:
   16h à 65°C, avec une solution comprenant NaPO₄ 0,5 M pH 7,2, EDTA-Na 0,001 M, 1% d'albumine de sérum bovin et 7% de dodécylsulfate de sodium ; suivies par 2 lavages dans une solution comprenant NaPO₄ 40 mM pH 7,2, EDTA 1mM, et SDS 1% ; le lavage final étant réalisé à 65°C pendant 5 min ;
- ou de l'ADN de séquence SEQ ID N°95, ou d'un ADN ou d'un ADN de Nm souche Z2491 et de Ng s'hybridant sur Southern blot avec l'ADN de séquence SEQ ID N°95, mais ne s'hybridant pas à une séquence d'ADN d'une souche de *Neisseria lactamica* (ci-après Nl), dans les conditions d'hybridation suivantes:
   16h à 65°C, avec une solution comprenant NaPO₄ 0,5 M pH 7,2, EDTA-Na 0,001 M, 1% d'albumine de sérum bovin et 7% de dodécylsulfate de sodium ; suivies par 2 lavages dans une solution comprenant NaPO₄ 40 mM pH 7,2, EDTA 1mM, et SDS 1% ; le lavage final étant réalisé à 65°C pendant 5 min.

### (Rev.2)

L'invention vise également les ADN complémentaires sur toute la longueur des séquences d'ADN telles que définies ci-dessus.

De façon surprenante, la majorité des différences génétiques entre les souches de méningocoques et celles de gonocoques apparaissent regroupées en régions distinctes, qui correspondraient à des ilôts de pathogénécités comme précédemment décrit pour *E. coli* et *Y. pestis.*

Ces régions sont données à titre indicatif mais n'entrent pas dans le champ de l'invention à l'exception des séquences définies plus haut des régions 2 et 4. Les ADN de la région 1 du chromosome comprennent une ou plusieurs séquence(s), telle(s) que présente(s) sur le chromosome de *Neisseria meningitidis* Z2491 entre *tufA* et *pil*T.

Dans la région 1, sont regroupés des gènes de la capsule de *Neisseria meningitidis.*

Des ADN de ce type présentent une séquence correspondant, pour tout ou partie, à SEQ ID N°9, 13, 22 ou 30.D'autres ADN sont constitués par une ou plusieurs séquence(s), telle(s) que présente(s) sur le chromosome de *Neisseria meningitidis* Z2491 entre *pil*Q et λ740, ou région 2 du chromosome..

De tels ADNs présentent une séquence correspondant, pour tout ou partie, à SEQ ID N°1, 2, 4, 6, 7, 10, 15, 31 ou 34. La région 2 comprend la séquence de l'invention SEQ ID N°10 identifiée ci-dessus ainsi que la séquence d'ADN SEQ ID N°36 de 15620 pb.

D'autres ADNs correspondent à une ou plusieurs séquence(s), telle(s) que présente(s) sur le chromosome de *Neisseria meningitidis* Z2491 entre *arg*F et *opa*B, ou région 3 du chromosome.

Des ADNs de cette région présentent une séquence correspondant pour tout ou partie à SEQ ID N°8, 21, 23, 25, 26, 28, 29, 32 ou 35.D'autres ADNsprésentent une ou plusieurs séquences telle(s) que présente(s) sur le chromosome de *Neisseria meningitidis* Z2491, mais ne font pas partie des régions 1, 2, 3 définies ci-dessus.

De tels ADN comprennent une ou plusieurs séquences correspondant pour tout ou partie à SEQ ID n°3, 5, 11, 12, 14, 16, 18, 19, 20, 24, 27 ou 33.

D'autres ADNs encore sont présents sur la région 4 (arg J à reg F) ou sur la région 5 (marqueur lambda 375 à pen A) sur le chromosome de Nm Z2491.

Les ADNs des régions 4 et 5, et ceux capables de s'hybrider avec ces ADN, sous réserve d'exprimer les fonctions propres à Nm, présentent l'avantage d'intervenir de manière majeure dans la virulence de Nm, en étant impliqués dans l'étape de colonisation et de pénétration initiales et dans la dissémination septicémique.

**(Rev.3)** L'ADN de séquence SEQ ID N°95 selon l'invention, défini plus haut, est localisé dans la région 4. Cet ADN est **caractérisé en ce qu'**il code pour une protéine exportée au-delà de la membrane cytoplasmique.

**(Rev.4)** Selon d'autres dispositions, l'invention vise les vecteurs de transfert et d'expression, tels que plasmides, cosmides ou bactériophages, comportant au moins un ADN de l'invention tel que défini ci-dessus.

**(Rev.5)** Elle vise aussi les cellules hôtes, plus particulièrement les cellules bactériennes ou cellules de Nm, transformées par l'insertion d'au moins un ADN tel que défini ci-dessus.

D'autres cellules hôtes de l'invention comportent des gènes ou des fragments de gènes spécifiques de Nm et sont **caractérisées en ce que** leur chromosome est délété d'au moins un ADN selon l'invention, en particulier d'un ADN responsable de la pathogénicité. Il s'agit plus spécialement de cellules bactériennes, notamment de Nm.

**(Rev.6)** L'invention a également pour objet les ARN dont la séquence correspond à la transcription d'une séquence d'ADN telle que définie ci-dessus, étant entendu que ces ARN s'hybrident avec la séquence SEQ ID N°10, 36 ou 95.

Un autre produit entrant dans le champ de l'invention est constitué par un polypeptide.

**(Rev.7)** Ce polypeptide est **caractérisé en ce qu'**il est codé par un ARN tel que défini ci-dessus étant entendu, en ce qui concerne la séquence SEQ ID N°36, qu'il s'agit des phases de lecture des positions 1330 à 2970 ou 3083 à 9025.

**(Rev.8)** L'invention vise également un polypeptide, **caractérisé en ce qu'**il présente un enchaînement d'acides aminés codé par une phase de lecture s'hybridant avec la séquence SEQ ID N°10; ou 36, correspondant aux phases de lecture des positions 1330 à 2970 ou 3083 à 9025; ou 95; dans les conditions d'hybridation telles que définies ci-dessus.

**(Rev.9)** L'invention vise le polypeptide **caractérisé en ce qu'**il présente la séquence SEQ ID N°37 ou SEQ ID N°38.

Conformément à l'invention, les différents produits considérés ci-dessus sont obtenus par voie de synthèse et/ou biologique en opérant selon les techniques classiques.

Les acides nucléiques peuvent être également obtenus à partir de banques constituées d'ADN Nm- spécifiques, telles qu'élaborées selon une technique soustractive, cette technique comprenant :
- le mélange de deux populations d'ADN,
- la réalisation d'au moins une itération d'hybridation-amplification soustractive, et
- la récupération du ou des ADN souhaités, suivie le cas échéant de leur purification avec l'élimination des séquences redondantes.

Les deux populations d'ADN proviennent respectivement d'une souche de *Neisseria meningitidis,* dite souche de référence, pour laquelle la banque spécifique doit être constituée, et d'une souche de *Neisseria,* dite souche de soustraction, présentant une homologie en séquences primaires d'ADN supérieure à environ 70% avec la souche de *Neisseria meningitidis,* les séquences d'ADN des souches de soustraction et de référence étant telles qu'obtenues respectivement par cisaillement aléatoire, et par clivage par une endonucléase de restriction capable de produire des fragments de taille inférieure à environ 1kb.

A titre indicatif, un procédé d'obtention de banques d'ADN *Neisseria meningitidis* spécifiques, comporte les étapes de :
- cisaillement aléatoire de l'ADN chromosomique d'une souche *Neisseria gonorrhoeae,* dite souche de soustraction, notamment par passages répétés à travers une seringue,
- clivage de l'ADN chromosomique d'une souche de *Neisseria meningitidis,* dite souche de référence, de préférence par une enzyme de restriction produisant des fragments de taille inférieure à 1kb environ,
- ligature des fragments d'ADN de la souche de référence, clivés par l'enzyme de restriction, avec des amorces oligonucléotidiques appropriées,
- réalisation d'une itération d'hybridation-amplification soustractive par :
   . mélange des deux populations d'ADN dans des conditions appropriées pour l'hybridation des séquences homologues, puis
   . amplification des fragments auto-réannelés et récupération de ces fragments,
   . digestion de ces fragments par une enzyme de restriction, et re-ligature à des amorces oligonucléotides suivie d'une
   - purification de l'ADN ligaturé, et le cas échéant, d'une nouvelle itération d'hybridation soustractive, comportant le mélange de fragments d'ADN de *Neisseria gonorrhoeae* cisaillé comme indiqué ci-dessus avec les fragments d'ADN de *Neisseria meningitidis* issus de l'itération précédente, suivi, si on le souhaite du clonage des ADN de la banque.

Les amorces utilisées sont des amorces oligodésoxynucléotidiques adaptées à l'endonucléase de restriction utilisée et permettant une insertion dans un site de clonage, tel que le site EcoRI du plasmide pBluescript. On choisira avantageusement de telles amorces parmi les oligodésoxynucléotides référencés dans le listing de séquence sous SEQ ID n°36 à 45.

Les banques ainsi obtenues sont formées d'ADN spécifiques des méningocoques et absents chez les gonocoques.

La spécificité des ADN a été vérifiée comme exposé dans les exemples, à chaque itération par Southern blots, avec des gènes communs à la souche de soustraction et à la souche de référence, ou avec l'ADN total de chacune des souches digéré par une endonucléase de restriction, telle que *Cla*I.

A chaque itération, a également été vérifiée l'exhaustivité de la banque d'ADN par Southern blotting avec des sondes connues pour être spécifiques de la souche de référence, à savoir pour *Neisseria meningitidis,* les gènes *frp, opc,* rotamase, notamment.

Les expériences réalisées ont montré que les banques obtenues sont dépourvues des gènes présentant une homologie significative avec des espèces de Neisseria autre que *Neisseria meningitidis,* par exemple les gènes, *ppk* ou *pilC*1, et ce généralement, en seulement 2 ou 3 itérations.

Si nécessaire, deux voies, non exclusives l'une de l'autre, peuvent être empruntées.

Il est possible de procéder à une (n+1)^{ème} itération, en utilisant l'ADN de l'itération n comme population d'ADN de la souche de référence.

En variante, on réalise une deuxième banque, indépendante de la première, avec une enzyme de restriction de spécificité différente de celle utilisée dans la première banque, par exemple *Mbo*I.

Dans tous les cas, il est préférable de conserver chacun des produits issus de chacune des itérations réalisées.

La technique soustractive décrite ci-dessus est utilisable pour obtenir des banques d'ADN communs entre Nm et Ng, mais spécifiques par rapport à Nl.

On constitue avantageusement trois banques différentes, dont deux par digestion de l'ADN chromosomique de Nm par *MboI* et *Tsp5091,* et la troisième, par digestion de l'ADN chromosomique de Nm avec *MspI.* Deux séries de soustraction permettent de récupérer des ADN présentant la spécificité recherchée, comme décrit dans les exemples.

En appliquant le procédé ci-dessus, on constituera avantageusement des banques d'ADN spécifiques d'espèces données de cryptocoques, d*'Haemophilus,* de pneumocoques ou encore d'*Escherichia coli,* ou plus généralement de tout agent bactérien appartenant à la même espèce et disposant de pathovars différents.

De même, à partir de ces banques, l'invention fournit des moyens de disposer de facteurs de virulence spécifiques d'une espèce ou d'un variant donné peuvent être développés.

De telles banques constituent donc des outils présentant un intérêt majeur pour disposer d'attributs responsables de la spécificité d'un pathogène, cette application étant plus spécialement illustrée dans le contexte de l'invention par l'obtention de banques renfermant les attributs responsables de la spécificité de la pathogénèse méningococcique.

L'étude des produits de l'invention, acides nucléiques, polypeptides, a permis de mettre en évidence une spécificité absolue vis-à-vis de *Neisseria meningitidis,* quelle que soit la souche et sa variabilité.

Ces produits sont donc particulièrement appropriés pour le diagnostic des infections et méningites provoquées par *Neisseria meningitidis,* que ce soit chez l'adulte ou l'enfant et quel que soit le sérogroupe de la souche en cause.

**(Rev.10)** La méthode de diagnostic, selon l'invention, d'une infection meningococcique, et plus particulièrement de la méningite méningococcique, par mise en évidence de la présence de *Neisseria meningitidis* dans un échantillon biologique, est caractérisé par les étapes de :
- mise en contact, d'un échantillon biologique à analyser, avec un réactif comprenant SEQ ID N° 10 ou SEQ ID N°95, ou l'ADN complémentaire de ces séquences, le cas échéant sous forme de sonde nucléotidique, dans des conditions permettant respectivement une hybridation ou une réaction de type antigène-anticorps, et
- révélation du produit de réaction éventuellement formé, et
- révélation du produit de réaction éventuellement formé.

Lorsque le réactif est élaboré à partir d'un acide nucléique, celui-ci peut se présenter sous forme de sonde nucléotidique dans laquelle l'acide nucléique, ou un fragment de ce dernier, est marqué afin de permettre sa révélation. Des marqueurs appropriés comprennent des marqueurs radio-actifs, fluorescents, enzymatiques ou luminescents.

En variante, l'acide nucléique est inclus dans une cellule hôte, utilisée comme réactif.

Dans ces différentes formes, l'acide nucléique est utilisé tel quel ou sous forme d'une composition avec des véhicules inertes.

**(Rev. 11)** L'invention vise ainsi une composition, **caractérisée en ce qu'**elle comprend un polypeptide tel que défini ci-dessus, en association avec un véhicule physiologiquement acceptable.

L'échantillon utilisé dans l'étape de mise en contact est un échantillon biologique, issu d'un mammifère, tel que liquide céphalo-rachidien, urine, sang, salive.

L'étape de révélation est réalisée dans des conditions permettant de mettre en évidence le produit de réaction lorsqu'il s'est formé. Des moyens classiques mettent en oeuvre des réactions de fluorescence, luminescence, colorées, radio-actives ou encore des techniques d'autoriadographie. Il est également possible de quantifier le produit.

La méthode définie ci-dessus peut être appliquée au diagnostic d'une réaction immunitaire spécifique d'une infection méningococcique.

On utilise alors comme réactif un polypeptide conforme à l'invention, tel que codé par lesdites séquences d'acides nucléiques, correspondant au produit natif, ou un polypeptide modifié, mais possèdant l'activité biologique et immunologique de polypeptide natif correspondant.

Il s'agit avantageusement d'un polypeptide tel qu'exporté au-delà de la membrane cytoplasmique de *Neisseria meningitidis,* plus particulièrement de la partie d'un tel polypeptide correspondant à la région conservée de l'ADN.

D'autres caractéristiques et avantages de l'invention sont donnés dans les exemples qui suivent afin d'illustrer celle-ci sans toutefois en limiter sa portée.

Dans ces exemples, il sera fait référence aux figures 1 à 11 qui représentent respectivement
- les figures 1A, 1B, 1C, 1D, 1E, 1F et 1G l'analyse de la banque soustractive *Tsp*5091,
- la figure 2, la distribution de séquences Nm- spécifiques par rapport à Ng sur le chromosome de la souche Z2491, (partie gauche) et de séquences Nm spécifiques par rapport à Nl (partie droite),
- la figure 3A à 3C, la réactivité des clones des 3 régions du chromosome, envers une panel de souches du genre *Neisseria,*
- la figure 4, la position, dans la région 2 du chromosome de Nm, d'oligonucléotides utilisés comme sondes,
- les figures 5, 6 et 7, les Southern blots d'un panel de souches du genre *Neisseria,* en utilisant des parties de la région 2 de Nm comme sondes,
- les figures 8A à 8C, les Southern blots avec 3 banques soustractives sur un panel de 12 souches de *Neisseria,* et
- les figures 9, 10 et 11, la réactivité de clones des 3 banques soustractives vis-à-vis de Nm, Nl et Ng.

Dans les exemples qui vont suivre, les matériels et méthodes suivants ont été utilisés :
**Souches bactériennes** - Pour la réalisation des banques soustractives, on a utilisé la souche Z2491 de Nm (Achtman et al., 1991, J. Infect. Dis. 164, 375-382) les souches MS11 (Swanson et al., 1974, Infect. Immun. 10, 633-644), et les souches 8064 et 9764 de Nl, étant entendu que tout autre souche de l'espèce considérée pourrait être utilisée.

Afin de vérifier la spécificité de ces banques, 6 souches de Nm, 4 souches de Ng, une souche de Nl (*Neisseria lactamica*) et une souche de Nc (*Neisseria cinerea*) ont été utilisées.

Les six souches de Nm sont : Nm Z2491 de sérogroupe A, Nm 8013 de sérogroupe C (XN collection), Nm 1121 non sérogroupable (XN collection), Nm 1912 sérogroupe A (XN collection), Nm7972 de sérogroupe A (XN collection) et Nm 8216 de sérogroupe B (XN collection).

Les quatre souches de Ng sont : Ng MS11 (Institut Pasteur, Paris), Ng 403 (Institut Pasteur, Paris), Ng 6934 (Institut Pasteur, Paris), Ng WI (isolée à partir d'une infection gonococcique disséminée), Ng 4Cl, Ng 6493 et Ng FA 1090.

Les souches de Nl sont Nl 8064 et Nl 9764 (XN collection) et celle de Nc, Nc 32165 (XN collection).

### Techniques de génétique moléculaire

Sauf indication contraire, les techniques et réactifs utilisés correspondent à ceux recommandés par Sambrook *et al* (Sambrook et al 1989, Molecular Cloning: A Laboratory Manual. Cold Spring Harbor Laboratory Press). Les oligodésoxynucléotides utilisés dans cette étude sont :

| | |
|---|---|
| RBam12, | 3'AGTGGCTCCTAG 54 (SEQ ID N°54) |
| RBam24, | 5' AGCACTCTCCAGCCTCTCACCGAG 3'; (SEQ ID N°55) |
| Jbam12, | 3' GATCCGTTCATG 5'; (SEQ ID N°60) |
| JBAM24, | 5' ACCGACGTCGACTATCCATGAACG 3'; (SEQ ID N°61) |
| REco12, | AGTGGCTCTTAA; (SEQ ID N°56) |
| REco24, | 5' AGCACTCTCCAGCCTCTCACCGAG 3'; (= RBam 24) |
| JEco12, | GTACTTGCTTAA; (SEQ ID N°62) |
| JEco24, | 5' ACCGACGTCGACTATCCATGAACG 3'; (= JBam24) |
| NEco2, | AATTCTCCCTCG; (SEQ ID N°64) |
| NEco24, | AGGCAACTGTGCTATCCGAGGGAG; (SEQ ID N°65). |

### Transferts sur membranes (Southern blots)

Les transferts sur membranes ont été réalisés par transferts capillaires sur des membranes en nylon chargées positivement (Boehringer Mannheim). Les hybridations ont été réalisées à 65°C dans une solution comprenant NaPi 0,5M pH 7,2/EDTA 1mM/SDS 7%/ BSA 1%. Les lavages des membranes ont été réalisées dans une solution comprenant NaPi 40mM pH 7,2/EDTA 1mM/SDS 1%. Le lavage final a été réalisé à 65°C pendant 5 min.

La sonde *frp*, obtenue avec des oligonucléotides basés sur la séquence de *frp*A correspond à 2,4 kb de l'extrémité 5' du gène de la souche Z2491. Les sondes *opc* et rotamase correspondant aux gènes entiers sont produites à partir de la souche Z2491 en utilisant des oligonucléotides réalisés sur la base de séquences publiées. Les sondes *pilC1* et *ppk* (polyphosphate kinase) correspondent aux inserts des plasmides pJL1 et pBluePPK6001, respectivement.

### Exemple 1 : Réalisation de banques d'ADN présents chez Nm et absents chez Ng.

### a. Banque "MboI"

**Réalisation** - L'ADN de Nm Z2491 a été clivé par l'endonucléase *Mbo*I et soumis à deux itérations d'une méthode, appelée ci-après CDA (Comprehensive Difference Analysis). Cette méthode comprend une hybridation soustractive en présence d'un excès d'ADN cisaillé de Ng MS11 et une amplification par PCR de celles des séquences méningococciques qui, étant absentes de ou ne présentant pas d'homologie significative avec l'ADN de Ng MS11, pouvaient se ré-anneler.

L'ADN chromosomique de la souche Ng MS11 est cisaillé de manière aléatoire par passages répétés à travers une seringue hypodermique jusqu'à obtention de fragments dont la taille s'échelonne de 3 à 10 kb. Ces fragments d'ADN sont purifiés par extraction phénolique.

L'ADN chromosomique de la souche Nm Z2491 est, quant à lui, clivé par l'endonucléase de restriction *Mbo*I. Ces fragments d'ADN (20 µg) sont ligaturés à 10 nmoles des oligonucléotides annelés RBam12 et RBam24. Les amorces en excès sont éliminées par électrophorèse sur un gel d'agarose à 2% à bas point de fusion. La partie du gel contenant des fragments amplifiés de taille supérieure à 200 pb est excisée et digérée par la β-agarase. Ces fragments sont purifiés par extraction phénolique.

Afin de réaliser une hybridation soustractive (première itération), 0,2 µg d'ADN Nm, ligaturé aux oligonucléotides RBam, est mélangé à 40 µg d'ADN Ng dans un volume total de 8 ml d'un tampon EE 3X (un tampon EE 1X est composé de N-(2-hydroxyéthyl) pipérazine-N'-(acide sulphonique propane 3) 10 mM et d'EDTA 1 mM, son pH est de 8.0). Cette solution est recouverte d'huile minérale et l'ADN est dénaturé par chauffage à 100°C pendant 2 min. 2 µl de NaCl 5M sont ajoutés et on laisse le mélange s'hybrider à 55°C pendant 48h. Le mélange réactionnel est dilué à 1/10 dans une solution préchauffée composée de NaCl et de tampon EE, puis immédiatement placé sur de la glace.

10 µl de cette dilution sont ajoutés à 400 µl de mélange réactionnel pour PCR (Tris.HCl pH9.0 10mM; KCl 50 mM; MgCl₂ 1,5 mM; Triton X100 0,1 %; 0,25 mM de chacun des quatre désoxynucléotides triphosphate ; Taq polymérase 50 unités par ml). Le mélange est incubé pendant 3 min à 70°C pour compléter les extrémités des fragments ré-annelés d'ADN méningococciques.

Après dénaturation à 94°C pendant 5 min et addition de l'oligonucléotide RBam24 à raison de 0, 1 nmole par 100 µl, les hydridations sont amplifiées par PCR (30 cycles de 1 min à 94°C, 1 min à 70°C et 3 min à 72°C suivis par 1 min à 94°C et 10 min à 72°C; Perkin-Elmer GeneAmp 9600).

Les fragments méningococciques amplifiés sont séparés sur gel des amorces et des ADN gonococciques de hauts poids moléculaires. Ils sont digérés par *Mbo*I et de nouveaux oligonucléotides JBam12 et JBam24 leur sont ligaturés. Ces ADN ligaturés sont à nouveau purifiés sur gel et extraits au phénol.

Une seconde itération d'hybridation soustractive est réalisée sur 40 µg d'ADN Ng cisaillé de manière aléatoire et 25 ng d'ADN ligaturé aux oligonucléotides JBam tel qu'obtenu à l'issue de la première itération d'hybridation soustractive. Lors de cette seconde itération, l'amplification de l'ADN Nm auto-annelé est réalisée à l'aide de l'oligonucléotide Jbam24.

**Spécificité -** Afin de confirmer leur Nm-spécificité, les séquences amplifliées après la seconde itération de la méthode CDA sont marquées et utilisées comme sonde pour de l'ADN digéré par *Cla*I issu d'un panel de six souches de *Neisseria meningitidis,* quatre de *Neisseria gonorrhoeae,* une de *Neisseria lactamica* et une de *Neisseria cinerea.*

Les Southern blots réalisés montrent que les séquences amplifliées à l'issue de la seconde itération de la méthode CDA présentent une forte réactivité avec de nombreuses bandes correspondant aux meningocoques et ne présentent pas de réactivité avec les bandes correspondant aux souches Ng, Nl, Nc.

La banque "MboI" apparaît donc comme Nm-spécifique.

**Exhaustivité -** Afin de tester l'exhaustivité de la banque, l'ensemble des produits issus de la première et de la seconde itérations de la méthode CDA ainsi que les matériaux chromosomiques initiaux de Nm Z2481 et de Ng MS11 sont soumis à électrophorèse sur gel d'agarose, transférés sur membrane et mis en contact avec des sondes comprenant des gènes connus pour être méningococcus-spécifiques, à savoir *frp, opc,* rotamase (Southern blot).

Il résulte de ces hybridations que le gène Nm-spécifique *frp* est représenté dans la banque *Mbo*I par un fragment de 600 pb, mais qu'aucune activité n'est observée pour les gènes rotamase et *opc.* La banque *Mbo*I, bien que Nm-spécifique, ne peut donc être considérée comme exhaustive.

Etant donné leur haute spécificité, les fragments issus de la seconde itération de la méthode CDA pour la banque *Mbo*I peuvent néanmoins être clonés sur le site *Bam*HI du plasmide pBluescript.

Une séquence correspondant à un quelconque des gènes Nm-spécifiques ne peut être incluse dans la banque soustractive que si elle est portée par un fragment de restriction de taille appropriée. Cette condition est fonction de deux facteurs. Premièrement, la probabilité pour que les plus grands fragments soient entièrement Nm-spécifiques est faible. Deuxièmement, même si de tels fragments existaient, ils seraient sous-représentés dans la banque du fait des limitations de la technique PCR dont l'efficacité d'amplification diminue avec l'augmentation de la taille des fragments. Les fragments de taille supérieure à environ 600 pb ne sont pas inclus dans la banque. Du fait de l'abscence, dans le chromosome de Nm Z2491, de fragments *Mbo* de taille appropriée, les gènes rotamase et *opc* ne peuvent être inclus dans la banque. Une enzyme quelconque ne peut à elle seule produire un petit fragment correspondant à un gène Nm-spécifique quelconque. Une deuxième banque a donc été réalisée en utilisant une autre enzyme de restriction avec une spécificité différente : *Tsp*509.

### b. Banque "Tsp509I"

**Réalisation** - L'enzyme *Tsp*5091 présente l'avantage de produire des fragments de plus petite taille (inférieure à 1 kb environ) que l'enzyme *MboI.*

*Tsp*509I reconnaît la séquence AATT et laisse, en saillie en 5', une séquence de 4 bases compatible avec *Eco*RI. Les oligonucléotides utilisés sont Reco, Jeco et NEco.

La méthode suivie est conforme à celle suivie pour la réalisation de la banque "*Mbo*I" décrite ci-dessus. De plus fortes quantités d'ADN méningococciques ont cependant été utilisées pour la première itération d'hybridation soustractive afin de compenser le plus grand nombre de fragments de faibles poids moléculaires produits par *Tsp*509I. Pour la première itération, 400 ng de fragments d'ADN Nm et, dans la seconde, 25 ng de fragments Nm sont soumis à hybridation soustractive avec 40 µg d'ADN Ng cisaillé de manière aléatoire.

Pour la réalisation de cette banque "*Tsp*509I", à titre de contrôle, une troisième itération d'hybridation soustractive est réalisée en utilisant 40 µg d'ADN Ng cisaillé et 0,2 ng de fragments Nm résultant d'une digestion par *Tsp*509I et d'une re-ligature aux adaptateurs NEco des fragments obtenus à l'issue de la seconde itération.

**Spécificité -** Comme décrit pour la banque précédente, le produit issu de la deuxième itération de la méthode CDA est marqué et utilisé comme sonde pour un panel de souches de *Neisseria.*

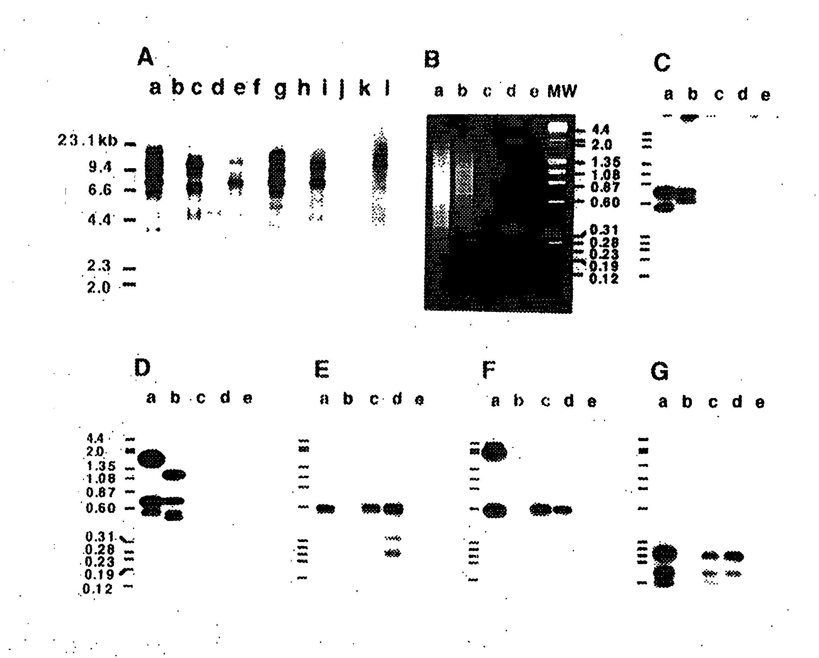

La figure 1A illustre l'hybridation Southern blot des produits de la seconde itération de la méthode CDA avec l'ADN digéré par *ClaI* de : Nm en piste a, de Ng MS11 en piste b, de Nm 8013 en piste c, de Ng 403 en piste d, de Nm 1121 en piste e, de Ng 6934 en piste f, de Nm 1912 en piste g, de Ng WI (souche DGI) en piste h, de Nm 7972 en piste i, de Nl 8064 en piste j, de Nc 32165 en piste k, de Nm 8216 en piste 1.

Contrairement à la forte réactivité observée avec toutes les souches Nm, une faible, ou aucune réactivité, est observée avec les souches Ng, Nl et Nc.

La spécifité de la banque a été étudiée plus avant en faisant réagir des transferts sur membrane (Southern blots) des produits issus de chacune des trois itérations de la méthode CDA avec des sondes correspondant à *pil*C1 et *ppk.* Ces deux gènes sont communs à Nm et Ng.

La figure 1B représente un gel d'agarose après électrophorèse des chromosomes de Nm Z2491 et Ng Ms11, digérés avec *Tsp*509 et des produits issus de chacune des itérations de la méthode CDA.

En piste a, a été déposé 1 µg du chromosome de Nm, en piste b 1 µg de celui de Ng, en piste c 0,15 µg des produits issus de la première itération CDA, en piste d 0,1 µg de ceux de la seconde itération, en piste e 0,05 µg de la troisième itération, MW représentant les marqueurs de taille moléculaire.

Les figures 1C et 1D représentent des gels réalisés comme décrits en figure 1B après transfert sur membrane (Southern blots) et hybridation avec *pil*C1 (figure 1C) et *ppk* (figure 1D).

A l'issue de la seconde intération de la méthode CDA, les séquences correspondant aux gènes *pil*C1 et ppk sont complètement exclues de la banque.

**Exhaustivité -** L'exhaustivité de la banque a été examinée en faisant réagir les produits issus de l'hybridation soustractive avec des sondes correspondant à trois gènes Nm-spécifiques (*frp*, rotamase et *opc*).

Ces sondes Nm-spécifiques réagissent avec les produits d'amplification issus de la première et de la seconde itération d'hybridation soustractive.

Les figures 1E,1F et 1G représentent des gels réalisés comme décrits en figure 1B après transfert sur membrane (Southern blots) et hybridation avec *frp*A (figure 1E), rotamase (figure 1F) et *opc* (figure 1G).

Une troisième itération d'hybridation soustractive conduit cependant à la perte de séquences Nm-spécifiques car les fragments réagissant avec les gènes rotamase et *opc* sont absents de cette troisième itération.

En considérant l'ensemble de ces données, il résulte que les produits issus de la seconde itération de la méthode CDA sont Nm-spécifiques et constituent également une banque exhaustive des séquences Nm-spécifiques.

Les produits issus de cette deuxième itération sont clonés au niveau du site *Eco*RI du plasmide pBluescript.

La banque produite par *Tsp*509I est plus exhautive que la banque produite par *Mbo*I, comme les considérations théoriques basées sur la production enzymatique de plus petits fragments de restriction le supposaient.

Selon cet aspect, il faut aussi noter que la banque *Tsp*509I est moins redondante que la banque *Mbo*I c'est-à-dire qu'elle comprend moins de duplication de clones. 86% des clones de la banque *Tsp*509I correspondent à des séquences distinctes alors que seulement 43% des clones correspondent à des séquences distinctes dans la banque *Mbo*I (données non présentées).

La banque produite par Tsp509I constitue donc une source de clones Nm-spécifiques.

### Exemple 2 : Analyse des clones des banques soustractives

### Clonage et séquençage des ADN Nm-spécifiques

Les ADN des banques soustractives sont clonés au niveau du site *Bam*HI (banque *Mbo*I) ou *Eco*RI (banque *Tsp*509I) du plasmide pBluescript, puis transformés dans DH5α de *E. coli.* Les inserts sont amplifiés par PCR réalisée sur les colonies transformées en utilisant les amorces M13-50 et M13-40, cette dernière amorce étant biotinylée à son extrémité 5'.

Le séquençage a été réalisé sur chaque produit PCR après séparation des brins biotinylés et non-biotinylés en utilisant le système Dynabeads M-280 à streptavidine (Dynal, Oslo). Les séquences sont criblées selon leurs homologies avec des séquences précédemment publiées en utilisant les programmes informatiques Blastn et Blastx (NCBI, USA et Fasta).

Les produits PCR issus des colonies de bactéries transformées, après utilisation des amorces M13-40 et M13-50 comme décrit ci-dessus, ont été marqués par incorporation avec amorçage aléatoire de α -³²P-dCTP et ont été utilisés comme sonde pour les transferts sur membrane de l'ADN chromosomique digéré par *Cla*I des souches Nm Z2491 et Ng MS11, comme décrit ci-dessus afin de vérifier leur spécificité.

### Cartographie des clones sur le chromosome de la souche Nm Z2491.

On rapporte les résultats des études effectuées avec 17 clones de la banque "*MboI*" (désignés par la lettre B) et 16 clones de la banque "*Tsp*5091" (désignés par la lettre E), chacun de ces clones présentant une séquence unique et sans contrepartie chez Ng.

Les positions des séquences d'ADN correspondant aux produits Nm-spécifiques clonés ont été déterminées par rapport à la carte publiée du chromosome de Nm Z2491 (Dempsey et al. 1995, J. Bacteriol. 177, 6390-6400) et à l'aide de transferts sur membranes (Southern blots) de gels d'agarose ayant été soumis à électrophorèse à champ pulsé (PFGE).

Les clones Nm-spécifiques sont utilisés comme sondes pour une hybridation sur membranes (Southern blots) de l'ADN de Nm Z2491 digéré avec des enzymes à rares sites de coupure, à savoir *Pac*I, *Pme*I, *Sgf*I*, Bgl*II, *Spe*I *Nhe*I que *Sgf*I.

Les gels (20 x 20 cm) étaient des gels à 1% d'agarose dans un tampon TBE 0,5X et ont été soumis à électrophorèse à 6 V/cm pendant 36 heures selon des périodes de pulsation variant de manière linéaire entre 5 et 35 secondes.

Les hybridations sur membrane (Southern blots) ont été réalisés comme décrit précédemment.

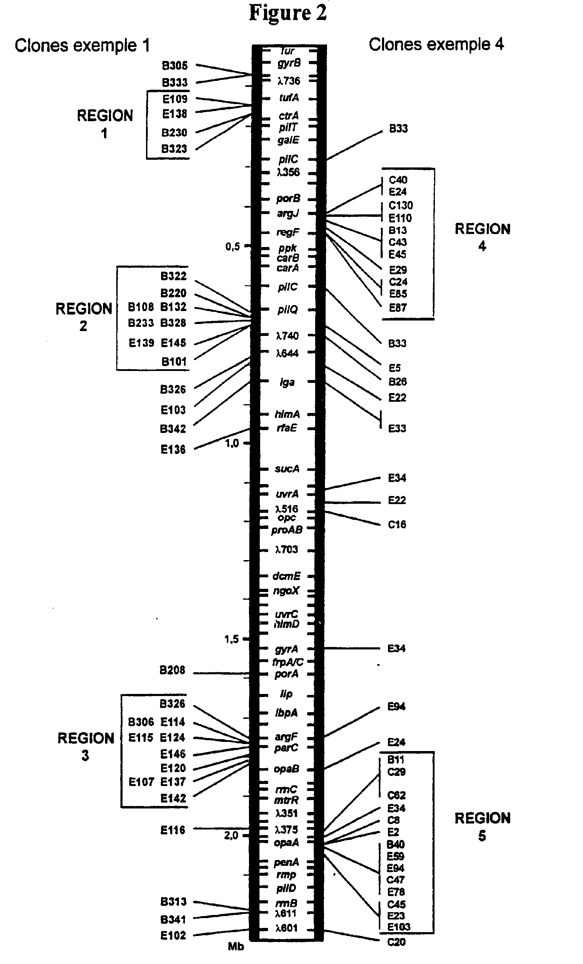

Les résultats obtenus sont rapportés sur la figure 2: la réactivité a été localisée par comparaison avec les positions des fragments de taille correspondante sur la carte publiée. Les positions de l'ensemble des marqueurs génétiques cartographiés par Dempsey *et al* (précédemment cité) sont visualisées à l'aide de points sur la carte linéaire chromosomique. Les gènes Nm-spécifiques précédemment divulgués sont marqués d'un astérisque. Les deux loci appelés *"frp"* correspondent aux gènes *frp*A et *frp*C. Les locis "*pil*C" correspondent aux gènes *pil*C1 et *pil*C2 qui sont des paires de gènes homologues et qui ne sont pas distingués sur la carte. La précision des positions des clones Nm-spécifiques de l'invention dépend des chevauchements des fragments de restriction réactifs. En moyenne, la position est de +/- 20 kb.

Cette cartographie révèle une distribution non aléatoire des séquences Nm-spécifiques. La majorité des séquences Nm-spécifiques appartiennent à trois groupes distincts. Un de ces groupes (région 1) correspond à la position de gènes relatifs à la capsule précédemment décrits.

On distingue :
- E109, E138, B230 et B323 comme étant la région 1,
- B322, B220, B108, B132, B233, B328, E139, E145 et B101 comme étant la région 2, et
- B306, E114, E115, E124, E146, E120, E107, E137 et E142 comme étant la région 3.
63% des séquences identifiées comme spécifiques des méningocoques sont localisées à l'intérieur de ces trois régions distinctes.

Ce regroupement contraste avec la distribution de gènes Nm-spécifiques précédemment divulgués (*frpA*, *frpC por*A, *opc* et la région relative à la capsule).

Cet art antérieur suggérait en effet que les gènes Nm-spécifiques étaient à l'exception des gènes fonctionnellement relatifs à la capsule, dispersés le long du chromosome.

La cartographie des séquences Nm-spécifiques sur le chromosome conduit à un résultat inattendu en regard de l'art antérieur.

La majorité des différences génétiques entre les souches meningoccale et gonococcale testées sont regroupées en trois régions distinctes.

La région 1 regroupe des gènes relatifs à la capsule des meningococci.

La fonction des gènes des autres régions n'est pas connue mais des homologies avec des séquences publiées (tableau 1) suggèrent des similarités entre certains gènes de la région 3 et les protéines transposases et de régulation de bactériophages. Aucun virus meningococcal n'a été caractérisé et il est tentant d'imaginer que ces séquences soient d'origine phagique. De manière intéressante, le génome de H. *influenzae* contient également une séquence homologue à celle de la protéine de régulation Ner du phage Mu mais on ne sait pas s'il s'agit d'un gène fonctionnel.

Le clone B208 présente une forte homologie (48% d'identité, 91% d'homologie pour 33 acides aminés) avec un clone des régions conservées (domaine III) dans la classe des protéines qui se lient aux sidérophores ferriques TonB-dépendants.

La proximité de ce clone avec les gènes Nm-spécifiques *por*A et les gènes régulés par le fer *frp,* et en particulier la possibilité qu'il s'agisse d'une protéine récepteur Nm-spécifique exposée sur la membrane externe font de lui un bon candidat pour de plus amples recherches.

Le clone B339 correspond à la séquence d'insertion Nm-spécifique IS*1106.*

La faible homologie entre le clone B134 et la séquence d'insertion d*'Aeromonas,* ainsi que la présence en copies multiples du clone B134 parmi des souches variées de Nm, suggèrent qu'il pourrait représenter un nouveau type de séquence d'insertion Nm-spécifique.

La possibilité pour que les régions contenant les clones Nm-spécifiques puissent correspondre à des îlots de pathogénicité comme précédemment décrit pour *E. coli* et *Y. pestis* est d'un intérêt particulier.

Les clones isolés dans cette invention vont permettre de mieux comprendre la pertinence des régions Nm-spécifiques en permettant le clonage et le séquençage de fragments chromosomiques plus grands et directement par leur utilisation pour des mutations de loci.

Enfin, la détection des gènes meningococcus-spécifiques, éventuellement impliqués dans la pathogénicité de l'organisme, permet de cibler des antigènes appropriés utilisables dans un vaccin anti-meningococcique.

L'efficacité et la rapidité de la méthode selon l'invention permettent son utilisation dans un grand nombre de situations pour lesquelles les différences génétiques responsables d'un phénotype particulier à un de 2 pathogènes proches sont recherchées.

### Etude de la réactivité des clones des régions 1, 2 et 3 vis-à-vis d'un panel de souches de Neisseria

Les produits PCR correspondant aux inserts de chacun des clones ont été rassemblés et utilisés comme sondes d'hybridation sur membranes (Southern blots) pour un panel de souches de Nm, de Ng, de Nl et de Nc.

Les régions 1 et 2 produisent un nombre limité de bandes pour chacun des méningocoques. Cela suggère que ces régions sont à la fois Nm-spécifiques et communes à tous les méningocoques.

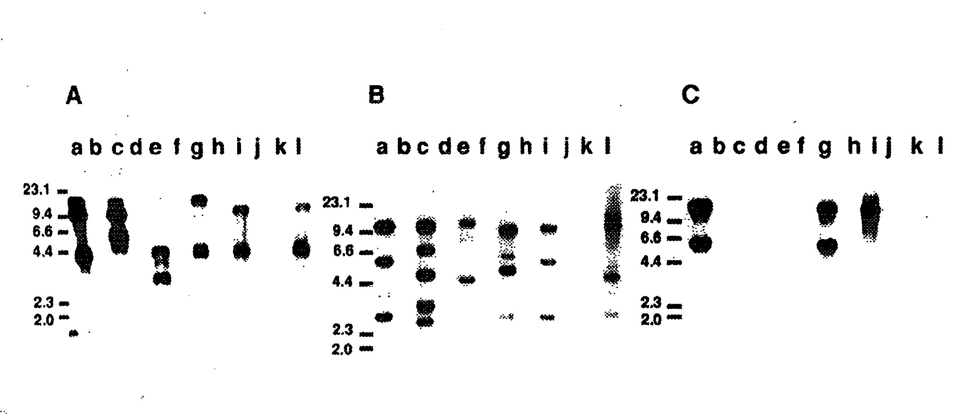

La figure 3 illustre la réactivité des clones des régions 1, 2 et 3 envers un panel de souches neisseriales. Les clones des régions 1 (figure 3A), 2 (figure 3B) et 3 (figure 3C) pris ensemble ont été utilisés comme sondes envers un panel de meningococci, gonococci et envers une souche de Nl et de Nc.

Les pistes sont les suivantes : ADN de Nm Z2491 en piste a, de Ng MS11 en piste b, de Nm 8013, en piste c, de Ng 403 en piste d, de Nm 1121 en piste e, de Ng 6934 en piste f, de Nm 1912 en piste g, de Ng WI (souche DGI) en piste h, de Nm 7972 en piste i, de Nl 8064 en piste j, de Nc 32165 en piste k, de Nm 8216 en piste 1.

De manière remarquable, la région 3 ne présente de réactivité qu'avec les meningococci de sérogroupe A. Cette région 3 est donc spécifique d'un sous-groupe de Nm.

Une comparaison avec des séquences connues dans les banques de données a été réalisée afin d'évaluer les possibles fonctions des régions clonées.

Le tableau 1 qui suit donne les positions des clones spécifiques sur la carte chromosomique et les homologies avec des séquences connues.

**TABLEAU 1 - Position des clones spécifiques sur la carte chromosomique et homologies avec des séquences connues**

| | | | Fragments réactifs | | | | | | |
|---|---|---|---|---|---|---|---|---|---|
| Nom du Clone* | Taille de l'insert | Pac | Pme | Bgl | Spe | Nhe | Sgf | Position sur Z2491 | Homologies des séquences protéiques |
| B305 | 259 | 18-20 | 15-17 | 22-23 | 18 | 11-13 | 2 | λ736 | |
| B333 | 235 | | 15-17 | 22-23 | 18 | 11-13 | 2 | λ736 | |
| E109¹⁺ | 211 | | 6-7 | 11-15 | 10 | 11-13 | 2 | *tufA*/*ctrA* | protéine LipB *N. meningitidis* (3 x10⁻²⁶) |
| E138¹⁺ | 315 | 1 | 6-7 | 11-15 | 10 | 11-13 | 2 | *tufA*/*ctrA* | protéine LipB *N. meningitidis* (4 x10⁻⁷⁵) |
| B230¹ | 356 | 1-3 | 6-7 | 1 | 10 | 11-13 | 2 | ctrA | protéine KpsC *E.coli* (3 x 10⁻⁵³) |
| B323¹ | 363 | 1 | 6-7 | 1 | 10 | 11-13 | 2 | ctrA | protéine CtrB *N. meningitidis* (2 x 10⁻⁶⁴) |
| B322² | 210 | | 2 | 16-18 | 6 | 1 | 5 | pil*Q*/λ740 | HlyB *S. marcescens* (4 x 10⁻¹⁵) |
| B220² | 341 | | 2 | 16-18 | 6 | ≥18 | 5 | pil*Q*/λ740 | |
| B108² | 275 | | 2 | 19-21 | 6 | >18 | 5 | pil*Q*/λ740 | |
| B132² | 411 | 2 | 2 | 19-21 | 6 | ≥18 | 5 | pil*Q*/λ740 | |
| B233² | 164 | 1-3 | 2 | 19-21 | 6 | ≥18 | 5 | pil*Q*/λ740 | |
| B328² | 256 | 1-3 | 2 | 22-23 | 6 | ≥18 | 5 | pil*Q*/λ790 | |
| E139² | 324 | 2 | 2 | 19-21 | 6 | ≥18 | 5 | pil*Q*/λ790 | |
| E145² | 343 | 2 | 2 | 19-21 | 6 | ≥18 | 5 | pil*Q*/λ790 | |
| B101² | 254 | ≥20 | 2 | 19-21 | 6 | ≥18 | 5 | pil*Q*/λ740 | |
| E103q | 334 | | 2 | 11-15 | 3-5 | 10 | 3 | λ644 | |
| B326^{§} | 314 | | 2 | 11-15 | 3-4 | 10 | 3 | λ644 | |
| B326 (faible réactivité) | | | 5 | 6 | 16 | 2 | 1 | *argF* | |
| B342 | 167 | | 2 | 19 | 3-4 | 6-7 | 3 | *iga* | |
| E136 | 249 | | 2 | 7 | 1 | 3 | 3 | *lepA* | |
| B208 | 177 | | 1 | 2 | 3-4 | 2 | 1 | *porA* | Récepteur de la pyocheline FeIII *P. aeruginosa* (5.10⁻⁴) |
| = B306^{3#} | 219 | 11 | 5 | 11-12 | 5 | 2 | 4 | parC | |
| E114³ | 227 | 11 | 5 | 11-12 | 5 | 2 | 4 | parC | |
| E115^{3#} | 251 | | 5 | 11-15 | 5 | 2 | 4 | parC | |
| E124³ | 208 | | 5 | 11-12 | 5 | 2 | 4 | parC | |
| E146³ | 146 | | 5 | 11-15 | 5 | | 4 | parC | |
| E120³ | 263 | | 5 | 3-4 | 5 | 16 | 4 | opaB | |
| E107³ | 248 | 11 | 14-17 | 3-4 | 5 | 16 | 4 | opaB | |
| E137³ | 274 | | 14-17 | 3-4 | 5 | 16 | 4 | opaB | Transposase Bacteriophage D3112 (6 x 10⁻¹²) |
| E142³ | 230 | | 14-17 | 3-4 | 5 | 16 | 4 | opaB | Protéine Ner-Like. *H. influenzae* (6 x 10⁻²³) Protéine se liant à l'ADN Ner, Phage mu (3 x 10⁻¹⁸) |
| E116 | 379 | 5-7 | 11-13 | 3-4 | 2 | 6-7 | 8 | λ375 | |
| B313 | 436 | 9 | 9 | 3-4 | 13-14 | 5 | 2 | λ611 | |
| B341 | 201 | 8-10 | 9 | 3-4 | 13-14 | 5 | 2 | λ611 | |
| E102 | 238 | | 11-13 | 3-4 | 19 | 5 | 2 | λ601 | Protéine hypothétique HI1730 *H. influenzae* (7 x 10⁻²⁴) |
| B134 | 428 | | | multiple | | | | | transposase I*SAS2, Aeromonas* |
| B339 | 259 | | | multiple | | | | | *salmonicida* (5 x 10⁻⁵) tranposase IS *1106 N. meningitidis* (6 x 10⁻⁴⁵) |

| | | | | | | | | | |
|---|---|---|---|---|---|---|---|---|---|
| Entre Parenthèses figure la signification des homologies trouvées, telle que donnée par le programme Blastx *) Les clones marqués de l'exposant "1", "2" ou "3" appartiennent aux régions "1", "2" ou "3" respectivement du chromosome de *N. meningitidis Z2491.* +) E109 et E138 sont des clones contigus §) B306 et E115 se chevauchent #) B236 présente également une faible réactivité dans la région de *arg* F q) Le clone E103 contient un site *Tsp509* I et peut donc contenir deux inserts, cependant, comme il ne réagit qu'avec un seul fragment *Cla*I (Oks) du chromosome de *N.meningitidis* Z2491 et n'occupe qu'une position sur la carte, ce clone est inclus ici. | | | | | | | | | |

On peut voir, tout d'abord, que les clones de la région 1 correspondent tous aux gènes impliqués dans la biosynthèse de la capsule. Ces gènes ont été précédemment étudiés parmi les Nm de sérogroupe B (Frosch et al. 1989, Proc. Natl. Acad. Sci. USA 86, 1669-1673 et Frosch et Muller 1993, Mol. Microbiol. 8 483-493).

A l'exception d'une faible homologie avec l'activateur de hémolysine de *Serratia marcescens,* les clones de la région 2 ne présentent aucune homologie significative avec les séquences publiées, que ce soit au niveau de l'ADN ou des protéines.

Deux des clones de la région 3 présentent d'intéressantes homologies avec des protéines qui se lient à l'ADN, en particulier les protéines de régulation et les protéines transposases de bacteriophages.

Le clone B208 présente une forte homologie avec une des régions conservées dans une classe de récepteurs (sidérophore ferrique TonB-dependant).

Les clones B134 et B339 s'hybrident avec de nombreuses régions du chromosome (au moins 5 et au moins 8, respectivement).

Les données concernant les séquences montrent que le clone B339 correspond à la séquence d'insertion Nm-spécifique *S1106.*

La traduction du clone B143 présente une homologie limitée avec la transposase d'une séquence d'insertion *Aeromonas* (SAS2) (Gustafson et al. 1994, J. Mol. Biol. 237, 452-463). Nous avons pu démontrer par transfert sur membrane (Southern blots) que ce clone est une entité Nm-spécifique présente en multiples copies dans les chromosomes de chaque meningocoque du panel testé.

Les autres clones ne présentent pas d'homologie significative avec les séquences neisseriales publiées ni d'ailleurs avec aucune séquence publiée. Ces clones constituent donc, avec la majorité, des autres clones isolés, une banque de loci Nm-spécifiques totalement nouveaux.

### Exemple 3: Etude de la région 2 du chromosome de Nm

### Détermination et caractérisation de la séquence de la région 2

On procède à une amplification par PCR avec de l'ADN chromosomique de la souche Z2491 de sérogroupe A, sous-groupe IV-1, en utilisant des amorces d'oligonucléotides élaborées à partir de chacune des séquences de clones de la région 2, selon de nombreuses combinaisons différentes. On séquence les produits de la PCR qui se chevauchent à partir des 2 brins en utilisant la technique de terminaison de chaîne et le séquençage automatisé (ABI 373 ou 377).

Pour prolonger la séquence au-delà des limites des clones disponibles, on clone des fragments partiels SauIIIA de 15 kb, de la souche Z2491, dans Lambda DASH-II (Stratagène).

On identifie les phages contenant les inserts chevauchant la région 2 par hybridation avec comme sondes des clones de cette région. L'ADN inséré est séquencé à partir des extrémités des inserts et ces séquences sont utilisées pour élaborer de nouvelles amorces qui serviront à amplifier directement l'ADN chromosomique et non l'ADN phagique.

On obtient une amplification de l'ADN chromosomique en utilisant ces nouvelles amorces et celles de la séquence précédemment disponible.

Ces produits PCR sont également séquencés à partir des 2 brins , ce qui conduit à une séquence complète de 15620 pb (SEQ ID N°36). On analyse les cadres de lecture de cette séquence qui commencent par ATG ou GTG et qui sont caractérisés par un indice d'usage de codons élevés. Cette analyse révèle 7 COLs de ce type qui remplissent la plus grande partie de la séquence de 15620pb. Les positions de ces COLs sont les suivantes:
COL.-1: 1330 à 2970 (SEQ ID N°37); COL-2: 3083 à 9025 (SEQ ID N°38); COL-3: 9044 à 9472 (SEQ ID N°39); COL-4: 10127 à 12118 (SEQ ID N°40); COL-5: 12118 à 12603 (SEQ ID N°41); COL-6: 12794 à 13063 (SEQ ID N°43); COL-7: 13297 à 14235 (SEQ ID N°44); et COL-8: 14241 à 15173(SEQ ID N°45).

Le COL-4 commence avec le codon GTG et chevauche un COL légèrement plus petit (SEQ ID N°41) dans le même cadre de lecture (9620-12118, cadre 2) et qui commence par le codon ATG.

COL-4 code pour une protéine qui présente des homologies structurelles avec une famille de polypeptides comprenant les pyocines (Pseudomonas aeruginosa), collcines et intimines (Escherichia coli) qui sont des toxines bactéricides (pyocines, collcines) ou des protéines de surfaces impliquées dans l'adhésion des bactéries aux protéines eucaryotes. Le COL-7 encode une protéine dont la séquence contient une région potentiellement transmembranaire, et qui présente des homologies structurelles avec des protéines périplasmiques ou insérées dans la membrane externe des bactéies. Les homologies structurelles de COL-4 et COL-7 ont été identifiées à l'aide du programme PropSearch.

La recherche de séquences homologues aux autres COL dans GenBank à l'aide du programme BLAST a révélé une homologie entre les régions N-terminales de COL-2 et l'hémagglutinine filamenteuse B de *Bordetella pertussis* (43% de similarité, 36% d'identité sur 352 acides aminés) et entre COL-1 et la protéine fhaCde *Bordetella pertussis* (35% de similarité, 27% d'identité sur 401 acides aminés). COL-1 et COL-2 sont des gènes voisins dans la souche Z2491 et l'hémagglutinine filamenteuse B de *Bordetella pertussis* et fhaC sont des gènes voisins dans Bordetella *pertussis,* ce qui renforce la probabilité que ces homologies reflètent des homologies fonctionnelles.

. Confirmation de la spécificité de la région 2 vis-à-vis de Nm

On effectue des Southern blots en utilisant des sondes d'ADN obtenues par amplification par PCR de différentes parties de la région 2 en utilisant des amorces oligonucléotidiques élaborées à partir de séquences de clones de la région 2.

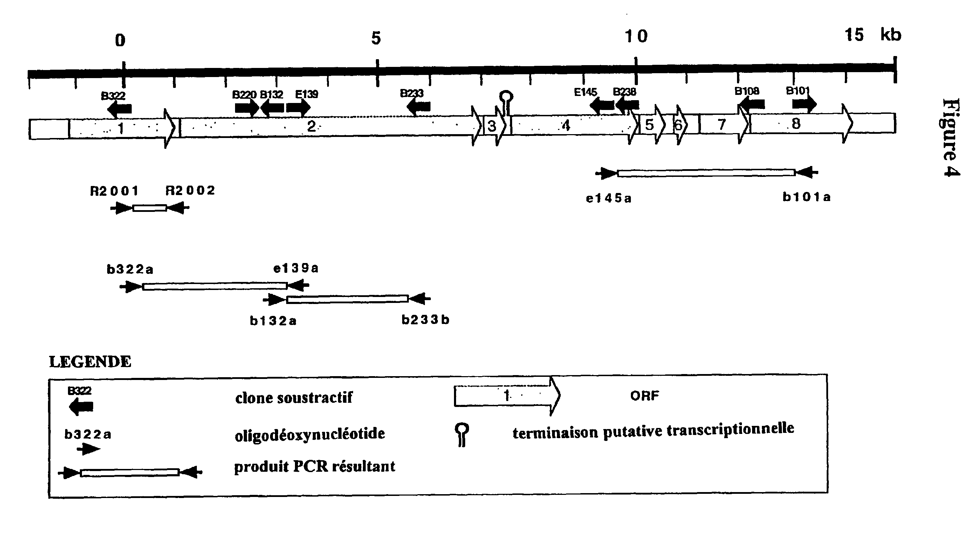

On a représenté sur la figure 4 la position approximative de ces oligonucléotides.

Il s'agit, dans une moitié de COL-1, des oligonucléotides appelés R2001 (SEQ ID N°46) et R2002 (SEQ ID N°47), dans une moitié de COL-1+la majeure partie de COL-2, des oligonucléotides b332a (SEQ ID N°48), e139a (SEQ ID N°49), b132a (SEQ ID N°50) et b233b (SEQ ID N°51), et dans 1/3 de COL-4+ COL-5 à 7, des oligonucléotides e145a (SEQ ID N°52) et b101a (SEQID N°53).

Les trois Southerns sont réalisés dans les conditions d'hybridation suivantes:
16 h à 65°C,
NaPO₄ 0,5M, pH 7,2
EDTA-Na 0.001M
1% de dodécylsulfate de sodium.

Pour le lavage, on chauffe 10 min à 65°C et on utilise NaPO₄ 0,5M, pH 7,2; EDTA-Na 0,001M, 1% de dodécylsulfate de sodium.

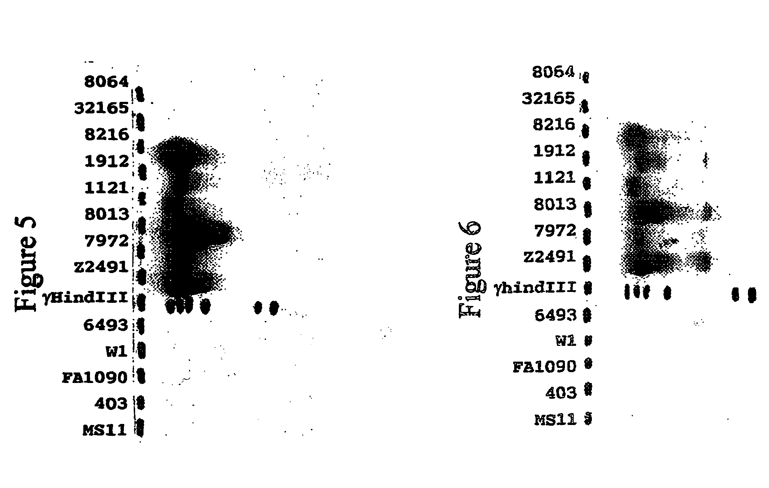

Les figures 5, 6 et 7 représentent respectivement les Southern blots obtenus avec chacune des parties de COL mentionnées plus haut.

Les 14 pistes correspondent respectivement, dans chacun des Southerns, à
1: MS11 (Ng)
2: 403 (Ng)
3: FA1090 (Ng)
4: W1 (Ng)
5: 6493 (Ng)
6: marqueur (lambda hindIII)
7: Z2491 (Nm, gpA)
8: 7972 (Nm gpA)
9: 8013 (Nm, gpC)
10: 1121 ( Nm non groupable)
11: 1912 (Nm, gpB)
13: 32165 (Nc)
14: 8064 (Nl).

Etant donné qu'un panel de souches de *Neisseria* est utilisé dans ces expériences et que chaque puits est chargé avec une quantité similaire d'ADN digéré, ces 3 Southerns blots montrent clairement que les séquences correspondant à la région 2 sont trouvées dans tous les méningocoques testés et qu'il n'existe pas dans le génome de Ng des souches testées de séquences homologues significatives.

### Exemple 4: Identification de régions du génome de Nm absentes de Nl et communes avec Ng

On opère selon la technique décrite dans l'exemple 1, mais on utilise l'ADN chromosomique d'une souche de Nm (Z2491) et de 2 souches de Nl (collection XN) dont on mélange les ADN à parts égales.

On efffectue 2 soustractions en utilisant les séries d'amorces R et J. Trois banques différentes sont ainsi réalisées.

Deux banques, appelées Bam et Eco, sont respectivement obtenues par digestion de l'ADN chromosomique de Nm Z2491 par *MboI* et *Tsp5091*; une troisième banque, appelée Cla, qui résulte de la digestion de l'ADN chromosomique de Nm par *MspI,* est obtenue en utilisant le jeu d'amorces RMsp10, RMsp24, JMsp10 et JMsp24. L'ensemble des amorces utilisées est donné dans le tableau 2 suivant.

**Tableau 2**

| Adaptateurs pour banques différentielles | | |
|---|---|---|
| ADN chromosomique digéré par | | Clonage dans pBluescript par |
| | | |
| *Mbo*I | → | *Bam*HI |
| *Tsp*509I | → | *Eco*RI |
| *Msp*I | → | *Cla*I |
| | | |
| Premier tour de soustraction | | |
| RBam12 : | 3' AGTGGCTCCTAG | 5' (SEQ ID N°54) |
| RBam24 : | 5' AGCACTCTCCAGCCTCTCACCGAG | 3' (SEQ ID N°55) |
| | | |
| REco12 : | AGTGGCTCTTAA | (SEQ ID N°56) |
| RBam24 : | 5'AGCACTCTCCAGCCTCTCACCGAG (REco 24 = RBam 24) | 3' (SEQ ID N°55) |
| | | |
| RMsp10 : | AGTGGCTGGC | (SEQ ID N°57) |
| RMsp24 : | 5'AGCACTCTCCAGCCTCTCACCGAC | 3' (SEQ ID N°58) |
| | | |
| Deuxième tour de soustraction | | |
| Jbam12 : | 3' GTACTTGCCTAG | 5' (SEQ ID N°59) |
| JBam24 : | 5' ACCGACGTCGACTATCCATGAACG | 3' (SEQ ID N°60) |
| | | |
| JEco12 : | GTACTTGCTTAA | (SEQ ID N°61) |
| JBam24 : | 5'ACCGACGTCGACTATCCATGA ACG (JEco 24 = JBam 24) | 3' (SEQ ID N°60) |
| | | |
| JMsp10 : | GTACTTGGGC | (SEQ ID N°62) |
| JMsp24 : | 5' ACCGACGTCGACTATCCATGAACC | 3'(SEQ ID N°63) |

Après 2 soustractions, on marque la totalité du produit de chaque amplification et on l'utilise comme sonde.

On effectue un contrôle des banques soustractives par Southern blot sur un panel de 12 souches de *Neisseria* (ADN chromosomique coupé par *ClaI).* Les conditions d'hybridation sont identiques à celles données dans l'exemple 1.

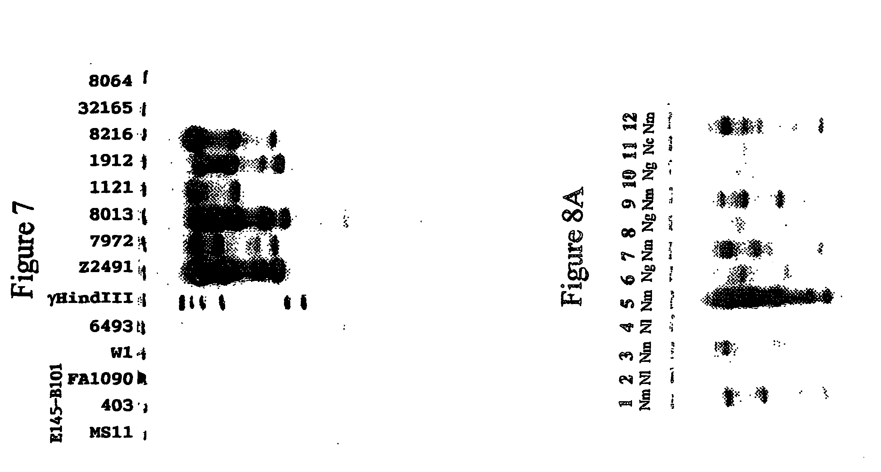

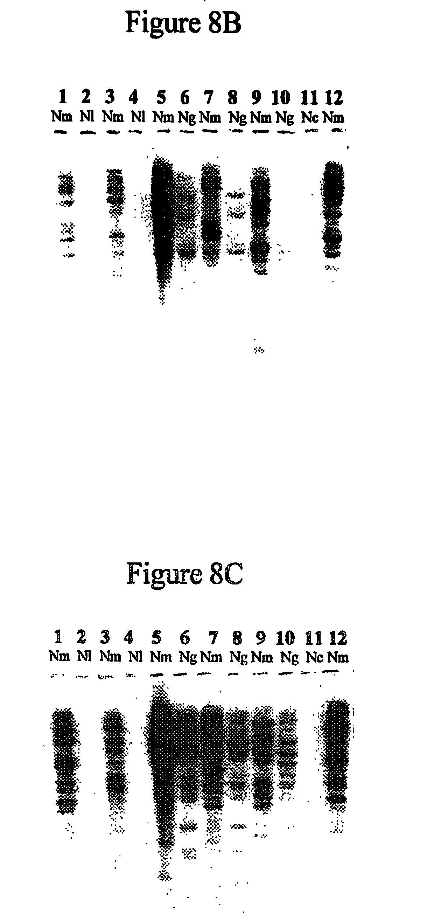

Ces Southern blots sont donnés sur les figures 8A à 8C, qui sont respectivement relatives à la banque *MboI*/*BamHI,* à la banque *MspI*/*ClaI* et à la banque *Tsp5091*/*ECORI.*

Les 12 pistes correspondent respectivement à :
1: Nm Z2491 (groupe A)
2: Nl 8064
3: Nm 8216 (groupe B)
4: Nl 9764
5: Nm 8013 (groupe C)
6: Ng MS11
7: Nm 1912 (groupe A)
8: Ng 4C1
9: Nm 1121 (non groupable)
10: Ng FA1090
11: Nc 32165
12: Nm 7972 (groupe A).

L'examen des Southern blots montre que les séquences contenues dans chaque banque sont spécifiques de Nm et ne sont pas trouvées chez Nl. De plus, la réactivité observée avec les souches de Ng suggère que certaines de ces séquences sont présentes chez Ng.

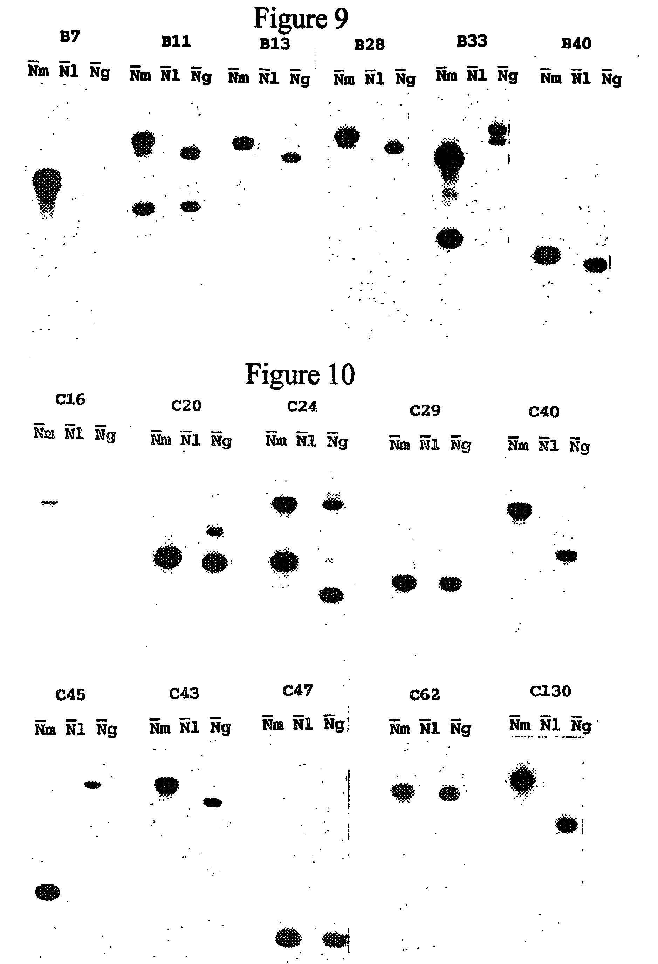

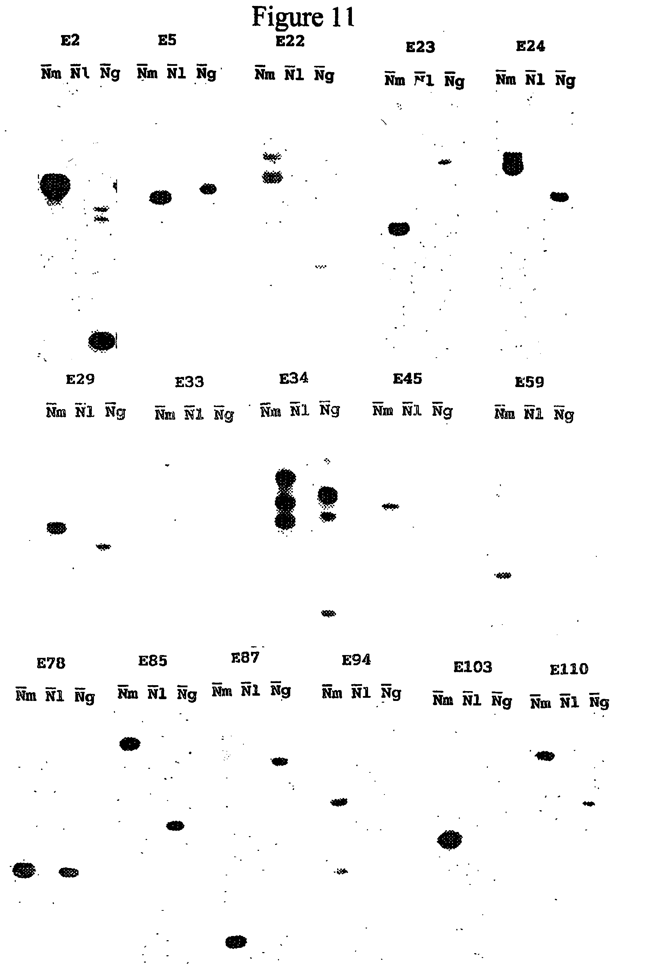

Chacune de ces banques a ensuite été clonée dans pBluescript au site *BamHI* pour Bam, ou *EcoRI* pour Eco, ou *ClaI* pour Cla. Afin de confirmer la spécificité des clones vis-à-vis du génome de Nm, on a procédé à une restriction des clones individuels et à leur radiomarquage. Les clones montrant à la fois une réactivité pour Nm et Ng ont été conservés pour des études ultérieures. Ces clones sont représentés sur les figures 9, 10 et 11, qui donnent les profils, vis-à-vis de Nm, Nl et Ng, de 5 clones de la banque Bam (figure 9), de 16 clones de la banque Eco (figure 10), et de 13 clones de la banque Cla (figure 11).

Ces clones ont été séquencés en utilisant des amorces universelles et inverses. Il s'agit
- des clones Bam
   B11 partiel de 140 pb (SEQ ID N°66), B13 partiel estimé à 425 pb (SEQ ID N° 67), B26 de 181 pb (SEQ ID N° 68), B33 de 307 pb (SEQ ID N° 69), B40 de 243 pb (SEQ ID N° 70),
- des clones Cla
   C16 de 280 pb (SEQ ID N° 72), C20 partiel estimé à 365 pb (SEQ ID N° 73), C24 partiel estimé à 645 pb (SEQ ID N° 74), C29 partiel estimé à 245 pb (SEQ ID N° 75), C34 de 381pb (SEQ ID N°76), C40 de 269 pb (SEQ ID N° 77),C42 de 203 pb (SEQ ID N°78) ,p C43 de 229 pb (SEQ ID N° 79), C45 de 206 pb (SEQ ID N° 80),C47 de 224 pb (SEQ ID N° 81), C62 de 212 pb (SEQ ID N° 82), et C130 (5'...) estimé à 900 pb (SEQ ID N° 83), et
- des clones Eco
   E2 de 308 pb (SEQ ID N° 84), E5 partiel, estimé à 170 pb (SEQ ID N° 85), E22 partiel estimé à 300 pb (SEQ ID N° 86), E23 de 273 pb (SEQ ID N° 87), E24 de 271 pb (SEQ ID N° 88), E29 de 268 pb (SEQ ID N° 89), E33 partiel, estimé à 275 pb (SEQ ID N°90), E34 partiel, estimé à 365 pb (SEQ ID N° 91), E45 de 260 pb (SEQ ID N° 92), E59 estimation supérieure à 380 pb (SEQ ID N° 93), E78 de 308 pb (SEQ ID N° 94), E85 de 286 pb (SEQ ID N° 95), E87 de 238 pb (SEQ ID N° 96), E94 partiel, supérieur à 320 pb (SEQ ID N° 97), E103 partiel, supérieur à 320 pb (SEQ ID N° 98) et E110 de 217 pb (SEQ ID N° 99).

La cartographie de chaque clone a été effectuée sur le chromosome de Nm Z2491 en opérant comme décrit dans l'exemple 2. Les résultats obtenus sont donnés sur la partie droite de la figure 2. On constate que ces clones correspondent aux régions appelées 4 et 5. Ces régions sont donc constituées de séquences présentes à la fois chez Nm et chez Ng, mais non trouvées chez Nl. Il est donc considéré qu'il s'agit de séquences codant pour des facteurs de virulence responsables de la colonisation initiale et de la pénétration de la muqueuse. La région 4 est localisée entre *argF* et *regF* sur le chromosome de Nm 2491 et la région 5 entre le marqueur lambda 375 et *penA.* Cette région contient vraissemblablement des séquences codant pour un variant Opa et une protéine liant la transferrine.

Une comparaison avec les séquences connues dans les banques de données a moitié que dans la région 4 seul le clone C130 présente une homologie, à savoir avec *MspI* méthylase. Dans la région 5, aucune homologie avec des séquences connues n'a été trouvée avec les clones C8, E2, B40, C45, E23 et E103. Pour les autres clones, les homologies sont les suivantes :
B11 arginine décarboxylase SpeA; C29 arginine décarboxylase SpeA; C62 oxoglutarate/malate transporteur; repetitive DNA élément; E34 élément répétitif d'ADN ; E94 endopeptidase MepA murine ; C47 citrate synthase PrpC; E78 citrate synthase PrpC

### Exemple 5 : Composition vaccinale incluant dans son spectre une prophylaxie à visée anti-méningococcique et destinée à prévenir toute forme d'infection par Neisseria meningitidis.

Le peptide codé par une séquence incluant la SEQ ID n°10 est conjugué à une toxine.

Ce peptide conjugué est alors ajouté à une composition comportant le vaccin anti-*Haemophilus* et anti-pneumocoque, ou tout autre vaccin de l'enfance.

La composition résultante peut, après avoir été rendue stérile, être injectée par voie parentérale, sous-cutanée ou intramusculaire.

Cette même composition peut également être pulvérisée au niveau des muqueuses à l'aide d'un spray.

## Revendications

1. ADN isolé spécifique de *Neisseria* **caractérisé en ce qu'**il s'agit de
- l'ADN de séquence SEQ ID N°10, ou l'ADN de séquence SEQ ID N°36 - ou d'un ADN spécifique de Nm souche Z2491 s'hybridant sur Southern blot avec l'ADN de séquence SEQ ID N°10, mais ne s'hybridant pas sur Southern blot à une séquence d'ADN d'une souche de *Neisseria gonorrhoeae* (ci-après Ng), dans les conditions d'hybridation suivantes:
16h à 65°C, avec une solution comprenant NaPO₄ 0,5 M pH 7,2, EDTA-Na 0,001 M, 1% d'albumine de sérum bovin et 7% de dodécylsulfate de sodium ; suivies par 2 lavages dans une solution comprenant NaPO₄ 40 mM pH 7,2, EDTA 1mM, et SDS 1% ; le lavage final étant réalisé à 65°C pendant 5 min ;
- ou de l'ADN de séquence SEQ ID N°95 ou, d'un ADN ou d'un ADN de Nm souche Z2491 et de Ng souche MS11 s'hybridant sur Southern blot avec l'ADN de séquence SEQ ID N°95, mais ne s'hybridant pas à une séquence d'ADN d'une souche de *Neisseria lactamica* (ci-après Nl), dans les conditions d'hybridation suivantes:
16h à 65°C, avec une solution comprenant NaPO₄ 0,5 M pH 7,2, EDTA-Na 0,001 M, 1% d'albumine de sérum bovin et 7% de dodécylsulfate de sodium ; suivies par 2 lavages dans une solution comprenant NaPO₄ 40 mM pH 7,2, EDTA 1mM, et SDS 1% ; le lavage final étant réalisé à 65°C pendant 5 min.

2. Les ADN complémentaires sur toute la longueur des séquences d'ADN selon la revendication 1.

3. ADN selon la revendication 1, **caractérisé en ce qu'**il code pour une protéine exportée au-delà de la membrane cytoplasmique.

4. ADN selon l'une quelconque des revendications 1 à 3, **caractérisé en ce qu'**il est inséré dans un vecteur de transfert ou d'expression tel que cosmide, plasmide ou bactériophage.

5. Cellule hôte, plus particulièrement cellule bactérienne ou cellule de Nm, transformée par l'insertion d'au moins un ADN selon l'une quelconque des revendications 1 à 3.

6. ARN, **caractérisé en ce que** sa séquence correspond à la transcription d'une séquence d'ADN selon l'une quelconque des revendications 1 à 4, étant entendu que ces ARN hybrident avec la séquence SEQ ID N°10, 36, ou 95.

7. Polypeptide, **caractérisé en ce qu'**il est tel que codé par un ARN selon la revendication 6, étant entendu, en ce qui concerne la séquence SEQ ID N°36, qu'il s'agit des phases de lecture des positions 1330 à 2970 ou 3083 à 9025.

8. Polypeptide, **caractérisé en ce qu'**il présente un enchaînement d'acides aminés codé par une phase de lecture s'hybridant avec la séquence SEQ ID N°10; ou 36, correspondant aux phases de lecture des positions 1330 à 2970 ou 3083 à 9025; ou 95; dans les conditions d'hybridation définies dans la revendication 1.

9. Polypeptide **caractérisé en ce qu'**il présente la séquence SEQ ID N°37 ou SEQ ID N°38.

10. Méthode de diagnostic d'une infection méningococcique, et plus particulièrement de la méningite méningococcique, par mise en évidence de la présence de Nm dans un échantillon biologique, **caractérisée en ce qu'**elle comprend les étapes de :
- mise en contact d'un échantillon biologique à analyser, avec un réactif comprenant SEQ ID N°10 ou SEQ ID N°95, ou l'ADN complémentaire de ces séquences, le cas échéant sous forme de sonde nucléotidique, dans des conditions permettant respectivement une hybridation ou une réaction de type antigène-anticorps, et
- révélation du produit de réaction éventuellement formé.

11. Composition, **caractérisée en ce qu'**elle comprend au moins un polypeptide selon l'une des revendications 7 à 9, en association avec un véhicule physiologiquement acceptable.

## Claims

1. An isolated DNA specific to Neisseria which is
- DNA of sequence SEQ ID N°10, or DNA of sequence SEQ ID N°36 or a DNA specific to Nm strain Z2491, hybridizing on Southern blot to the DNA of sequence SEQ ID N°10, but not hybridizing on Southern blot to a DNA sequence of Neisseria gonorrheae strain (hereinafter Ng), under the following hybridization conditions:
- 16 h at 65°C, with a solution comprising 0.5 M NaPO₄ pH 7.2, 0.001 M EDTA-Na, 1% bovine serum albumin and 7% sodium dodocylsulphate, followed by at least two washes in a solution comprising 40 mM Na PO₄ pH 7.2, 1 mM EDTA, and 1% SDS, the final wash being conducted at 6°C for 5 minutes,
- or DNA of sequence SEQ ID N°95, or, a DNA or a DNA of Nm strain Z2491 and Ng strain MS11, hybridizing on Southern blot with the DNA of sequence SEQ ID N°95, but not hybridizing on Southern blot to a DNA sequence of Neisseria lactamica strain (hereinafter N1), under the following hybridization conditions:
- 16 h at 65°C, with a solution comprising 0.5 M NaPO₄ pH 7.2, 0.001 M EDTA-Na, 1% bovine serum albumin and 7% sodium dodocylsulphate, followed by at least two washes in a solution comprising 40 mM Na PO₄ pH 7.2, 1 mM EDTA, and 1% SDS, the final wash being conducted at 65°C for 5 minutes.

2. The complementary DNA on the whole length of the DNA sequences according to claim 1.

3. DNA according claim 1, **characterized in that** it codes for a protein exported beyond the cytoplasmic membrane.

4. DNA according to any one of claims 1 to 3, **characterized in that** it is inserted in a transfer or expression vector, such as a cosmid, plasmid or bacteriophage.

5. Host cell, more particularly bacterial cell or Nm cell, transformed by insertion of at least one DNA according to any one of claims 1 to 3.

6. RNA, **characterized in that** its sequence corresponds to the transcription of a DNA sequence according to anyone of claims 1 to 4,this being understood that said RNAs hybridize to sequence SEQ ID N°10, 36 or 95.

7. Polypeptide, **characterized in that** it is such as coded by a RNA according to claim 6, this being understood that, concerning sequence SEQ ID N°36, it corresponds to reading frames of positions 1330 to 2970 or 3083 to 9025.

8. Polypeptide having an amino acid chain coded by a reading frame hybridizing to sequence SEQ ID N°10; or 36, corresponding to the reading frames of positions 1330 to 2970 or 3083 to 9025; or 95; under the hybridization conditions defined in claim 1.

9. Polypeptide having sequence SEQ ID N°37 or SEQ ID N°38.

10. Method for diagnosis of a meningococcal infection, and more particularly of meningococcal meningitis, by demonstration of the presence of Nm in a biological sample, **characterized in that** it comprises the steps of:
- bringing into contact a biological sample to be analysed with a reagent comprising SEQ ID N°10 or SEQ ID N°95, or the complementary DNA of these sequences, optionally under the form of nucleotidic probe, under conditions which allow respectively hybridization or a reaction of the antigen-antibody type, and
- detecting any reaction product eventually formed.

11. Composition, comprising at least a polypeptide according to anyone of claims 7 to 9, in association with a physiologically acceptable carrier.

## Patentansprüche

1. Spezifische isolierte DNA von *Neisseria,* **dadurch gekennzeichnet, dass** es sich um Folgendes handelt:
- DNA der Sequenz SEQ ID Nr. 10 oder DNA der Sequenz SEQ ID Nr. 36 oder eine spezifische DNA des Nm-Stamms Z2491, der im Southern Blot mit der DNA der Sequenz SEQ ID Nr. 10 hybridisiert, aber nicht im Southern Blot mit einer DNA-Sequenz eines Stamms von *Neisseria gonorrhoeae* (im Folgenden Ng) hybridisiert, mit den folgenden Hybridisierungsbedingungen:
16 h bei 65 °C mit einer Lösung, die 0,5 M NaPO₄, pH 7,2, 0,001 M EDTA-Na, 1% Rinderserumalbumin und 7% Natriumdodecylsulfat umfasst; danach zweimaliges Waschen in einer Lösung, die 40 mM NaPO₄, pH 7,2, 1 mM EDTA und 1% SDS umfasst; wobei die letzte Waschung 5 min lang bei 65 °C durchgeführt wird;
- oder DNA der Sequenz SEQ ID Nr. 95 oder DNA oder DNA des Nm-Stamms Z2491 und des Ng-Stamms MS11, der im Southern Blot mit der DNA der Sequenz SEQ ID Nr. 95 hybridisiert, aber nicht im Southern Blot mit einer DNA-Sequenz eines Stamms von *Neisseria lactamica* (im Folgenden NI) hybridisiert, mit den folgenden Hybridisierungsbedingungen:
16 h bei 65 °C mit einer Lösung, die 0,5 M NaPO₄, pH 7,2, 0,001 M EDTA-Na, 1% Rinderserumalbumin und 7% Natriumdodecylsulfat umfasst; danach zweimaliges Waschen in einer Lösung, die 40 mM NaPO₄, pH 7,2, 1 mM EDTA und 1% SDS umfasst; wobei die letzte Waschung 5 min lang bei 65 °C durchgeführt wird.

2. DNA, die über die ganze Länge komplementär zu den DNA-Sequenzen gemäß Anspruch 1 sind.

3. DNA gemäß Anspruch 1, **dadurch gekennzeichnet, dass** sie für ein Protein codiert, das nach jenseits der Cytoplasmamembran exportiert wird.

4. DNA gemäß einem der Ansprüche 1 bis 3, **dadurch gekennzeichnet, dass** sie in einen Transfer- oder Expressionsvektor, wie ein Cosmid, Plasmid oder einen Bakteriophagen, inseriert ist.

5. Wirtszelle, insbesondere Bakterienzelle oder Nm-Zelle, transformiert durch Insertion wenigstens einer DNA gemäß einem der Ansprüche 1 bis 3.

6. RNA, **dadurch gekennzeichnet, dass** ihre Sequenz der Transcription einer DNA-Sequenz gemäß einem der Ansprüche 1 bis 4 entspricht, wobei diese RNA mit der Sequenz SEQ ID Nr. 10, 36 oder 95 hybridisieren sollen.

7. Polypeptid, **dadurch gekennzeichnet, dass** es durch eine RNA gemäß Anspruch 6 codiert ist, wobei es sich, was die Sequenz SEQ ID Nr. 36 betrifft, um Leseraster der Positionen 1330 bis 2970 oder 3083 bis 9025 handeln soll.

8. Polypeptid, **dadurch gekennzeichnet, dass** es eine Aminosäuresequenz aufweist, die von einem Leseraster codiert wird, das mit der Sequenz SEQ ID Nr. 10 oder 36, was den Leserastern der Positionen 1330 bis 2970 oder 3083 bis 9025 entspricht, oder 95 hybridisiert, unter den Hybridisierungsbedingungen, wie sie in Anspruch 1 definiert sind.

9. Polypeptid, **dadurch gekennzeichnet, dass** es die Sequenz SEQ ID Nr. 37 oder SEQ ID Nr. 38 aufweist.

10. Verfahren zur Diagnose einer Infektion durch Meningokokken und insbesondere der Meningokokken-Meningitis durch Nachweis der Anwesenheit von Nm in einer biologischen Probe, **dadurch gekennzeichnet, dass** es die folgenden Schritte umfasst:
- In-Kontakt-Bringen einer zu analysierenden biologischen Probe mit einem Reagens, das SEQ ID Nr. 10 oder SEQ ID Nr. 95 oder zu diesen Sequenzen komplementäre DNA umfasst, gegebenenfalls in Form einer Nucleotidsonde, unter Bedingungen, die eine Hybridisierung bzw. eine Reaktion des Antigen-Antikörper-Typs ermöglichen; und
- Nachweis des gegebenenfalls gebildeten Reaktionsprodukts.

11. Zusammensetzung, **dadurch gekennzeichnet, dass** sie wenigstens ein Polypeptid gemäß einem der Ansprüche 7 bis 9 in Verbindung mit einem physiologisch annehmbaren Träger umfasst.
